# EUROPEAN PATENT APPLICATION

(11) **EP 4 570 795 A1**
(43) Date of publication of application: **18.06.2025**
(21) Application number: 23852555.4
(22) Date of filing: 08.08.2023
(51) Int. Cl.: C07D 401/14, A61K 31/496, A61K 31/513, A61K 31/517, A61P 35/00, A61P 43/00, C07D 403/14, C07D 405/14, C07D 471/10, C07D 487/10

(54) **HETEROCYCLIC COMPOUND FOR INDUCING DEGRADATION OF G12V MUTANT KRAS PROTEIN**

(30) Priority: 09.08.2022 JP 2022126815
(71) Applicant: Astellas Pharma, Inc., Chuo-ku Tokyo 103-8411 (JP)
(72) Inventor: MORIKAWA, Takahiro, Tokyo 103-8411 (JP); IMAIZUMI, Tomoyoshi, Tokyo 103-8411 (JP); OKUMURA, Mitsuaki, Tokyo 103-8411 (JP); HAMAGUCHI, Hisao, Tokyo 103-8411 (JP); IMADA, Sunao, Tokyo 103-8411 (JP); KOGANEMARU, Yohei, Tokyo 103-8411 (JP); KAWAMINAMI, Eiji, Tokyo 103-8411 (JP); YOSHINARI, Tomohiro, Tokyo 103-8411 (JP); KURAMOTO, Kazuyuki, Tokyo 103-8411 (JP); NISHIZONO, Yoshihiro, Tokyo 103-8411 (JP)
(74) Representative: Hoffmann Eitle
(86) International application number: PCT/JP2023/028864
(87) International publication number: WO 2024/034593

(57) **Abstract**

To provide a compound useful as an active ingredient of a pharmaceutical composition for treating pancreatic cancer and/or lung cancer. The present inventors have studied about a compound that is useful as an active ingredient of a pharmaceutical composition for treating pancreatic cancer and/or lung cancer and have found that heterocyclic compounds represented by the formula (I) have an excellent degradation-inducing action on a G12V mutant KRAS protein and a G12V mutant KRAS inhibition activity and can be used as a therapeutic agent for pancreatic cancer and/or lung cancer, thus completing the present invention. The heterocyclic compound of the present invention or a salt thereof can be used as a therapeutic agent for pancreatic cancer and/or lung cancer.

## Description

### Technical Field

The present invention relates to pharmaceutical compositions and, in particular, to a heterocyclic compound that is excellent in a degradation-inducing action on a G12V mutant KRAS protein and that is expected to be useful as a G12V mutant KRAS inhibitor and to be useful, for example, as an active ingredient of a pharmaceutical composition for treating pancreatic cancer and/or lung cancer.

### Background Art

Pancreatic cancer mainly including pancreatic ductal adenocarcinoma is a cancer with a very poor prognosis having a five-years survival rate of 10% or less (CA Cancer J. Clin., 2016, 66, p.7-30), and about 460,000 new cases are reported per year in the world (CA Cancer J. Clin., 2018, 68, p.394-424). The most effective therapy for treating pancreatic cancer is a surgery. However, the cancer has often metastasized since early detection is difficult, and the therapeutic effect of a surgery cannot be expected in many cases. When the cancer is not treated by operation, chemotherapy or radiotherapy is adopted, but the survival rate is not so good. Today, the FOLFIRINOX therapy (multidrug treatment of three chemotherapy agents of 5-FU, irinotecan and oxaliplatin, plus levofolinate) is used as a standard therapy of pancreatic cancer. However, due to the strong toxicity, the subject patient has to be cautiously selected, for example, the therapy is to be applied only to patients of an ECOG performance status of 1 or less (J. Clin. Oncol., 2018, 36, p.2545-2556). As a molecular target drug, an epidermal growth factor receptor (EGFR) inhibitor, Erlotinib, has been approved in a combination therapy with Gemcitabine. However, the extension of the overall survival is only about two weeks as compared with Gemcitabine alone, and no satisfying therapeutic effect has been achieved. A highly effective therapeutic agent remains needed (J. Clin. Oncol., 2007, 25, p.1960-1966).

The number of deaths due to lung cancer is the largest, and about 2.1 million new cases are reported per year in the world (World Cancer Report 2020). In particular, non-small-cell lung cancer (NSCLC) accounts for 80 to 85% of lung cancer cases (American Cancer Society, Cancer Facts and Figures, 2022). Although surgical therapy has been considered until a certain stage, after that stage, surgery is rarely indicated while chemotherapy or radiotherapy is used as a main treatment. Based on cell morphology, adenocarcinoma and squamous cell carcinoma are classified as the most common subtypes of NSCLC. The clinical course of these tumors is similar, but adenocarcinoma is characterized by the peripheral localization of the lungs.

RAS proteins are low molecular weight guanosine triphosphate (GTP)-binding proteins of about 21 kDa constituted of 188-189 amino acids and include four main types of proteins (KRAS (KRAS 4A and KRAS 4B), NRAS and HRAS) produced by three genes of a KRAS gene, an NRAS gene and an HRAS gene. RAS proteins are divided into an active GTP-binding type and an inactive GDP-binding type. A RAS protein is activated by replacement of guanosine diphosphate (GDP) with GTP due to, for example, ligand stimulation to a membrane receptor, such as EGFR. The active RAS binds to effector proteins as much as twenty, such as RAF, PI3K and RALGDS, to activate the downstream signal cascade. On the other hand, the active RAS is converted to the inactive type by replacement of GTP with GDP due to the intrinsic GTP hydrolysis (GTPase) activity. The GTPase activity is enhanced by a GTPase-activating protein (GAP). As can be seen from the above statement, RAS bears an important function of "molecular switch" in an intracellular signal transduction pathway for EGFR or the like and plays a critical role in the processes of cell growth, proliferation, angiogenesis and the like (Nature Rev. Cancer, 2011, 11, p.761-774, Nature Rev. Drug Discov., 2014, 13, p.828-851, Nature Rev. Drug Discov., 2016, 15, p.771-785).

Substitution of an amino acid by spontaneous mutation of the RAS gene results in a constant activated state due to hypofunction of RAS as GTPase or hyporeactivity to GAP, and then, signals are continuously sent downstream. The excessive signaling causes carcinogenesis or cancer growth acceleration. It is said that pancreatic ductal adenocarcinoma occurs through a weakly heteromorphic stage and a subsequent highly heteromorphic stage in the pancreatic intraepithelial neoplasia (PanIN), and mutation of the KRAS gene has already been recognized in an initial stage of PanIN. Subsequently, abnormality occurs in INK4A, p53 and SMAD4, which are tumor suppression genes, leading to malignancy (Nature Rev. Cancer, 2010, 10, p.683-695). Furthermore, in 90% or more of the cases of pancreatic ductal adenocarcinoma, mutation is seen in the KRAS gene, and a majority of them are a spontaneous point mutation in the codon 12 located in the KRAS exon 2 (Cancer Cell 2017, 32, p.185-203). In the lung cancer, mutation is seen in the RAS gene in 32% of the cases of lung adenocarcinoma. It is reported that the breakdown of the mutation frequency is 96% of the KRAS gene, 3% of the NRAS gene and 1% of the HRAS gene, with a high prevalence of spontaneous point mutations in the KRAS exon 2 (codon 12 and codon 13) (Nature Rev. Drug Discov., 2014, 13, p.828-851). As can be seen from the above statement, KRAS plays a critical role in the processes of carcinogenesis and development of pancreatic cancer and lung adenocarcinoma.

As a mutation of a KRAS gene, in particular, a KRAS G12V mutation, in which glycine at codon 12 is replaced by valine, a KRAS G12D mutation, in which the glycine is replaced by aspartic acid, a KRAS G12C mutation, in which the glycine is replaced by cysteine, and the like are known. In recent years, several G12C mutation-selective inhibitors have been developed, among which Sotorasib has been approved by FDA as a therapeutic agent for non-small-cell lung cancer (Drugs, 2021, 81, p.1573-1579).

Patent Documents 1, 2 and 3 disclose RAS inhibitors, and Patent Documents 2 and 3 disclose compounds represented by the following formulae (A) and (B) (the meanings of the symbols in the formulae can be found in the patent documents), respectively. Patent Documents 1, 2 and 3 state that the inhibitors are useful for a cancer with a mutation in the codon 12 of KRAS. Although the G12V mutation is one of such mutations, any effect on the G12V mutant KRAS is not described.

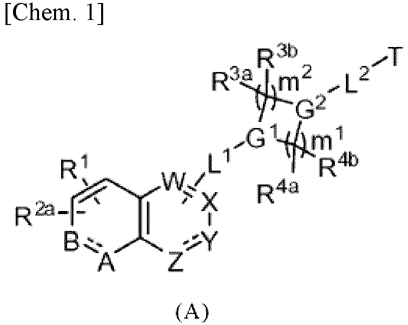

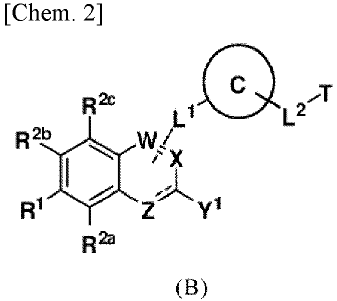

Moreover, Patent Document 9 discloses a pan-KRAS inhibitor.

In recent years, as a technique for inducing degradation of a target protein, bifunctional compounds collectively called as PROTAC (PROteolysis-TArgeting Chimera) or SNIPER (Specific and Nongenetic IAP-dependent Protein Eraser) are found and are expected as one novel technique of drug development modality (Drug. Discov. Today Technol., 2019, 31, p15-27). Such a bifunctional compound promotes formation of a composite of the target protein and an E3 ligase in a cell, and degradation of the target protein is induced using the ubiquitin-proteasome system. The ubiquitin-proteasome system is one of intracellular protein degradation mechanisms. A protein called E3 ligase recognizes a protein to be degraded to proceed ubiqutination of the protein, whereby degradation by proteasome is promoted.

There are 600 or more E3 ligases in the living body, which can be broadly classified into four subclasses: HECT-domain E3s, U-box E3s, monomeric RING E3s and multi-subunit E3s. The E3 ligases used in bifunctional degradation inducers called PROTAC, SNIPER and the like are currently limited, and typical examples thereof include Von Hippel-Lindau (VHL), celebron (CRBN), inhibitor of apoptosis protein (IAP) and mouse double minute 2 homolog (MDM2). In particular, VHL and CRBN are reported in Patent Document 4 and Patent Document 5, respectively.

The bifunctional compounds are compounds in which a ligand of a target protein and a ligand of an E3 ligase are bound via a Linker, and some bifunctional compounds for degrading a KRAS protein have ever been reported (Non-patent Document 1, Non-Patent Document 2, Patent Document 6, Patent Document 7, Patent Document 8, and Patent Documents 10 to 22).

### Citation List

### Patent Document

Patent Document 1: WO 2016/049565
Patent Document 2: WO 2016/049568
Patent Document 3: WO 2017/172979
Patent Document 4: WO 2013/106643
Patent Document 5: WO 2015/160845
Patent Document 6: US Patent Application Publication No. 2018/0015087
Patent Document 7: WO 2019/195609
Patent Document 8: WO 2020/018788
Patent Document 9: WO 2022/132200
Patent Document 10: WO 2021/051034
Patent Document 11: WO 2022/087335
Patent Document 12: WO 2021/207172
Patent Document 13: WO 2022/111521
Patent Document 14: WO 2022/061348
Patent Document 15: WO 2022/148422
Patent Document 16: WO 2022/173032
Patent Document 17: WO 2022/228576
Patent Document 18: WO 2023/059609
Patent Document 19: WO 2023/077441
Patent Document 20: WO 2023/280026
Patent Document 21: Chinese Patent Application Publication No. 113956233
Patent Document 22: Chinese Patent Application Publication No. 115785199

### Non-Patent Document

Non-patent Document 1: Cell. Chem. Biol., 2020, 27, p.19-31
Non-patent Document 2: ACS Cent. Sci., 2020, 6, p.1367-1375

### Summary of Invention

### Technical Problem

A pharmaceutical composition, for example, a heterocyclic compound that is excellent in a degradation-inducing action on a G12V mutant KRAS protein and that is expected to be useful as a G12V mutant KRAS inhibitor and to be useful as an active ingredient of a pharmaceutical composition for treating pancreatic cancer and/or lung cancer, in particular, G12V mutant KRAS-positive pancreatic cancer and/or G12V mutant KRAS-positive lung cancer, is provided.

### Solution to Problem

The present inventors have intensively and extensively studied about a compound that is useful as an active ingredient of a pharmaceutical composition for treating pancreatic cancer and/or lung cancer. As a result, the present inventors have found that a heterocyclic compound of a formula (I), in particular, a bifunctional compound of the formula (I) characterized in that a substituent on the 8-position of a heterocyclic compound selected from the group consisting of quinazoline and quinoline is bound to a ligand of an E3 ligase or that a substituent on the 8-position of a heterocyclic compound selected from the group consisting of quinazoline and quinoline is bound to a ligand of an E3 ligase via a Linker, has an excellent degradation-inducing action on a G12V mutant KRAS protein and a G12V mutant KRAS inhibition activity, thus completing the present invention.

Specifically, the present invention relates to a compound of the formula (I) or a salt thereof and a pharmaceutical composition that contains a compound of the formula (I) or a salt thereof and one or more pharmaceutically acceptable excipients. 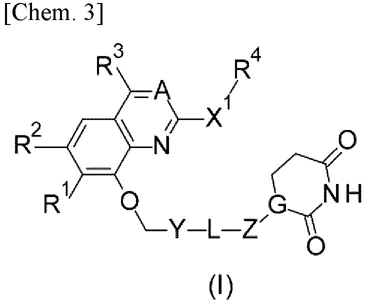 (wherein in the formula,
A is CR^{A} or N,
R^{A} is H or C₁₋₃ alkyl,
X¹ is -CH₂-, -O- or -NR^{X1}-,
R^{X1} is H or optionally substituted C₁₋₃ alkyl,
when X¹ is -NR^{X1}-, R^{X1} and R⁴ present on the same nitrogen atom, together with a nitrogen atom adjacent thereto, optionally form an optionally substituted 4-membered to 6-membered saturated heterocyclic group,
R¹ is naphthyl optionally substituted with OH, or R¹ is the formula (II) or the formula (III) below, 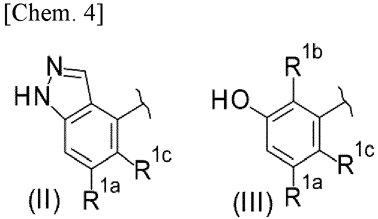
R^{1a} and R^{1b}, which are the same as or different from each other, are H, methyl, F or Cl,
R^{1c} is F, Cl, methyl or ethyl,
R² is H, halogen, C₁₋₃ alkyl, cyclopropyl or vinyl, where the C₁₋₃ alkyl is optionally substituted with a group selected from the group consisting of OH and OCH₃,
R³ is a group selected from the group consisting of the formula (IV), the formula (V), the formula (VI), the formula (VII), the formula (VIII), the formula (IX), the formula (X), the formula (XI), the formula (XII) and the formula (XXVI) below, 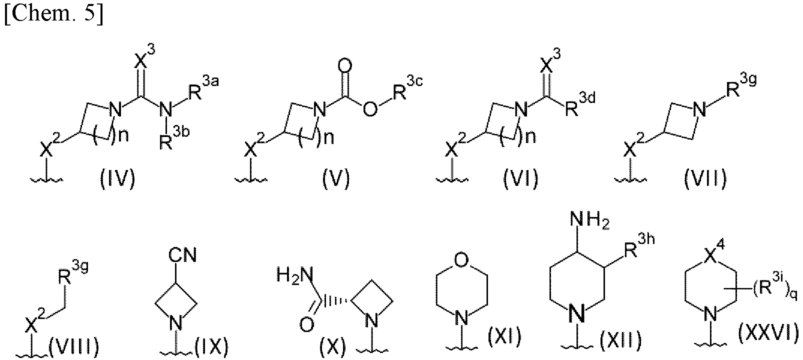
R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2};
R^{3b} is H or C₁₋₃ alkyl,
R^{3c} and R^{3d} are -(CH₂)ₚCHR^{3e}-NR^{N1}R^{N2}, -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f} and -NR^{N1}R^{N2};
R^{3e} is H, F or C₁₋₃ alkyl,
R^{3f} is H or C₁₋₃ alkyl,
R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
R^{3h} is H, F or C₁₋₃ alkyl,
each R³ⁱ, which is the same as or different from each other, is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
two R³ⁱ present on the same carbon atom, together with the carbon atom adjacent thereto, optionally form a spiro ring having a ring selected from the group consisting of a C₃₋₆ cycloalkane and a 4-membered to 6-membered saturated hetero ring, where the spiro ring is optionally substituted with one or two groups selected from the group consisting of C₁₋₃ alkyl, -O-(C₁₋₃ alkyl), OH, halogen and oxo, or
R³ⁱ present on two adjacent carbon atoms, together with the two carbon atoms, optionally forms a fused ring having a ring selected from the group consisting of a C₃₋₆ cycloalkane and a 4-membered to 6-membered saturated hetero ring, where the fused ring is optionally substituted with one or two groups selected from the group consisting of C₁₋₃ alkyl, -O-(C₁₋₃ alkyl), OH, halogen and oxo, or
R³ⁱ present on two non-adjacent carbon atoms, together with the two carbon atoms, optionally forms a bridged structure composed of one or two carbon atoms, and a ring having the bridged structure is optionally substituted with one or two groups selected from the group consisting of C₁₋₃ alkyl, -O-(C₁₋₃ alkyl), OH, halogen and oxo,
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group, or
R^{3e} and R^{N1}, together with the carbon atom and the nitrogen atom to which they are attached, optionally form an optionally substituted 4-membered to 6-membered saturated heterocyclic group,
X² is -O-, -NH- or -N(C₁₋₃ alkyl)-,
X³ is O or S,
X⁴ is -CH₂-, -CH₂-CH₂- or -O-CH₂-,
n is 1 or 2,
p is 1 or 2,
q ranges from 1 to 8,
R⁴ is C₁₋₆ alkyl, piperidinyl optionally substituted with R^{4a} or tetrahydropyranyl, where the C₁₋₆ alkyl is optionally substituted with a group selected from the group consisting of F, OH, OCH₃, R^{4a}, cyclopropyl, N(R^{4a})₂, pyrrolidinyl optionally substituted with R^{4a} and tetrahydrofuranyl,
R^{4a} is optionally substituted C₁₋₃ alkyl,
Y is phenylene optionally substituted with F or Cl or pyridinediyl,
L is -(L¹-L²-L³-L⁴-L⁵).
L¹, L², L³, L⁴ and L⁵, which are the same as or different from each other, are groups selected from the group consisting of a bond, -O-, -NR^{L1}-, an optionally substituted saturated heterocyclic divalent group containing one or two nitrogen atoms, optionally substituted C₁₋₃ alkylene and C=O,
R^{L1} is H or C₁₋₃ alkyl,
Z is a group selected from the group consisting of the formula (XIII), the formula (XIV), the formula (XV), the formula (XVI), the formula (XVII), the formula (XVIII), the formula (XIX) and the formula (XX) below, 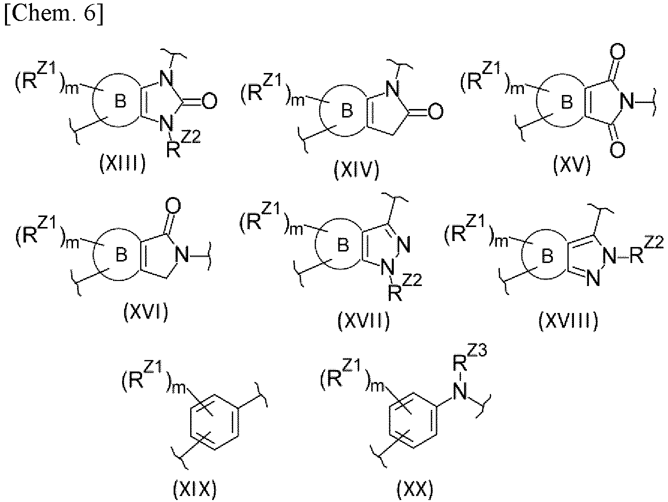
ring B is a benzene ring or a 6-membered hetero ring containing one or two nitrogen atoms,
R^{Z1} is H, C₁₋₃ alkyl, -O-(C₁₋₃ alkyl), -NR^{Z4}₂, -CONR^{Z4}₂ or -NR^{Z4}COR^{Z5},
R^{Z2} is H or C₁₋₃ alkyl,
R^{Z3} is H or C₁₋₃ alkyl,
each R^{Z4}, which is the same as or different from each other, is H or C₁₋₃ alkyl,
R^{Z5} is C₁₋₃ alkyl,
L is attached to ring B in the formulae (XIII) to (XVIII) above or to the benzene ring in the formula (XIX) and the formula (XX),
m is 1 or 2, and
G is CH or N,
provided that when G is N, Z is the formula (XVII), the formula (XVIII) or the formula (XIX) above).

Note that, when a symbol in a chemical formula herein is used in another chemical formula, the same symbol represents the same meaning unless otherwise specified.

The present invention also relates to a pharmaceutical composition containing the compound of the formula (I) or a salt thereof and one or more pharmaceutically acceptable excipients, in one embodiment, a pharmaceutical composition for treating pancreatic cancer, in one embodiment, a pharmaceutical composition for treating G12V mutant KRAS-positive pancreatic cancer, in one embodiment, a pharmaceutical composition for treating metastatic pancreatic cancer, in one embodiment, a pharmaceutical composition for treating locally advanced pancreatic cancer, in one embodiment, a pharmaceutical composition for treating recurrent or refractory pancreatic cancer, in one embodiment, a pharmaceutical composition for treating pancreatic cancer of a patient who is untreated and/or has a treatment history, in one embodiment, a pharmaceutical composition for treating metastatic G12V mutant KRAS-positive pancreatic cancer, in one embodiment, a pharmaceutical composition for treating locally advanced G12V mutant KRAS-positive pancreatic cancer, in one embodiment, a pharmaceutical composition for treating recurrent or refractory G12V mutant KRAS-positive pancreatic cancer, in one embodiment, a pharmaceutical composition for treating G12V mutant KRAS-positive pancreatic cancer of a patient who is untreated and/or has a treatment history. Note that the pharmaceutical composition for treating pancreatic cancer, the composition containing the compound of the formula (I) or a salt thereof and one or more pharmaceutically acceptable excipients, includes a therapeutic agent containing the compound of the formula (I) or a salt thereof for pancreatic cancer, and in one embodiment, for G12V mutant KRAS-positive pancreatic cancer.

The present invention relates to use of the compound of the formula (I) or a salt thereof for producing a pharmaceutical composition for treating pancreatic cancer, in one embodiment, G12V mutant KRAS-positive pancreatic cancer, in one embodiment, metastatic pancreatic cancer, in one embodiment, locally advanced pancreatic cancer, in one embodiment, recurrent or refractory pancreatic cancer, in one embodiment, pancreatic cancer of a patient who is untreated and/or has a treatment history, in one embodiment, metastatic G12V mutant KRAS-positive pancreatic cancer, in one embodiment, locally advanced G12V mutant KRAS-positive pancreatic cancer, in one embodiment, recurrent or refractory G12V mutant KRAS-positive pancreatic cancer, and in one embodiment, G12V mutant KRAS-positive pancreatic cancer of a patient who is untreated and/or has a treatment history, to use of the compound of the formula (I) or a salt thereof for treating pancreatic cancer, and in one embodiment, G12V mutant KRAS-positive pancreatic cancer, to the compound of the formula (I) or a salt thereof for use in treatment of pancreatic cancer, and in one embodiment, G12V mutant KRAS-positive pancreatic cancer, and to a method for treating pancreatic cancer, and in one embodiment, G12V mutant KRAS-positive pancreatic cancer, the method including administering an effective amount of the compound of the formula (I) or a salt thereof to a subject.

The present invention also relates to a pharmaceutical composition containing the compound of the formula (I) or a salt thereof and one or more pharmaceutically acceptable excipients, in one embodiment, a pharmaceutical composition for treating lung cancer, in one embodiment, a pharmaceutical composition for treating G12V mutant KRAS-positive lung cancer, in one embodiment, a pharmaceutical composition for treating metastatic lung cancer, in one embodiment, a pharmaceutical composition for treating locally advanced lung cancer, in one embodiment, a pharmaceutical composition for treating recurrent or refractory lung cancer, in one embodiment, a pharmaceutical composition for treating lung cancer of a patient who is untreated and/or has a treatment history, in one embodiment, a pharmaceutical composition for treating metastatic G12V mutant KRAS-positive lung cancer, in one embodiment, a pharmaceutical composition for treating locally advanced G12V mutant KRAS-positive lung cancer, in one embodiment, a pharmaceutical composition for treating recurrent or refractory G12V mutant KRAS-positive lung cancer, in one embodiment, a pharmaceutical composition for treating G12V mutant KRAS-positive lung cancer of a patient who is untreated and/or has a treatment history. Note that the pharmaceutical composition for treating lung cancer, the composition containing the compound of the formula (I) or a salt thereof and one or more pharmaceutically acceptable excipients, also includes a therapeutic agent containing the compound of the formula (I) or a salt thereof for lung cancer, and in one embodiment, for G12V mutant KRAS-positive lung cancer.

The present invention also relates to use of the compound of the formula (I) or a salt thereof for the manufacture of a pharmaceutical composition for treating lung cancer, in one embodiment, G12V mutant KRAS-positive lung cancer, in one embodiment, metastatic lung cancer, in one embodiment, locally advanced lung cancer, in one embodiment, recurrent or refractory lung cancer, in one embodiment, lung cancer of a patient who is untreated and/or has a treatment history, in one embodiment, metastatic G12V mutant KRAS-positive lung cancer, in one embodiment, locally advanced G12V mutant KRAS-positive lung cancer, in one embodiment, recurrent or refractory G12V mutant KRAS-positive lung cancer, in one embodiment, G12V mutant KRAS-positive lung cancer of a patient who is untreated and/or has a treatment history, to use of the compound of the formula (I) or a salt thereof for treating lung cancer, in one embodiment, G12V mutant KRAS-positive lung cancer, to the compound of the formula (I) or a salt thereof for use in treatment of lung cancer, in one embodiment, G12V mutant KRAS-positive lung cancer and to a method for treating lung cancer, in one embodiment, G12V mutant KRAS-positive lung cancer, the method including administering an effective amount of the compound of the formula (I) or a salt thereof to a subject.

The present invention also relates to the compound of the formula (I) or a salt thereof that is a G12V mutant KRAS protein degradation inducer and/or a G12V mutant KRAS inhibitor, to the compound of the formula (I) or a salt thereof for use as a G12V mutant KRAS protein degradation inducer and/or a G12V mutant KRAS inhibitor and to a G12V mutant KRAS protein degradation inducer and/or a G12V mutant KRAS inhibitor containing the compound of the formula (I) or a salt thereof.

Note that the "subject" is a human or another animal that needs the treatment, and in one embodiment, the "subject" is a human who needs the prevention or treatment. Advantageous Effects of Invention

The compound of the formula (I) or a salt thereof has a degradation-inducing action on a G12V mutant KRAS protein and a G12V mutant KRAS inhibition activity and can be used as a therapeutic agent for pancreatic cancer and/or lung cancer, in particular, G12V mutant KRAS-positive pancreatic cancer and/or G12V mutant KRAS-positive lung cancer. Description of Embodiments

The present invention will be described in detail below.

As used herein, "optionally substituted" means being unsubstituted or having one to five substituents. In one embodiment, the "optionally substituted" means being unsubstituted or having one to three substituents. Note that when there are multiple substituents, the substituents may be the same as or different from each other.

"C₁₋₁₂ Alkyl" is linear or branched alkyl having 1 to 12 carbon atoms, and examples thereof include methyl, ethyl, n-propyl, isopropyl, n-butyl, isobutyl, sec-butyl, tert-butyl, n-pentyl, n-hexyl, dodecyl and the like (the carbon atom numbers are described similarly hereinafter). The "C₁₋₁₂ alkyl" is ethyl or dodecyl in one embodiment.

Similarly, "C₁₋₆ alkyl" is linear or branched alkyl having 1 to 6 carbon atoms, and examples thereof include methyl, ethyl, n-propyl, isopropyl, n-butyl, isobutyl, sec-butyl, tert-butyl, n-pentyl and n-hexyl. The "C₁₋₆ alkyl" is methyl, ethyl, n-propyl, isopropyl or sec-butyl in one embodiment, methyl, ethyl, isopropyl or tert-butyl in one embodiment, or methyl, ethyl, n-propyl, isopropyl or n-butyl in one embodiment.

Similarly, "C₁₋₃ alkyl" is linear or branched alkyl having 1 to 3 carbon atoms, and examples thereof include methyl, ethyl, n-propyl, isopropyl. The "C₁₋₃ alkyl" is methyl or ethyl in one embodiment, n-propyl or isopropyl in one embodiment, methyl or isopropyl in one embodiment, ethyl or isopropyl in one embodiment, methyl in one embodiment, ethyl in one embodiment, isopropyl in one embodiment or n-propyl in one embodiment.

"C₃₋₆ Cycloalkane" is cycloalkane having 3 to 6 carbon atoms, and examples thereof include cyclopropane, cyclobutane, cyclopentane and cyclohexane.

"C₃₋₆ Cycloalkyl" is cycloalkyl having 3 to 6 carbon atoms, and examples thereof include cyclopropyl, cyclobutyl, cyclopentyl and cyclohexyl. The "C₃₋₆ cycloalkyl" is cyclobutyl, cyclopentyl or cyclohexyl in one embodiment, cyclobutyl or cyclopentyl in one embodiment, cyclopentyl or cyclohexyl in one embodiment, cyclopropyl or cyclobutyl in one embodiment, cyclopropyl in one embodiment, cyclobutyl in one embodiment, cyclopentyl in one embodiment or cyclohexyl in one embodiment.

"C₁₋₃ Alkylene" is a divalent group formed by removing the hydrogen atom from the C₁₋₃ alkyl. The "C₁₋₃ alkylene" is linear or branched C₁₋₃ alkylene, and examples thereof include methylene, ethylene, trimethylene, methylmethylene, 1,1-dimethylmethylene and the like. The "C₁₋₃ alkylene" is linear or branched C₁₋₃ alkylene in one embodiment, methylene, ethylene or trimethylene in one embodiment, methylene or ethylene in one embodiment, methylene in one embodiment or ethylene in one embodiment.

"Saturated hetero ring" is a saturated hydrocarbon ring containing hetero atom selected from the group consisting of oxygen, sulfur and nitrogen as a ring-forming atom. Further, the sulfur atom as a ring-forming atom of the saturated hetero ring is optionally oxidized. Therefore, "4-membered to 6-membered saturated hetero ring" is a 4-membered to 6-membered saturated hydrocarbon ring containing hetero atom selected from the group consisting of oxygen, sulfur and nitrogen as ring-forming atom. The "4-membered to 6-membered saturated hetero ring" is a 4-membered to 6-membered saturated hetero ring containing one or two hetero atoms selected from the group consisting of oxygen, sulfur and nitrogen as ring-forming atoms in one embodiment or oxetane, tetrahydrofuran, tetrahydropyran, azetidine, pyrrolidine, piperidine, oxazolidine, imidazolidine, piperazine, morpholine, thiomorpholine or dioxothiomorpholine in one embodiment.

"Saturated heterocyclic group" is a saturated hydrocarbon ring group containing hetero atom selected from the group consisting of oxygen, sulfur and nitrogen as a ring-forming atom. Further, the sulfur atom as a ring-forming atom of the saturated heterocyclic group is optionally oxidized.

Therefore, "4-membered to 6-membered saturated heterocyclic group" is a 4-membered to 6-membered saturated heterocyclic group containing hetero atom selected from the group consisting of oxygen, sulfur and nitrogen as ring-forming atom. The "4-membered to 6-membered saturated heterocyclic group" in one embodiment is a 4-membered to 6-membered saturated heterocyclic group containing one or two hetero atoms selected from the group consisting of oxygen, sulfur and nitrogen as ring-forming atoms. The 4-membered to 6-membered saturated heterocyclic group containing one or two hetero atoms selected from the group consisting of oxygen, sulfur and nitrogen as ring-forming atoms is a 4-membered to 6-membered saturated heterocyclic group containing one hetero atom selected from the group consisting of oxygen, sulfur and nitrogen as a ring-forming atom in one embodiment, a 5-membered or 6-membered saturated heterocyclic group containing one or two hetero atoms selected from the group consisting of oxygen, sulfur and nitrogen as ring-forming atoms in one embodiment, a 4-membered saturated heterocyclic group containing one hetero atom selected from the group consisting of oxygen, sulfur and nitrogen as a ring-forming atom in one embodiment, a 5-membered saturated heterocyclic group containing one or two hetero atoms selected from the group consisting of oxygen, sulfur and nitrogen as ring-forming atoms in one embodiment, a 6-membered saturated heterocyclic group containing one or two hetero atoms selected from the group consisting of oxygen, sulfur and nitrogen as ring-forming atoms in one embodiment, oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl or dioxothiomorpholinyl in one embodiment, oxetanyl, tetrahydrofuranyl, tetrahydropyranyl, azetidinyl, pyrrolidinyl, piperidinyl, piperazinyl, morpholinyl, thiomorpholinyl or dioxothiomorpholinyl in one embodiment, oxetanyl, tetrahydrofuranyl, tetrahydropyranyl, pyrrolidinyl, piperidinyl or morpholinyl in one embodiment, oxetanyl, tetrahydrofuranyl, tetrahydropyranyl, pyrrolidinyl or piperidinyl in one embodiment, oxetanyl, tetrahydrofuranyl or tetrahydropyranyl in one embodiment, pyrrolidinyl or piperidinyl in one embodiment, oxetanyl in one embodiment, tetrahydrofuranyl in one embodiment, tetrahydropyranyl in one embodiment, pyrrolidinyl in one embodiment, piperidinyl in one embodiment, morpholinyl in one embodiment or oxazolidinyl in one embodiment.

"Saturated heterocyclic divalent group containing one or two nitrogen atoms" is a 4-membered to 11-membered saturated heterocyclic divalent group containing one or two nitrogen atoms as ring-forming atoms and may be a saturated heterocyclic divalent group having a spiro ring and a fused ring. The "saturated heterocyclic divalent group containing one or two nitrogen atoms" in one embodiment is azetidinediyl, pyrrolidinediyl, imidazolidinediyl, piperidinediyl, piperazinediyl, azepanediyl, diazepanediyl, azocanediyl, diazocanediyl, azonanediyl, diazonanediyl or a divalent group represented by the following formulae (XXI) to (XXV).

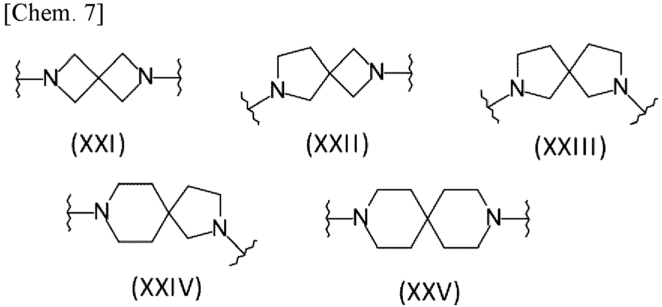

The "saturated heterocyclic divalent group containing one or two nitrogen atoms" in one embodiment is pyrrolidinediyl, piperazinediyl or a divalent group represented by the following formula (XXI) or (XXII).

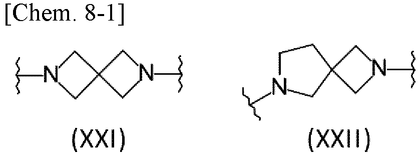

The "saturated heterocyclic divalent group containing one or two nitrogen atoms" in one embodiment is pyrrolidinediyl, piperazinediyl or a divalent group represented by the following formula (XXI), (XXII) or (XXV).

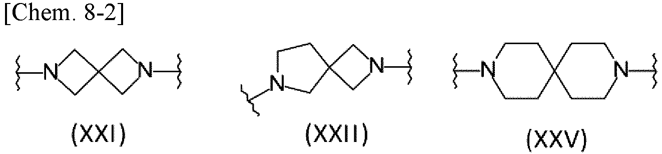

"Saturated heterocyclic divalent group containing two nitrogen atoms" is a 4-membered to 11-membered saturated heterocyclic divalent group containing two nitrogen atoms as ring-forming atoms and may be a saturated heterocyclic divalent group having a spiro ring and a fused ring.

The "saturated heterocyclic divalent group containing two nitrogen atoms" in one embodiment is imidazolidinediyl, piperazinediyl, diazepanediyl, diazocanediyl, diazonanediyl or a divalent group represented by the following formulae (XXI) to (XXV).

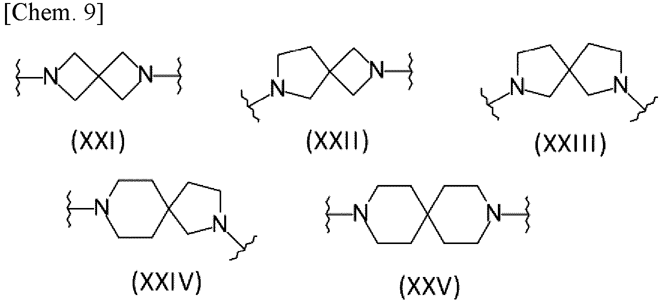

The "saturated heterocyclic divalent group containing two nitrogen atoms" in one embodiment is piperazinediyl or a divalent group represented by the following formula (XXI) or (XXII).

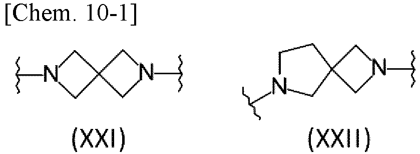

The "saturated heterocyclic divalent group containing two nitrogen atoms" in one embodiment is piperazinediyl or a divalent group represented by the following formula (XXI), (XXII) or (XXV).

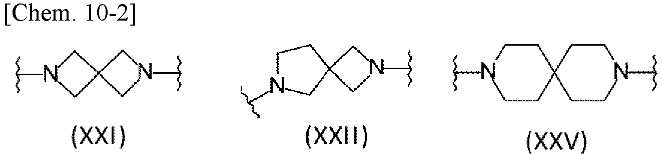

"Hetero ring" is an aromatic hydrocarbon ring containing hetero atom selected from the group consisting of oxygen, sulfur and nitrogen as ring-forming atoms.

Therefore, "6-membered hetero ring containing one or two nitrogen atoms" is a 6-membered aromatic hydrocarbon ring containing one or two nitrogen atoms as ring-forming atoms. The "6-membered hetero ring containing one or two nitrogen atoms" is a pyridine ring, a pyridazine ring, a pyrimidine ring, a pyrazine ring or a triazine ring in one embodiment or a pyridine ring in one embodiment.

"Heteroaryl" is a heterocyclic group containing hetero atoms selected from the group consisting of oxygen, sulfur and nitrogen as ring-forming atoms.

Therefore, "5-membered heteroaryl" is a 5-membered heterocyclic group containing one to four hetero atoms selected from the group consisting of oxygen, sulfur and nitrogen as ring-forming atoms.

The "5-membered heteroaryl" is a 5-membered heterocyclic group containing one to three hetero atoms selected from the group consisting of oxygen, sulfur and nitrogen as ring-forming atoms in one embodiment, pyrazolyl, imidazolyl, triazolyl, tetrazolyl, oxazolyl, isoxazolyl, thiazolyl, isothiazolyl, oxadiazolyl or thiadiazolyl in one embodiment, pyrazolyl, imidazolyl, triazolyl, oxazolyl or thiazolyl in one embodiment, pyrazolyl, imidazolyl, oxazolyl or thiazolyl in one embodiment, pyrazolyl, imidazolyl, triazolyl or isoxazolyl in one embodiment, pyrazolyl, oxazolyl or thiazolyl in one embodiment, pyrazolyl, triazolyl or isoxazolyl in one embodiment, pyrazolyl or thiazolyl in one embodiment, pyrazolyl or triazolyl in one embodiment, pyrazolyl in one embodiment, imidazolyl in one embodiment, oxazolyl in one embodiment, thiazolyl in one embodiment or triazolyl in one embodiment.

"6-Membered heteroaryl" is a 6-membered heterocyclic group containing one to three nitrogen atoms as ring-forming atoms. The "6-membered heteroaryl" is pyridyl, pyrimidinyl, pyrazinyl, pyridazinyl or triazinyl in one embodiment, pyridyl or pyridazinyl in one embodiment, pyridyl or pyrimidinyl in one embodiment, pyridyl in one embodiment or pyrimidinyl in one embodiment.

"Halogen" means F, Cl, Br and I. The "halogen" is F, Cl or Br in one embodiment, F or Cl in one embodiment, F or Br in one embodiment, F in one embodiment, Cl in one embodiment or Br in one embodiment.

"Spiro ring" means a polycyclic ring structure in which two ring structures are bonded with one shared spiro atom as a quaternary carbon, and "fused ring" means a polycyclic ring structure in which two or more ring structures are bonded with two or more adjacent atoms forming one of the rings being shared. "Bridged structure" means a divalent chain structure linked to two non-adjacent atoms among the ring-forming atoms in one ring.

Substituents acceptable in "optionally substituted C₁₋₆ alkyl", "optionally substituted C₁₋₃ alkyl" and "optionally substituted C₁₋₃ alkylene" are F, OH, OCH₃, N(C₁₋₃ alkyl optionally substituted with F)₂, optionally substituted C₃₋₆ cycloalkyl, azabicyclo[3.3.0]octanyl or an optionally substituted 4-membered to 6-membered saturated heterocyclic group in one embodiment, F, OH, OCH₃, cyclopropyl, N(C₁₋₃ alkyl optionally substituted with F)₂, pyrrolidinyl optionally substituted with (C₁₋₃ alkyl optionally substituted with F) and tetrahydrofuranyl in one embodiment, F, OH, OCH₃, N(CH₃)₂, hydroxymethyl, methoxymethyl, difluoroethyl, optionally substituted cyclopropyl, tetrahydrofuranyl, optionally substituted tetrahydropyranyl, morpholinyl, optionally substituted pyrrolidinyl or optionally substituted piperidinyl in one embodiment, F, OH, OCH₃, N(CH₃)₂, hydroxymethyl, methoxymethyl, optionally substituted cyclopropyl, tetrahydrofuranyl, optionally substituted tetrahydropyranyl or optionally substituted pyrrolidinyl in one embodiment, F, OH, OCH₃, N(CH₃)₂, hydroxymethyl, methoxymethyl, cyclopropyl, (hydroxymethyl)cyclopropyl, (methoxymethyl)cyclopropyl, tetrahydrofuranyl, tetrahydropyranyl, (hydroxymethyl)tetrahydropyranyl, (methoxymethyl)tetrahydropyranyl, pyrrolidinyl or methylpyrrolidinyl in one embodiment, F, OH, OCH₃, (methoxymethyl)cyclopropyl, tetrahydrofuranyl or methylpyrrolidinyl in one embodiment, F, OH or cyclopropyl in one embodiment, F, OH or OCH₃ in one embodiment, OH or OCH₃ in one embodiment, F or OCH₃ in one embodiment, OH in one embodiment, F in one embodiment or OCH₃ in one embodiment.

Substituent acceptable in "optionally substituted 5-membered heteroaryl" and "optionally substituted 6-membered heteroaryl" are C₁₋₃ alkyl optionally substituted with a group selected from the group consisting of OH and OCH₃, -SO₂CH₃, halogen, OH, OCH₃ or C₃₋₆ cycloalkyl in one embodiment, C₁₋₃ alkyl optionally substituted with a group selected from the group consisting of OH and OCH₃ in one embodiment, C₁₋₃ alkyl optionally substituted with OH in one embodiment, C₁₋₃ alkyl optionally substituted with OCH₃ in one embodiment, C₁₋₃ alkyl or halogen in one embodiment, methyl, ethyl, methoxymethyl or F in one embodiment or methyl, ethyl or F in one embodiment.

Substituents acceptable in "optionally substituted 4-membered to 6-membered saturated heterocyclic group", "optionally substituted pyrrolidinyl", "optionally substituted C₃₋₆ cycloalkyl", "optionally substituted saturated heterocyclic divalent group containing one or two nitrogen atoms" and " optionally substituted saturated heterocyclic divalent group containing 2 nitrogen atoms" are C₁₋₃ alkyl optionally substituted with a group selected from the group consisting of F, OH, OCH₃ and N(CH₃)₂, F, OH, OCH₃, oxo or oxetanyl in one embodiment, F, OH or OCH₃ in one embodiment, OH or methyl in one embodiment, C₁₋₃ alkyl optionally substituted with a group selected from the group consisting of F, OH and OCH₃, F, oxo or oxetanyl in one embodiment, C₁₋₃ alkyl optionally substituted with a group selected from the group consisting of F, OH, OCH₃ and N(CH₃)₂ or oxo in one embodiment, C₁₋₃ alkyl optionally substituted with a group selected from the group consisting of F, OH, OCH₃ and N(CH₃)₂ in one embodiment, C₁₋₃ alkyl optionally substituted with F in one embodiment, C₁₋₃ alkyl optionally substituted with OH in one embodiment, C₁₋₃ alkyl optionally substituted with OCH₃ in one embodiment, C₁₋₃ alkyl optionally substituted with N(CH₃)₂ in one embodiment, C₁₋₃ alkyl in one embodiment or C₁₋₃ alkyl optionally substituted with N(CH₃)₂ or oxo in one embodiment.

"C₁₋₃ Alkyl optionally substituted with OH" in one embodiment is methyl optionally substituted with one OH or ethyl optionally substituted with one or two OH. Examples thereof include methyl, ethyl, hydroxymethyl, 1-hydroxyethyl, 2-hydroxyethyl and 1,2-dihydroxyethyl. The "C₁₋₃ alkyl optionally substituted with OH" is methyl, ethyl or hydroxymethyl in one embodiment, methyl or hydroxymethyl in one embodiment, hydroxymethyl or hydroxyethyl in one embodiment, hydroxymethyl in one embodiment or hydroxyethyl in one embodiment.

"C₁₋₆ Alkyl optionally substituted with OCH₃" and "C₁₋₃ alkyl optionally substituted with OCH₃" in one embodiment are methyl optionally substituted with one OCH₃ or ethyl optionally substituted with one or two OCH₃. Examples thereof include methyl, ethyl, methoxymethyl, 1-methoxyethyl, 2-methoxyethyl and 1,2-dimethoxyethyl. The "C₁₋₆ alkyl optionally substituted with OCH₃" and "C₁₋₃ alkyl optionally substituted with OCH₃" are methoxymethyl or methoxyethyl in one embodiment, methoxymethyl in one embodiment or methoxyethyl in one embodiment.

"C₁₋₆ Alkyl optionally substituted with N(C₁₋₃ alkyl)₂" is "C₁₋₃ alkyl optionally substituted with N(CH₃)₂" in one embodiment or methyl optionally substituted with one N(C₁₋₃ alkyl)₂, ethyl optionally substituted with one N(C₁₋₃ alkyl)₂ or n-propyl optionally substituted with one N(C₁₋₃ alkyl)₂ in one embodiment. The C₁₋₆ alkyl optionally substituted with N(C₁₋₃ alkyl)₂ in one embodiment is methyl optionally substituted with one N(C₁₋₃ alkyl)₂ or ethyl optionally substituted with one N(C₁₋₃ alkyl)₂.

The "C₁₋₃ alkyl optionally substituted with N(CH₃)₂" in one embodiment is methyl optionally substituted with one N(CH₃)₂ or ethyl optionally substituted with one N(CH₃)₂. The C₁₋₃ alkyl optionally substituted with N(CH₃)₂ is methyl, ethyl, dimethylaminomethyl or dimethylaminoethyl in one embodiment, methyl or dimethylaminomethyl in one embodiment, dimethylaminomethyl in one embodiment or dimethylaminoethyl in one embodiment.

"Phenylene optionally substituted with F or Cl" in one embodiment is phenylene optionally substituted with one or two F or Cl. The phenylene optionally substituted with F or Cl is phenylene optionally substituted with one F in one embodiment, phenylene optionally substituted with one Cl in one embodiment, phenylene or fluorophenylene in one embodiment, phenylene in one embodiment, 2-fluoro-1,4-phenylene in one embodiment or 3-fluoro-1,4-phenylene in one embodiment.

"G12V Mutation" represents a mutation in which the amino acid residue corresponding to the codon 12 in a wildtype protein is converted from glycine to valine.

"G12V Mutant KRAS" represents KRAS having the above "G12V mutation".

"Pancreatic cancer" is a malignant tumor occurring in the pancreas. Examples thereof include pancreatic ductal carcinoma and pancreatic ductal adenocarcinoma, and the "pancreatic cancer" is pancreatic ductal carcinoma in one embodiment or pancreatic ductal adenocarcinoma in one embodiment. Moreover, the "pancreatic cancer" is metastatic pancreatic cancer in one embodiment, locally advanced pancreatic cancer in one embodiment, recurrent or refractory pancreatic cancer in one embodiment or pancreatic cancer of a patient who is untreated and/or has a treatment history in one embodiment.

"Lung cancer" is a malignant tumor occurring in the lung. Examples thereof include small-cell lung cancer and non-small-cell lung cancer, and the lung cancer is small-cell lung cancer in one embodiment or non-small-cell lung cancer in one embodiment. Moreover, the lung cancer is metastatic lung cancer in one embodiment, locally advanced lung cancer in one embodiment, recurrent or refractory lung cancer in one embodiment or lung cancer of a patient who is untreated and/or has a treatment history in one embodiment.

"G12V Mutant KRAS-positive pancreatic cancer" is pancreatic cancer that is positive for G12V mutant KRAS. Examples thereof include a pancreatic cancer in which the KRAS G12V mutation occurs and a pancreatic cancer which has a high positive rate for G12V mutant KRAS. The G12V mutant KRAS-positive pancreatic cancer is G12V mutant KRAS-positive pancreatic ductal carcinoma in one embodiment or G12V mutant KRAS-positive pancreatic ductal adenocarcinoma in one embodiment.

"G12V Mutant KRAS-positive lung cancer" is lung cancer that is positive for G12V mutant KRAS. Examples thereof include a lung cancer in which the KRAS G12V mutation occurs and a lung cancer which has a high positive rate for G12V mutant KRAS. The G12V mutant KRAS-positive lung cancer is G12V mutant KRAS-positive small-cell lung cancer in one embodiment or G12V mutant KRAS-positive non-small-cell lung cancer in one embodiment.

Embodiments of the compound of the formula (I) or a salt thereof of the present invention are shown below.
(1-1) The compound of the formula (I) or a salt thereof in which A is CR^{A} or N, and R^{A} is H or C₁₋₃ alkyl.
(1-2) The compound of the formula (I) or a salt thereof in which A is CR^{A} or N, and R^{A} is H.
(1-3) The compound of the formula (I) or a salt thereof in which A is CR^{A} or N, and R^{A} is C₁₋₃ alkyl.
(2-1) The compound of the formula (I) or a salt thereof in which X¹ is -CH₂-, -O- or -NR^{X1}-,
   R^{X1} is H or optionally substituted C₁₋₃ alkyl, and
   when X¹ is -NR^{X1}-, R^{X1} and R⁴ present on the same nitrogen atom, together with the nitrogen atom adjacent thereto, optionally form an optionally substituted 4-membered to 6-membered saturated heterocyclic group.
(2-2) The compound of the formula (I) or a salt thereof in which X¹ is -O- or -NR^{X1}-,
   R^{X1} is H or optionally substituted C₁₋₃ alkyl, and
   when X¹ is -NR^{X1}-, R^{X1} and R⁴ present on the same nitrogen atom, together with the nitrogen atom adjacent thereto, optionally form an optionally substituted 4-membered to 6-membered saturated heterocyclic group.
(2-3) The compound of the formula (I) or a salt thereof in which X¹ is -O- or -NR^{X1}-, and
   R^{X1} is H or C₁₋₃ alkyl.
(2-4) The compound of the formula (I) or a salt thereof in which X¹ is -O-.
(3-1) The compound of the formula (I) or a salt thereof in which R¹ is naphthyl optionally substituted with OH, or R¹ is the formula (II) or the formula (III) below, 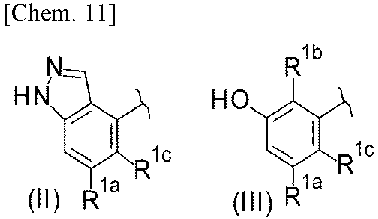
   R^{1a} and R^{1b}, which are the same as or different from each other, are H, methyl, F or Cl, and
   R^{1c} is F, Cl, methyl or ethyl.
(3-2) The compound of the formula (I) or a salt thereof in which R¹ is the formula (II) below, 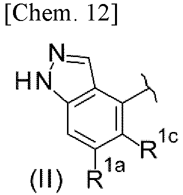
   R^{1a} is H, methyl, F or Cl, and
   R^{1c} is F, Cl, methyl or ethyl.
(3-3) The compound of the formula (I) or a salt thereof in which R¹ is the formula (II) below, 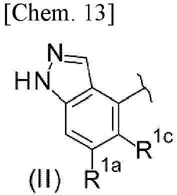
   R^{1a} is F, and
   R^{1c} is methyl.
(4-1) The compound of the formula (I) or a salt thereof in which R² is H, halogen, C₁₋₃ alkyl, cyclopropyl or vinyl, where the C₁₋₃ alkyl is optionally substituted with a group selected from the group consisting of OH and OCH₃.
(4-2) The compound of the formula (I) or a salt thereof in which R² is cyclopropyl or vinyl.
(4-3) The compound of the formula (I) or a salt thereof in which R² is cyclopropyl.
(5-1) The compound of the formula (I) or a salt thereof in which R³ is a group selected from the group consisting of the formula (IV), the formula (V), the formula (VI), the formula (VII), the formula (VIII), the formula (IX), the formula (X), the formula (XI), the formula (XII) and the formula (XXVI) below, 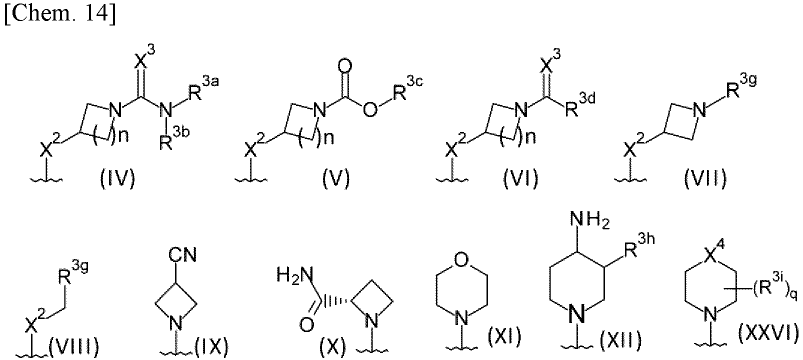
   R^{3a} is -(CH₂)ₚCHR^{3e}-NR^{N1}R^{N2}, -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2};
   R^{3b} is H or C₁₋₃ alkyl,
   R^{3c} and R^{3d} are -(CH₂)ₚCHR^{3e}-NR^{N1}R^{N2}, -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f} and -NR^{N1}R^{N2};
   R^{3e} is H, F or C₁₋₃ alkyl,
   R^{3f} is H or C₁₋₃ alkyl,
   R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
   R^{3h} is H, F or C₁₋₃ alkyl,
   each R³ⁱ, which is the same as or different from each other, is a group selected from the group consisting of H, OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, or
   two R³ⁱ present on the same carbon atom, together with the carbon atom adjacent thereto, optionally form a spiro ring having a ring selected from the group consisting of a C₃₋₆ cycloalkane and a 4-membered to 6-membered saturated hetero ring, where the spiro ring is optionally substituted with one or two groups selected from the group consisting of C₁₋₃ alkyl, -O-(C₁₋₃ alkyl), OH, halogen and oxo, or
   R³ⁱ present on two adjacent carbon atoms, together with the two carbon atoms, optionally forms a fused ring having a ring selected from the group consisting of a C₃₋₆ cycloalkane and a 4-membered to 6-membered saturated hetero ring, where the fused ring is optionally substituted with one or two groups selected from the group consisting of C₁₋₃ alkyl, -O-(C₁₋₃ alkyl), OH, halogen and oxo, or
   R³ⁱ present on two non-adjacent carbon atoms, together with the two carbon atoms, optionally forms a bridged structure composed of one or two carbon atoms, and a ring having the bridged structure is optionally substituted with one or two groups selected from the group consisting of C₁₋₃ alkyl, -O-(C₁₋₃ alkyl), OH, halogen and oxo,
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group, or
   R^{3e} and R^{N1}, together with the carbon atom and the nitrogen atom to which they are attached, optionally form an optionally substituted 4-membered to 6-membered saturated heterocyclic group,
   X² is -O-, -NH- or -N(C₁₋₃ alkyl)-,
   X³ is O or S,
   X⁴ is -CH₂-, -CH₂-CH₂- or -O-CH₂-,
   n is 1 or 2,
   p is 1 or 2, and
   q ranges from 1 to 8.
(5-2) The compound of the formula (I) or a salt thereof in which R³ is a group selected from the group consisting of the formula (IV), the formula (VII), the formula (VIII), the formula (IX), the formula (X), the formula (XI) and the formula (XII) below, 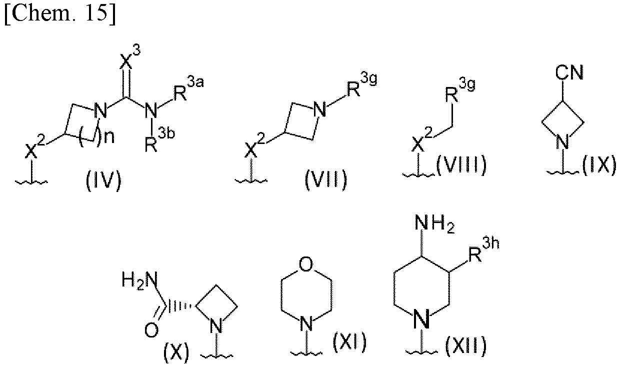
   R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2};
   R^{3b} is H or C₁₋₃ alkyl,
   R^{3e} is H, F or C₁₋₃ alkyl,
   R^{3f} is H or C₁₋₃ alkyl,
   R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
   R^{3h} is H, F or C₁₋₃ alkyl,
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group, or
   R^{3e} and R^{N1}, together with the carbon atom and the nitrogen atom to which they are attached, optionally form an optionally substituted 4-membered to 6-membered saturated heterocyclic group,
   X² is -O-, -NH- or -N(C₁₋₃ alkyl)-,
   X³ is O or S,
   n is 1 or 2, and
   p is 1 or 2.
(5-3) The compound of the formula (I) or a salt thereof in which R³ is a group selected from the group consisting of the formula (IV), the formula (VII), the formula (VIII), the formula (IX), the formula (X), the formula (XI) and the formula (XII) below, 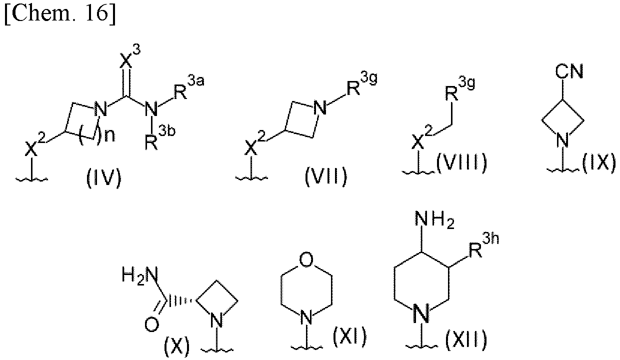
   R^{3a} is -(CH₂)ₚCHR^{3e}-NR^{N1}R^{N2},
   R^{3b} is H or C₁₋₃ alkyl,
   R^{3e} is H,
   R^{3g} is optionally substituted 6-membered heteroaryl,
   R^{3h} is H or F,
   R^{N1} and R^{N2}, which are the same as or different from each other, are C₁₋₃ alkyl, or
   R^{N1} and R^{N2}, together with the nitrogen atom to which they are attached, optionally form an optionally substituted 4-membered to 6-membered saturated heterocyclic group,
   X² is -O- or -NH-,
   X³ is O or S,
   n is 1, and
   p is 1.
(5-4) The compound of the formula (I) or a salt thereof in which R³ is a group selected from the group consisting of the formula (IV), the formula (VII), the formula (VIII), the formula (IX), the formula (X), the formula (XI) and the formula (XII) below, 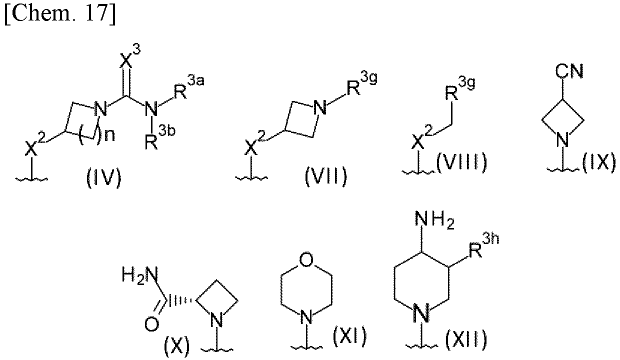
   R^{3a} is -(CH₂)ₚCHR^{3e}-NR^{N1}R^{N2},
   R^{3b} is H or C₁₋₃ alkyl,
   R^{3e} is H,
   R^{3g} is optionally substituted 6-membered heteroaryl,
   R^{3h} is H or F,
   R^{N1} and R^{N2}, which are the same as or different from each other, are C₁₋₃ alkyl,
   X² is -O- or -NH-,
   X³ is O or S,
   n is 1, and
   p is 1.
(5-5) The compound of the formula (I) or a salt thereof in which R³ is a group selected from the group consisting of the formula (IV), the formula (VII), the formula (VIII), the formula (IX), the formula (X), the formula (XI) and the formula (XII) below, 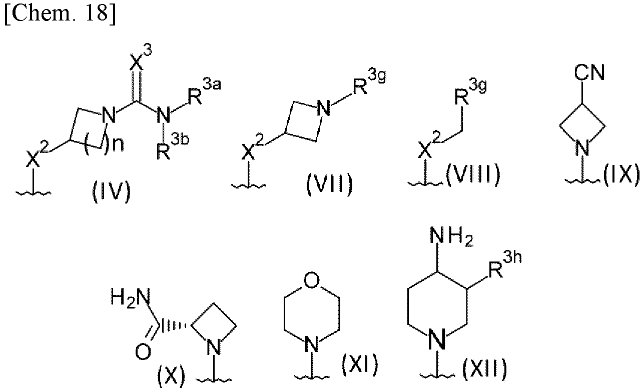
   R^{3a} is -(CH₂)ₚCHR^{3e}-NR^{N1}R^{N2},
   R^{3b} is H,
   R^{3e} is H,
   R^{3g} is optionally substituted 6-membered heteroaryl,
   R^{3h} is F,
   R^{N1} and R^{N2}, which are the same as or different from each other, are C₁₋₃ alkyl,
   X² is -O- or -NH-,
   X³ is O, and
   n and p are both 1.
(5-6) The compound of the formula (I) or a salt thereof in which R³ is a group selected from the group consisting of the formula (IV-1), the formula (VII-1), the formula (VIII-1), the formula (IX), the formula (X), the formula (XI) and the formula (XII-1) below. 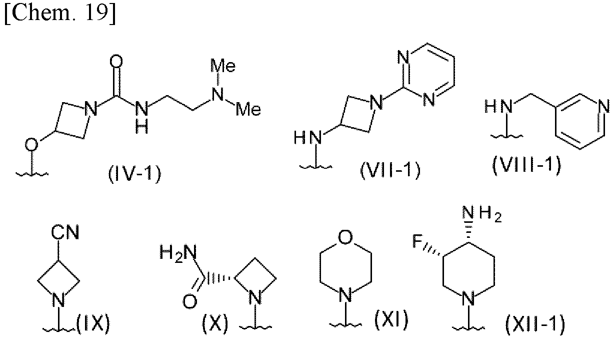
(6-1) The compound of the formula (I) or a salt thereof in which R⁴ is C₁₋₆ alkyl, piperidinyl optionally substituted with R^{4a} or tetrahydropyranyl, where the C₁₋₆ alkyl is optionally substituted with a group selected from the group consisting of F, OH, OCH₃, R^{4a}, cyclopropyl, N(R^{4a})₂, pyrrolidinyl optionally substituted with R^{4a} and tetrahydrofuranyl, and
   R^{4a} is optionally substituted C₁₋₃ alkyl.
(6-2) The compound of the formula (I) or a salt thereof in which R⁴ is C₁₋₆ alkyl optionally substituted with a group selected from the group consisting of OCH₃, N(R^{4a})₂ and pyrrolidinyl optionally substituted with R^{4a}, and
   R^{4a} is optionally substituted C₁₋₃ alkyl.
(6-3) The compound of the formula (I) or a salt thereof in which R⁴ is C₁₋₆ alkyl optionally substituted with OCH₃ or N(C₁₋₃ alkyl)₂, piperidinyl optionally substituted with C₁₋₃ alkyl or tetrahydropyranyl.
(6-4) The compound of the formula (I) or a salt thereof in which R⁴ is C₁₋₆ alkyl optionally substituted with a group selected from the group consisting of OCH₃, N(C₁₋₃ alkyl)₂ and pyrrolidinyl optionally substituted with R^{4a}, or tetrahydropyranyl, and
   R^{4a} is optionally substituted C₁₋₃ alkyl.
(7-1) The compound of the formula (I) or a salt thereof in which Y is phenylene optionally substituted with F or Cl or pyridinediyl.
(7-2) The compound of the formula (I) or a salt thereof in which Y is phenylene optionally substituted with F or Cl.
(7-3) The compound of the formula (I) or a salt thereof in which Y is phenylene.
(8-1) The compound of the formula (I) or a salt thereof in which L is -(L¹-L²-L³-L⁴-L⁵)-,
   L¹, L², L³, L⁴ and L⁵, which are the same as or different from each other, are groups selected from the group consisting of a bond, -O-, -NR^{L1}-, an optionally substituted saturated heterocyclic divalent group containing one or two nitrogen atoms, optionally substituted C₁₋₃ alkylene and C=O, and
   R^{L1} is H or C₁₋₃ alkyl.
(8-2) The compound of the formula (I) or a salt thereof in which L is -(L¹-L²-L³-L⁴-L⁵)-,
   L¹, L², L³, L⁴ and L⁵, which are the same as or different from each other, are groups selected from the group consisting of a bond, -NR^{L1}-, an optionally substituted saturated heterocyclic divalent group containing one or two nitrogen atoms, optionally substituted C₁₋₃ alkylene and C=O, and
   R^{L1} is H or C₁₋₃ alkyl.
(8-3) The compound of the formula (I) or a salt thereof in which L is -(L¹-L²-L³-L⁴-L⁵)-, and L¹ contained in L is attached to Y,
   L¹ is C₁₋₃ alkylene or C=O,
   L² is an optionally substituted saturated heterocyclic divalent group containing one or two nitrogen atoms,
   L³ is C₁₋₃ alkylene,
   L⁴ is a bond, -O- or -N(C₁₋₃ alkyl)-, and
   L⁵ is a bond or C₁₋₃ alkylene.
(8-4) The compound of the formula (I) or a salt thereof in which L is one group selected from the group consisting of the following formulae (XXVII) to (XXXIV), in which a carbon atom with * is attached to Y. 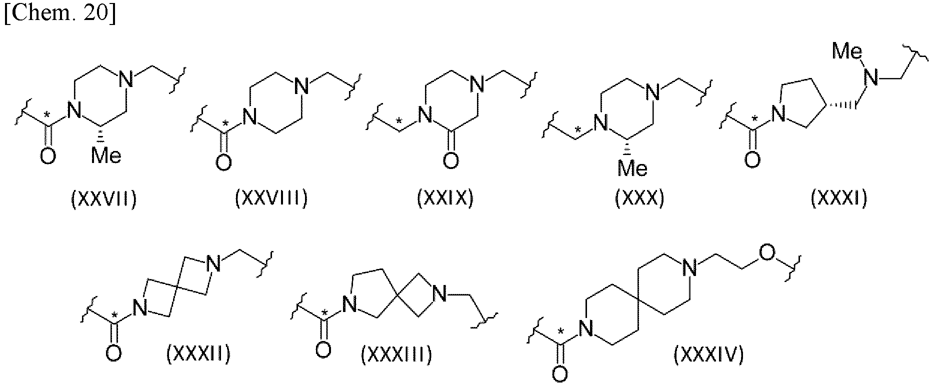
(9-1) The compound of the formula (I) or a salt thereof in which Z is a group selected from the group consisting of the formula (XIII), the formula (XIV), the formula (XV), the formula (XVI), the formula (XVII), the formula (XVIII), the formula (XIX) and the formula (XX) below, 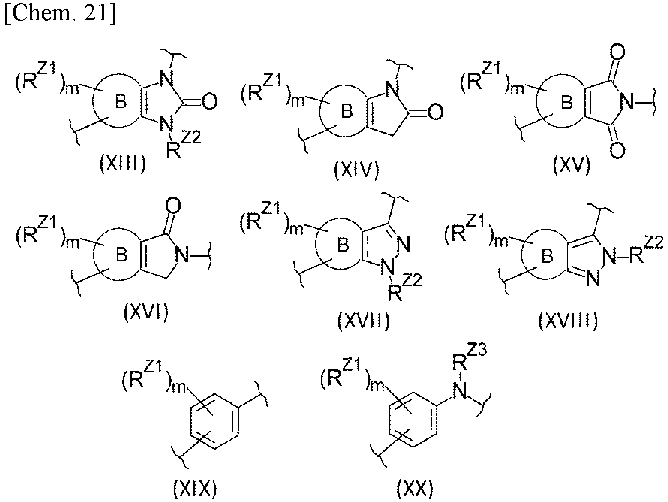
   ring B is a benzene ring or a 6-membered hetero ring containing one or two nitrogen atoms,
   R^{Z1} is H, C₁₋₃ alkyl, -O-(C₁₋₃ alkyl), -NR^{Z4}₂, -CONR^{Z4}₂ or -NR^{Z4}COR^{Z5},
   R^{Z2} is H or C₁₋₃ alkyl,
   R^{Z3} is H or C₁₋₃ alkyl,
   each R^{Z4}, which is the same as or different from each other, is H or C₁₋₃ alkyl,
   R^{Z5} is C₁₋₃ alkyl,
   L is attached to ring B in the formulae (XIII) to (XVIII) above or to the benzene ring in the formula (XIX) and the formula (XX), and
   m is 1 or 2.
(9-2) The compound of the formula (I) or a salt thereof in which Z is a group selected from the group consisting of the formula (XIII), the formula (XVII) and the formula (XIX) below, 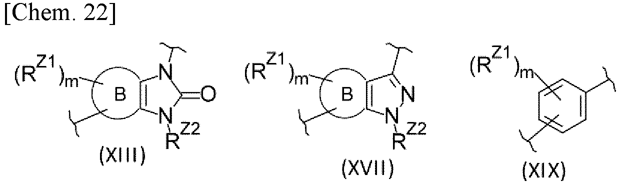
   ring B is a benzene ring or a 6-membered hetero ring containing one or two nitrogen atoms,
   R^{Z1} is H, C₁₋₃ alkyl, -O-(C₁₋₃ alkyl), -NR^{Z4}₂, -CONR^{Z4}₂ or -NR^{Z4}COR^{Z5},
   R^{Z2} is H or C₁₋₃ alkyl,
   each R^{Z4}, which is the same as or different from each other, is H or C₁₋₃ alkyl,
   R^{Z5} is C₁₋₃ alkyl,

   L is attached to ring B in the formula (XIII) or (XVII) above, and
   m is 1 or 2.
(9-3) The compound of the formula (I) or a salt thereof in which Z is a group selected from the group consisting of the formula (XIII), the formula (XVII) and the formula (XIX) below, 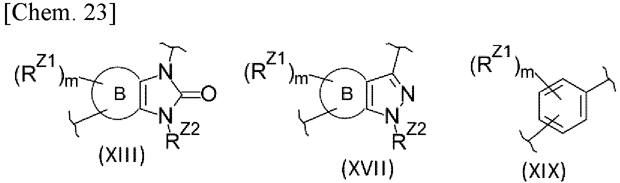
   ring B is a benzene ring,
   R^{Z1} is H or C₁₋₃ alkyl,
   R^{Z2} is H or C₁₋₃ alkyl,
   L is attached to ring B in the formula (XIII) or (XVII) above or to the benzene ring in the formula (XIX), and
   m is 1 or 2.
(9-4) The compound of the formula (I) or a salt thereof in which Z is a group selected from the group consisting of the formula (XIII), the formula (XVII) and the formula (XIX) below, 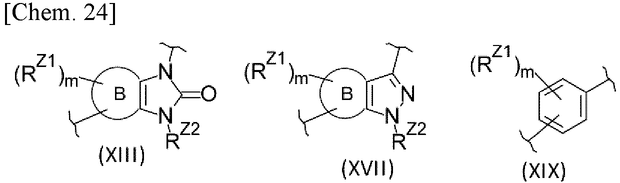
   ring B is a benzene ring,
   R^{Z1} is H,
   R^{Z2} is C₁₋₃ alkyl,
   L is attached to ring B in the formula (XIII) or (XVII) above or to the benzene ring in the formula (XIX), and
   m is 1.
(9-5) The compound of the formula (I) or a salt thereof in which Z is a group selected from the group consisting of the formula (XIII-1), the formula (XVII-1) and the formula (XIX-1) below, 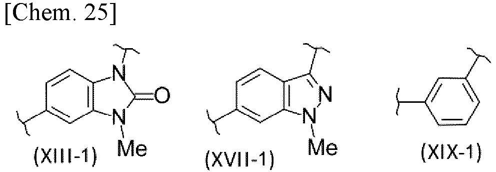 and
   L is attached to a benzene ring in the formula (XIII-1) and the formula (XVII-1) above.
(9-6) The compound of the formula (I) or a salt thereof in which Z is the formula (XIII) or the formula (XVII) below, 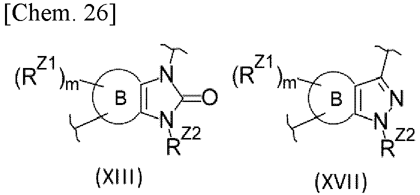
   ring B is a benzene ring,
   L is attached to ring B in the formula (XIII) and (XVII) above,
   R^{Z1} is H,
   R^{Z2} is C₁₋₃ alkyl, and
   m is 1.
(10-1) The compound of the formula (I) or a salt thereof in which G is CH or N,
   provided that when G is N, Z is the formula (XVII), the formula (XVIII) or the formula (XIX) above.
(10-2) The compound of the formula (I) or a salt thereof in which G is CH or N, provided that when G is N, Z is the formula (XVII) or the formula (XIX) above.
(10-3) The compound of the formula (I) or a salt thereof in which G is CH or N, provided that when G is N, Z is the formula (XVII-1) or the formula (XIX-1) above.
(10-4) The compound of the formula (I) or a salt thereof in which G is CH or N, provided that when G is N, Z is the formula (XVII) above.
(11) The compound or a salt thereof which is a combination of any two or more of the embodiments compatible with each other described in (1-1) to (10-4) above.

Specific examples of the combination described in (11) above include the following embodiments.
(11-1-1) The compound of the formula (I) or a salt thereof. 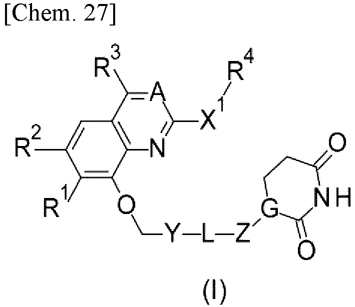 (In the formula,
   A is CR^{A} or N,
   R^{A} is H,
   X¹ is -O-,
   R¹ is the formula (II) below, 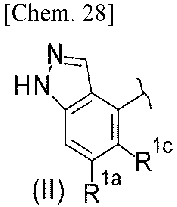
   R^{1a} is F,
   R^{1c} is methyl,
   R² is cyclopropyl,
   R³ is a group selected from the group consisting of the formula (IV), the formula (VII), the formula (VIII), the formula (IX), the formula (X), the formula (XI) and the formula (XII) below, 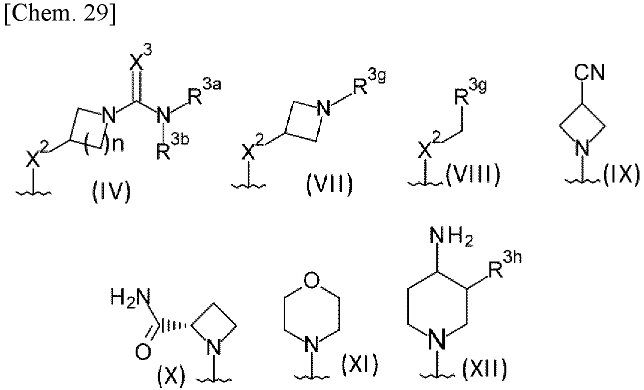
   R^{3a} is -(CH₂)ₚCHR^{3e}-NR^{N1}R^{N2},
   R^{3b} is H,
   R^{3e} is H,
   R^{3g} is optionally substituted 6-membered heteroaryl,
   R^{3h} is F,
   R^{N1} and R^{N2}, which are the same as or different from each other, are C₁₋₃ alkyl,
   X² is -O- or -NH-,
   X³ is O,
   n and p are both 1,
   R⁴ is C₁₋₆ alkyl optionally substituted with a group selected from the group consisting of OCH₃, N(R^{4a})₂ and pyrrolidinyl optionally substituted with R^{4a},
   R^{4a} is optionally substituted C₁₋₃ alkyl,
   Y is phenylene,
   L is -(L¹-L²-L³-L⁴-L⁵).
   L¹, L², L³, L⁴ and L⁵, which are the same as or different from each other, are groups selected from the group consisting of a bond, -NR^{L1}-, an optionally substituted saturated heterocyclic divalent group containing one or two nitrogen atoms, optionally substituted C₁₋₃ alkylene and C=O, and
   R^{L1} is H or C₁₋₃ alkyl,
   Z is the formula (XIII) or the formula (XVII) below, 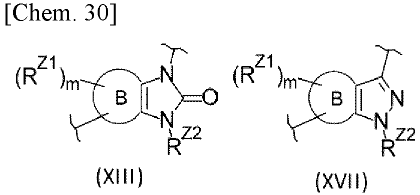
   ring B is a benzene ring,
   L is attached to ring B in the formula (XIII) and (XVII) above,
   R^{Z1} is H,
   R^{Z2} is C₁₋₃ alkyl,
   m is 1, and
   G is CH or N,
   provided that when G is N, Z is the formula (XVII) above.)
(11-1-2) A compound of the formula (I) or a salt thereof, 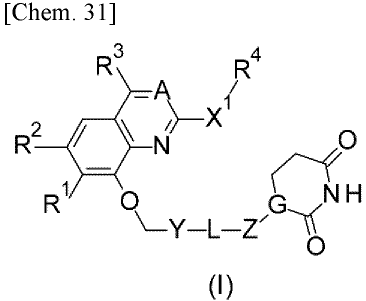 (wherein in the formula,
   A is CR^{A} or N,
   R^{A} is H or C₁₋₃ alkyl,
   X¹ is -CH₂-, -O- or -NR^{X1}-,
   R^{X1} is H or optionally substituted C₁₋₃ alkyl,
   when X¹ is -NR^{X1}-, R^{X1} and R⁴ present on the same nitrogen atom, together with a nitrogen atom adjacent thereto, optionally form an optionally substituted 4-membered to 6-membered saturated heterocyclic group,
   R¹ is naphthyl optionally substituted with OH, or R¹ is the formula (II) or the formula (III) below, 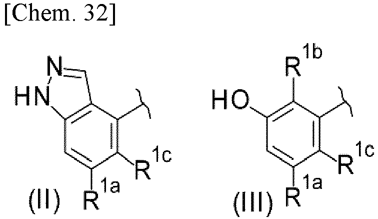
   R^{1a} and R^{1b}, which are the same as or different from each other, are H, methyl, F or Cl,
   R^{1c} is F, Cl, methyl or ethyl,
   R² is H, halogen, C₁₋₃ alkyl, cyclopropyl or vinyl, where the C₁₋₃ alkyl is optionally substituted with a group selected from the group consisting of OH and OCH₃,
   R³ is a group selected from the group consisting of the formula (IV), the formula (V), the formula (VI), the formula (VII), the formula (VIII), the formula (IX), the formula (X), the formula (XI), the formula (XII) and the formula (XXVI) below, 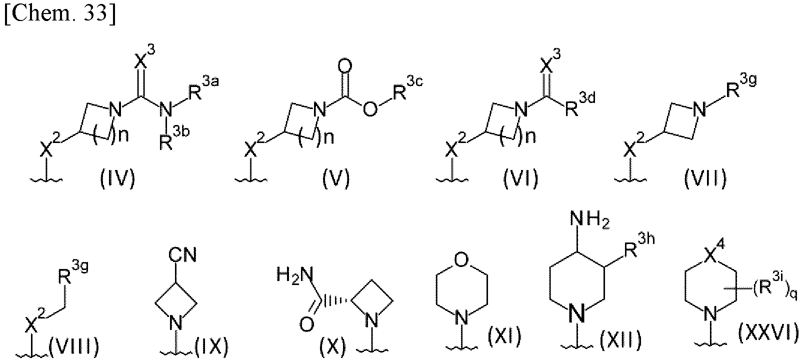
   R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2};
   R^{3b} is H or C₁₋₃ alkyl,
   R^{3c} and R^{3d} are -(CH₂)ₚCHR^{3e}-NR^{N1}R^{N2}, -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f} and -NR^{N1}R^{N2};
   R^{3e} is H, F or C₁₋₃ alkyl,
   R^{3f} is H or C₁₋₃ alkyl,
   R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
   R^{3h} is H, F or C₁₋₃ alkyl,
   each R³ⁱ, which is the same as or different from each other, is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
   two R³ⁱ present on the same carbon atom, together with the carbon atom adjacent thereto, optionally form a spiro ring having a ring selected from the group consisting of a C₃₋₆ cycloalkane and a 4-membered to 6-membered saturated hetero ring, where the spiro ring is optionally substituted with one or two groups selected from the group consisting of C₁₋₃ alkyl, -O-(C₁₋₃ alkyl), OH, halogen and oxo, or
   R³ⁱ present on two adjacent carbon atoms, together with the two carbon atoms, optionally forms a fused ring having a ring selected from the group consisting of a C₃₋₆ cycloalkane and a 4-membered to 6-membered saturated hetero ring, where the fused ring is optionally substituted with one or two groups selected from the group consisting of C₁₋₃ alkyl, -O-(C₁₋₃ alkyl), OH, halogen and oxo, or
   R³ⁱ present on two non-adjacent carbon atoms, together with the two carbon atoms, optionally forms a bridged structure composed of one or two carbon atoms, and a ring having the bridged structure is optionally substituted with one or two groups selected from the group consisting of C₁₋₃ alkyl, -O-(C₁₋₃ alkyl), OH, halogen and oxo,
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group, or
   R^{3e} and R^{N1}, together with the carbon atom and the nitrogen atom to which they are attached, optionally form an optionally substituted 4-membered to 6-membered saturated heterocyclic group,
   X² is -O-, -NH- or -N(C₁₋₃ alkyl)-,
   X³ is O or S,
   X⁴ is -CH₂-, -CH₂-CH₂- or -O-CH₂-,
   n is 1 or 2,
   p is 1 or 2,
   q ranges from 1 to 8,
   R⁴ is C₁₋₆ alkyl, piperidinyl optionally substituted with R^{4a} or tetrahydropyranyl, where the C₁₋₆ alkyl is optionally substituted with a group selected from the group consisting of F, OH, OCH₃, R^{4a}, cyclopropyl, N(R^{4a})₂, pyrrolidinyl optionally substituted with R^{4a}and tetrahydrofuranyl,
   R^{4a} is optionally substituted C₁₋₃ alkyl,
   Y is phenylene optionally substituted with F or Cl or pyridinediyl,
   L is -(L¹-L²-L³-L⁴-L⁵).
   L¹, L², L³, L⁴ and L⁵, which are the same as or different from each other, are groups selected from the group consisting of a bond, -O-, -NR^{L1}-, an optionally substituted saturated heterocyclic divalent group containing one or two nitrogen atoms, optionally substituted C₁₋₃ alkylene and C=O,
   R^{L1} is H or C₁₋₃ alkyl,
   Z is a group selected from the group consisting of the formula (XIII), the formula (XIV), the formula (XV), the formula (XVI), the formula (XVII), the formula (XVIII), the formula (XIX) and the formula (XX) below, 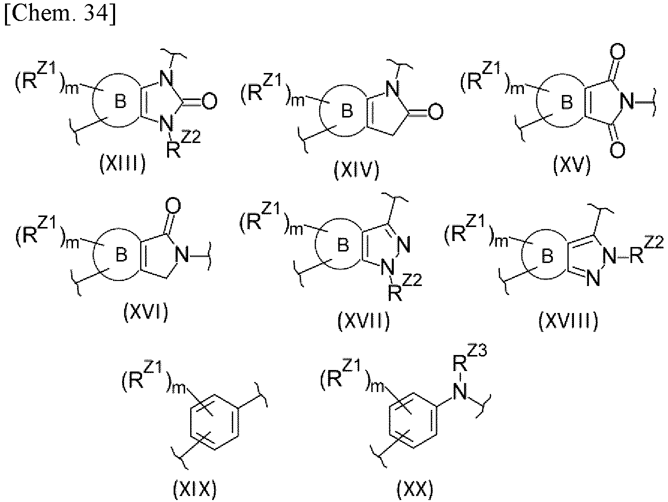
   ring B is a benzene ring or a 6-membered hetero ring containing one or two nitrogen atoms,
   R^{Z1} is H, C₁₋₃ alkyl, -O-(C₁₋₃ alkyl), -NR^{Z4}₂, -CONR^{Z4}₂ or -NR^{Z4}COR^{Z5},
   R^{Z2} is H or C₁₋₃ alkyl,
   R^{Z3} is H or C₁₋₃ alkyl,
   each R^{Z4}, which is the same as or different from each other, is H or C₁₋₃ alkyl,
   R^{Z5} is C₁₋₃ alkyl,
   L is attached to ring B in the formulae (XIII) to (XVIII) above or to the benzene ring in the formula (XIX) and the formula (XX),
   m is 1 or 2, and
   G is CH or N,
   provided that when G is N, Z is the formula (XVII), the formula (XVIII) or the formula (XIX) above).
(11-2) The compound or a salt thereof according to (11-1-2) above,
   wherein X¹ is -O- or -NR^{X1}-,
   R^{X1} is H or optionally substituted C₁₋₃ alkyl,
   when X¹ is -NR^{X1}-, R^{X1} and R⁴ present on the same nitrogen atom, together with a nitrogen atom adjacent thereto, optionally form an optionally substituted 4-membered to 6-membered saturated heterocyclic group,
   R¹ is the formula (II) below, 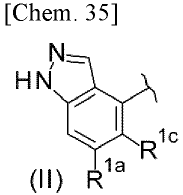
   R^{1a} is H, methyl, F or Cl,
   R^{1c} is F, Cl, methyl or ethyl,
   R² is cyclopropyl or vinyl,
   R³ is a group selected from the group consisting of the formula (IV), the formula (VII), the formula (VIII), the formula (IX), the formula (X), the formula (XI) and the formula (XII) below, 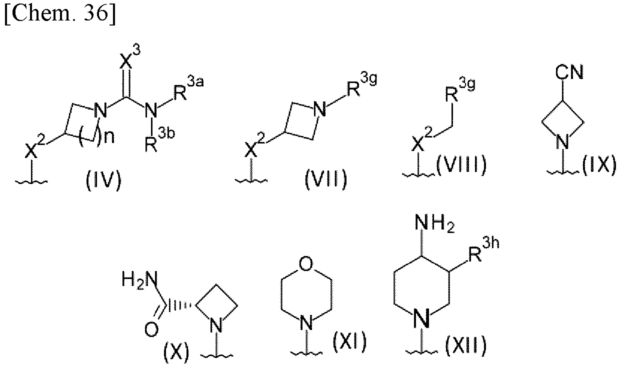
   R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}; or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2};
   R^{3b} is H or C₁₋₃ alkyl,
   R^{3e} is H, F or C₁₋₃ alkyl,
   R^{3f} is H or C₁₋₃ alkyl,
   R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
   R^{3h} is H, F or C₁₋₃ alkyl,
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group, or
   R^{3e} and R^{N1}, together with the carbon atom and the nitrogen atom to which they are attached, optionally form an optionally substituted 4-membered to 6-membered saturated heterocyclic group,
   X² is -O-, -NH- or -N(C₁₋₃ alkyl)-,
   X³ is O or S,
   n is 1 or 2,
   p is 1 or 2,
   Y is phenylene optionally substituted with F or Cl,
   Z is a group selected from the group consisting of the formula (XIII), the formula (XVII) and the formula (XIX) below, 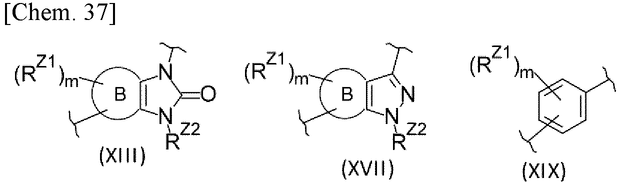
   ring B is a benzene ring or a 6-membered hetero ring containing one or two nitrogen atoms,
   R^{Z1} is H, C₁₋₃ alkyl, -O-(C₁₋₃ alkyl), -NR^{Z4}₂, -CONR^{Z4}₂ or -NR^{Z4}COR^{Z5},
   R^{Z2} is H or C₁₋₃ alkyl,
   each R^{Z4}, which is the same as or different from each other, is H or C₁₋₃ alkyl,
   R^{Z5} is C₁₋₃ alkyl,
   L is attached to ring B in the formula (XIII) or (XVII) above,
   m is 1 or 2, and
   G is CH or N,
   provided that when G is N, Z is the formula (XVII) or the formula (XIX) above.
(11-3) The compound or a salt thereof according to (11-2) above, in which X¹ is -O- or -NR^{X1}-,
   R^{X1} is H or C₁₋₃ alkyl,
   R³ is a group selected from the group consisting of the formula (IV), the formula (VII), the formula (VIII), the formula (IX), the formula (X), the formula (XI) and the formula (XII) below, 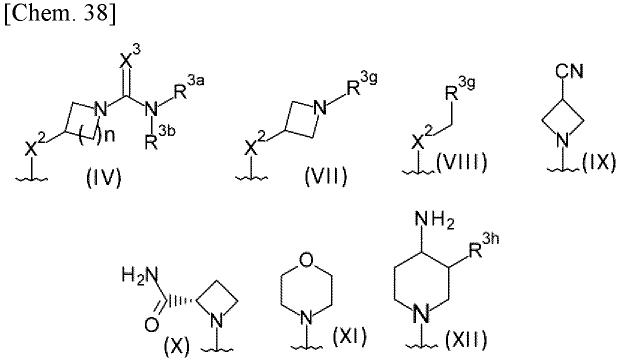
   R^{3a} is -(CH₂)ₚCHR^{3e}-NR^{N1}R^{N2},
   R^{3b} is H or C₁₋₃ alkyl,
   R^{3e} is H,
   R^{3g} is optionally substituted 6-membered heteroaryl,
   R^{3h} is H or F,
   R^{N1} and R^{N2}, which are the same as or different from each other, are C₁₋₃ alkyl, or
   R^{N1} and R^{N2}, together with the nitrogen atom to which they are attached, optionally form an optionally substituted 4-membered to 6-membered saturated heterocyclic group,
   X² is -O- or -NH-,
   X³ is O or S,
   n is 1,
   p is 1,
   Z is a group selected from the group consisting of the formula (XIII), the formula (XVII) and the formula (XIX) below, 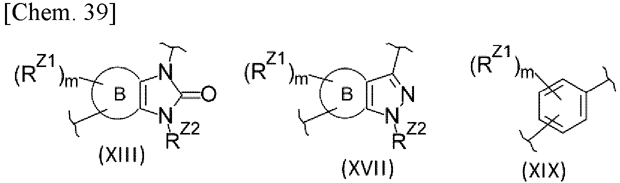
   ring B is a benzene ring,
   R^{Z1} is H or C₁₋₃ alkyl,
   R^{Z2} is H or C₁₋₃ alkyl,
   L is attached to ring B in the formula (XIII) or (XVII) above or to the benzene ring in the formula (XIX),
   m is 1 or 2, and
   G is CH or N,
   provided that when G is N, Z is the formula (XVII) or the formula (XIX) above.
(11-4) The compound or a salt thereof according to (11-2) above,
   wherein A is CR^{A} or N,
   R^{A} is H,
   X¹ is -O-,
   R¹ is the formula (II) below, 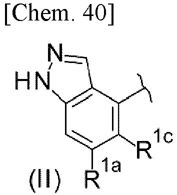
   R^{1a} is F,
   R^{1c} is methyl,
   R² is cyclopropyl,
   R³ is a group selected from the group consisting of the formula (IV), the formula (VII), the formula (VIII), the formula (IX), the formula (X), the formula (XI) and the formula (XII) below, 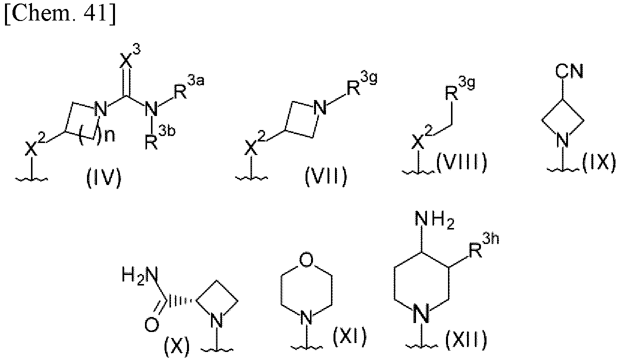
   R^{3a} is -(CH₂)ₚCHR^{3e}-NR^{N1}R^{N2},
   R^{3b} is H or C₁₋₃ alkyl,
   R^{3e} is H,
   R^{3g} is optionally substituted 6-membered heteroaryl,
   R^{3h} is H or F,
   R^{N1} and R^{N2}, which are the same as or different from each other, are C₁₋₃ alkyl,
   X² is -O- or -NH-,
   X³ is O or S,
   n is 1,
   p is 1,
   R⁴ is C₁₋₆ alkyl optionally substituted with a group selected from the group consisting of OCH₃, N(C₁₋₃ alkyl)₂ and pyrrolidinyl optionally substituted with R^{4a} or tetrahydropyranyl,
   R^{4a} is optionally substituted C₁₋₃ alkyl,
   Y is phenylene,
   L is -(L¹-L²-L³-L⁴-L⁵)-, and L¹ contained in L is attached to Y,
   L¹ is C₁₋₃ alkylene or C=O,
   L² is an optionally substituted saturated heterocyclic divalent group containing one or two nitrogen atoms,
   L³ is C₁₋₃ alkylene,
   L⁴ is a bond, -O- or -N(C₁₋₃ alkyl)-,
   L⁵ is a bond or C₁₋₃ alkylene,
   Z is a group selected from the group consisting of the formula (XIII), the formula (XVII) and the formula (XIX) below, 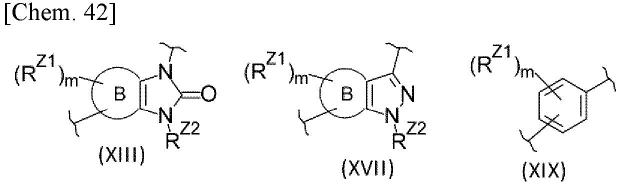
   ring B is a benzene ring,
   R^{Z1} is H or C₁₋₃ alkyl,
   R^{Z2} is H or C₁₋₃ alkyl,
   L is attached to ring B in the formula (XIII) or (XVII) above or to the benzene ring in the formula (XIX), and
   m is 1 or 2.
(11-5) The compound or a salt thereof according to (11-4) above, wherein R³ is a group selected from the group consisting of the formula (IV-1), the formula (VII-1), the formula (VIII-1), the formula (IX), the formula (X), the formula (XI) and the formula (XII-1) below, 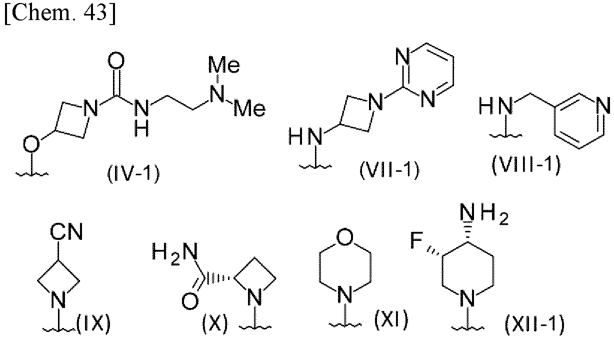
   L is one group selected from the group consisting of the following formulae (XXVII) to (XXXIV), wherein a carbon atom with * is attached to Y, 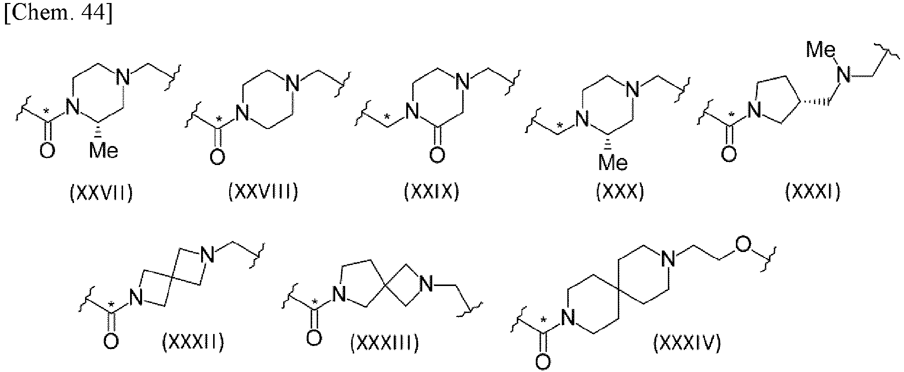
   Z is a group selected from the group consisting of the formula (XIII-1), the formula (XVII-1) and the formula (XIX-1), 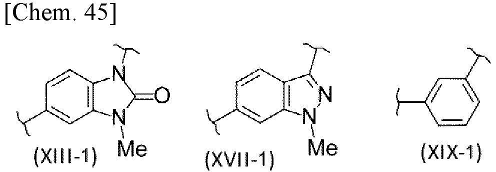
   L is attached to a benzene ring in the formula (XIII-1) and the formula (XVII-1) above, and
   G is CH or N,
   provided that when G is N, Z is the formula (XVII-1) or the formula (XIX-1) above.

Examples of specific compounds included in the present invention include the following compounds or salts thereof in one embodiment:
1-{6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-3-carbonitrile and
(2S)-1-{6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-2-carboxamide.

Examples of specific compounds included in the present invention include the following compounds or salts thereof in one embodiment:
1-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-3-carbonitrile and
(2S)-1-{(7M)-6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-2-carboxamide.

Examples of specific compounds included in the present invention include the following compounds or salts thereof in one embodiment:
1-{(7P)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-3-carbonitrile and
(2S)-1-{(7P)-6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-2-carboxamide.

The compound of the formula (I) may have tautomers or geometrical isomers depending on the type of the substituent. In this specification, the compound of the formula (I) is sometimes described only as one of isomers, but the present invention includes isomers other than the above one and includes separated isomers or mixtures thereof.

In addition, the compound of the formula (I) may have an asymmetric carbon atom or an axial chirality and may have diastereomers based on them. The present invention includes separated diastereomers of the compound of the formula (I) or mixtures thereof.

Furthermore, the present invention also includes a pharmaceutically acceptable prodrug of the compound represented by the formula (I). The pharmaceutically acceptable prodrug is a compound having a group that can be converted into an amino group, a hydroxy group, a carboxyl group or the like by solvolysis or under physiological conditions. Examples of a prodrug-forming group include groups described in Prog. Med., 5, 2157-2161 (1985) and "Pharmaceutical Research and Development" (Hirokawa Shoten, 1990), Vol. 7, Molecular Design, 163-198.

In addition, the salt of the compound of the formula (I) is a pharmaceutically acceptable salt of the compound of the formula (I) and may be an acid addition salt or a salt formed with a base depending on the type of the substituent. Examples thereof include salts shown in P. Heinrich Stahl, Handbook of Pharmaceutical Salts Properties, Selection, and Use, Wiley-VCH, 2008. Specific examples include an acid addition salt with an inorganic acid, such as hydrochloric acid, hydrobromic acid, hydroiodic acid, sulfuric acid, nitric acid or phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoiltartaric acid, ditoluoyltartaric acid, citric acid, methanesulfonic acid, ethanesulfonic acid, benzenesulfonic acid, p-toluenesulfonic acid, aspartic acid or glutamic acid, a salt with an inorganic metal, such as sodium, potassium, magnesium, calcium or aluminum, a salt with an organic base, such as methylamine, ethylamine or ethanolamine, a salt with various amino acids and amino acid derivatives, such as acetylleucine, lysine or ornithine, an ammonium salt and the like.

Furthermore, the present invention also includes various hydrates, solvates and crystal polymorphism substances of the compound of the formula (I) and a salt thereof.

The present invention also includes all the compounds of the formula (I) or salts thereof which are labeled with one or more pharmaceutically acceptable radioactive or nonradioactive isotopes. Examples of suitable isotopes used for isotopic labeling of the compound of the present invention include isotopes of hydrogen (²H, ³H and the like), carbon (¹¹C, ¹³C, ¹⁴C and the like), nitrogen (¹³N, ¹⁵N and the like), oxygen (¹⁵O, ¹⁷O, ¹⁸O and the like), fluorine (¹⁸F and the like), chlorine (³⁶Cl and the like), iodine (¹²³I, ¹²⁵I and the like) and sulfur (³⁵S and the like).

The isotope-labeled compound of the invention of the present application can be used for research and the like such as research on tissue distribution of drugs and/or substrates. For example, radioactive isotopes such as tritium (³H) and carbon 14 (¹⁴C) can be used for this purpose due to the easiness of labeling and the convenience of detection.

Substitution by heavier isotope, for example, substitution of hydrogen by deuterium (²H), is therapeutically advantageous through the improvement of metabolic stability in some cases (for example, increase in the *in vivo* half-life, decrease in the required dose or decrease in the interaction between drugs).

Substitution by positron-emitting isotope (¹¹C, ¹⁸F, ¹⁵O, ¹³N or the like) can be used in a positron emission tomography (PET) test for testing occupancy of a substrate receptor.

The isotope-labeled compound of the present invention can be generally produced by a conventional method known to a person skilled in the art or by the same production methods as in the Examples or the Production Examples and the like using suitable reagents which are labeled with an isotope in place of unlabeled reagents.

### (Production method)

The compound of the formula (I) and a salt thereof can be produced by applying various known synthetic methods using characteristics based on the basic structure or the type of substituent thereof. Here, depending on the type of functional group, it is sometimes effective as a production technique to substitute the functional group with an appropriate protective group (a group that can be easily converted to the functional group) in the process from a raw material to an intermediate. Examples of the protective group include protective groups described in P. G. M. Wuts and T. W. Greene, "Greene's Protective Groups in Organic Synthesis", 5th edition, John Wiley & Sons Inc., 2014 and the like, and a group appropriately selected from the protective groups is used depending on the reaction conditions. In such a method, a reaction is carried out with the protective group introduced, and then the protective group is removed, as required, whereby a desired compound can be obtained.

The pharmaceutically acceptable prodrug is a compound having a group that can be converted into an amino group, a hydroxy group, a carboxyl group or the like by solvolysis or under physiological conditions. Examples of a prodrug-forming group include groups described in Prog. Med., 5, 2157-2161 (1985) and "Pharmaceutical Research and Development" (Hirokawa Shoten, 1990), Vol. 7, Molecular Design, 163-198.

In addition, a prodrug of the compound of the formula (I) can be produced similarly to the protective group by introducing a specific group in the process from a raw material to an intermediate or by further performing the reaction using the resulting compound of the formula (I). This reaction can be performed by applying a method known to a person skilled in the art, such as common esterification, amidation and dehydration.

Typical methods for producing the compound of the formula (I) will be described below. The production methods can also be carried out with reference to a reference attached to the description. Note that the production method of the present invention is not limited to the examples described below.

In this specification, the following abbreviations are sometimes used.

DMF: N,N-dimethylformamide, DMAc: N,N-dimethylacetamide, THF: tetrahydrofuran, MeCN: acetonitrile, MeOH: methanol, EtOH: ethanol, iPrOH: isopropyl alcohol, tBuOH: tert-butanol, DOX: 1,4-dioxane, DMSO: dimethyl sulfoxide, TEA: triethylamine, DIPEA: N,N-diisopropylethylamine, tBuOK: potassium tert-butoxide, PdCl₂(dppf)·CH₂Cl₂: [1,1'-bis(diphenylphosphino)ferrocene]palladium(II) dichloride·dichloromethane adduct, Pd/C: palladium-loaded carbon, PyBOP: (benzotriazol-1-yloxy)tripyrrolidinophosphonium hexafluorophosphate, PyAOP: (7-azabenzotriazol-1-yloxy)trispyrrolidinophosphonium hexafluorophosphate, SFC: supercritical fluid chromatography, NMM: N-methylmorpholine, CDI: 1,1'-carbonyldiimidazole, HATU: O-(7-azabenzotriazol-1-yl)-N,N,N',N'-tetramethyluronium hexafluorophosphate, DABCO: 1,4-diazabicyclo[2.2.2]octane, TFA: trifluoroacetic acid, DBU: 1,8-diazabicyclo[5.4.0]-7-undecene, TBAF: tetra-n-butylammonium fluoride.

### (Production Method 1)

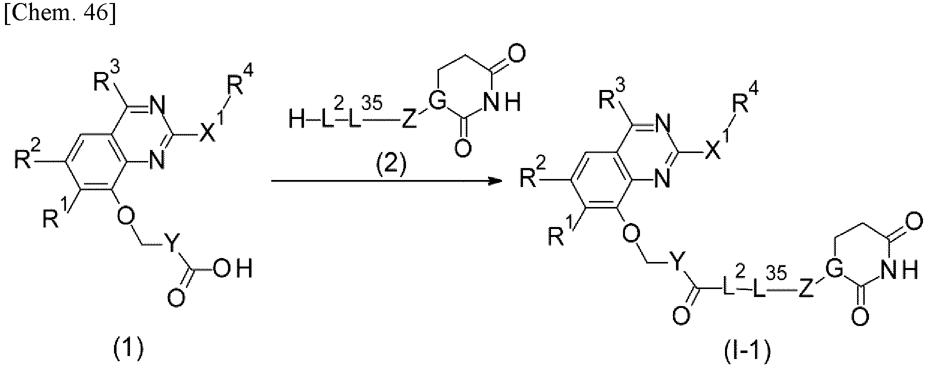

(In the formulae, -L³⁵- represents -L³-L⁴-L⁵- contained in -L-. The same shall apply hereinafter.)

This production method is a method for producing a compound of the formula (I-1) included in the compound of the formula (I), in which A is N, -L¹- contained in -L- is CO, - L²- is -N(R^{L2})- or an optionally substituted saturated heterocyclic divalent group containing one or two nitrogen atoms, -L³-L⁴-L⁵- is -L³⁵-, and R^{L2} is H or C₁₋₆ alkyl.

In this reaction, the compound (1) and the compound (2) are used in an equal amount or with one compound thereof in an excess amount, and the mixture of the compounds is stirred in the presence of a condensing agent, in a solvent inactive for the reaction, from under cooling to under heating, preferably at -20°C to 60°C, generally for 0.1 hours to 5 days, to obtain an amide compound. Examples of the solvent include, but are not particularly limited to, an aromatic hydrocarbon, such as toluene, an ether, such as THF or DOX, a halogenated hydrocarbon, such as dichloromethane, an alcohol, DMF, DMSO, ethyl acetate, MeCN and a mixture thereof. Examples of the condensing agent include PyBOP, O-(7-azabenzotriazol-1-yl)-N,N,N',N'-tetramethyluronium hexafluorophosphate (HATU), 1-(3-dimethylaminopropyl)-3-ethylcarbodiimide or the hydrochloride thereof, N,N'-dicyclohexylcarbodiimide (DCC), CDI, diphenylphosphoryl azide (DPPA) and the like. Use of an additive (for example, 1-hydroxybenzotriazole) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

Alternatively, a method in which the compound (1) is converted into a reactive derivative, which is then subjected to an acylation reaction, can be used. Examples of the reactive derivative of a carboxylic acid include an acid halogenation product obtained by a reaction with a halogenating agent, such as phosphorus oxychloride and thionyl chloride, a mixed acid anhydride obtained by a reaction with isobutyl chloroformate or the like, an active ester obtained by condensation with 1-hydroxybenzotriazole or the like. The reaction of the reactive derivative and the compound (2) can be performed in a solvent inactive for the reaction, such as a halogenated hydrocarbon, an aromatic hydrocarbon and an ether, from under cooling to under heating, preferably at -20°C to 120°C.

### [Reference]

S. R. Sandler and W. Karo, "Organic Functional Group Preparations", 2nd edition, Vol. 1, Academic Press Inc., 1991
The Chemical Society of Japan, "Jikken Kagaku Koza (Courses in Experimental Chemistry) (5th edition)", Vol. 16 (2005) (Maruzen)

### (Production Method 2)

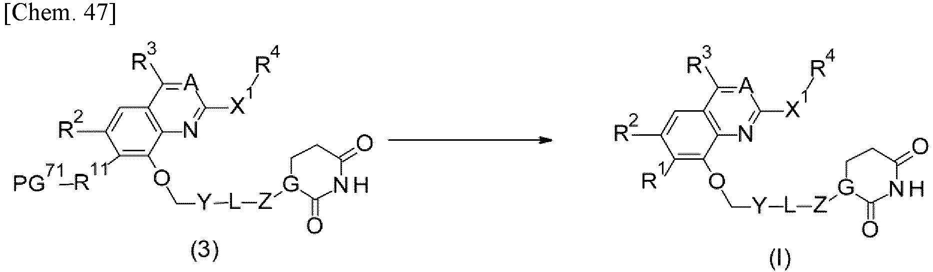

(PG⁷¹ represents a protective group of NH contained in R¹, and R¹¹ represents a divalent group formed by elimination of H from NH contained in R¹. The same shall apply hereinafter.)

This production method is another method for producing the compound of the formula (I).

The compound of the formula (I) can also be obtained by subjecting the compound (3) to a deprotection reaction. Examples of the protective group shown herein include a tert-butoxycarbonyl group, a triphenylmethyl group, a tetrahydro-2H-pyran-2-yl group, a methoxymethyl group, a dimethylmethanediyl group, a tert-butylsulfinyl group and the like.

This deprotection reaction is performed by stirring the compound under cooling to under reflux with heat, generally for 0.1 hours to 5 days. Examples of the solvent used here include, but are not particularly limited to, an alcohol, such as MeOH or EtOH, a halogenated hydrocarbon, such as dichloromethane, 1,2-dichloroethane or chloroform, an ether, such as diethyl ether, THF, DOX or dimethoxyethane, DMF, DMSO, MeCN or water and a mixture thereof. Examples of the deprotection reagent include, but are not particularly limited to, acids such as hydrogen chloride (DOX solution), trifluoroacetic acid, methanesulfonic acid and phosphoric acid.

By selecting a protective group, deprotection can also be performed by a catalytic hydrogenation reaction. Examples of the protective group include a benzyl group, a p-methoxybenzyl group, a benzyloxycarbonyl group and the like. Moreover, deprotection can also be performed with a fluoride ion source such as tetra-n-butylammonium fluoride. Examples of the protective group include a tert-butyl(dimethyl)silyl group, a (trimethylsilyl)ethoxymethyl group and the like. Furthermore, examples of the protective group which can be removed under basic conditions include an acetyl group, a trifluoroacetyl group, a benzoyl group and the like.

For example, the following can be referred as a reference.
P. G. M. Wuts and T. W. Greene, "Greene's Protective Groups in Organic Synthesis", 5th edition, John Wiley & Sons Inc., 2014
A. R. Katritzky and R. J. K. Taylor, "Comprehensive Organic Functional Group Transformations II", Vol. 2, Elsevier Pergamon, 2005

### (Production Method 3)

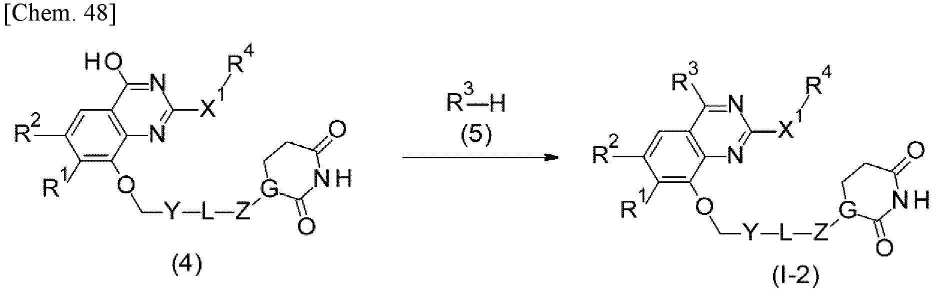

This production method is a method for producing a compound of the formula (I-2) included in the compound of the formula (I), in which A is N, by a reaction of the compound (4) and the compound (5).

In this reaction, the compound (4) and the compound (5) are used in an equal amount or with one in an excess amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include, but are not particularly limited to, an aromatic hydrocarbon, such as toluene, an ether, such as THF or DOX, a halogenated hydrocarbon, such as dichloromethane, an alcohol, DMF, DMSO, ethyl acetate, MeCN and a mixture thereof. Examples of the condensing agent include PyBOP, HATU, CDI, PyAOP and the like. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

Alternatively, the compound of the formula (I-2) can also be obtained by converting the compound (4) into a reactive derivative in which a hydroxyl group at the 4-position of the compound (4) is converted to a chloro group using a chlorinating reagent, such as phosphorus oxychloride and thionyl chloride, followed by addition of the compound (5) in the presence of an organic base, such as TEA, DIPEA or pyridine, or an inorganic base, such as potassium carbonate, cesium carbonate or potassium acetate. The reaction of the reactive derivative and the compound (5) can be performed in a solvent inactive for the reaction, such as a halogenated hydrocarbon, an aromatic hydrocarbon and an ether, from under cooling to under heating, preferably at -20°C to 120°C.

### (Production Method 4)

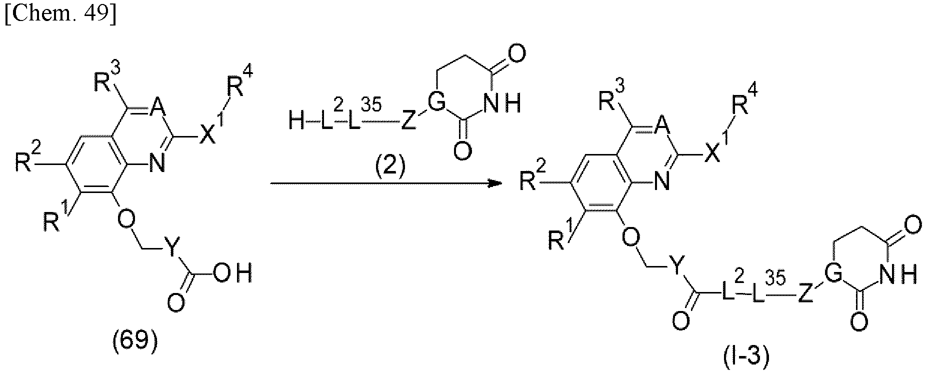

This production method is a method for producing a compound of the formula (I-3) included in the compound of the formula (I), in which -L¹- contained in -L- is CO, -L²- is - N(R^{L2})- or an optionally substituted saturated heterocyclic divalent group containing one or two nitrogen atoms, -L³-L⁴-L⁵- is -L³⁵-, and R^{L2} is H or C₁₋₆ alkyl.

In this reaction, the compound of the formula (I-3) can be obtained by subjecting the compound (69) and the compound (2) to the same reaction conditions as in the Production Method 1.

### (Raw Material Synthesis 1)

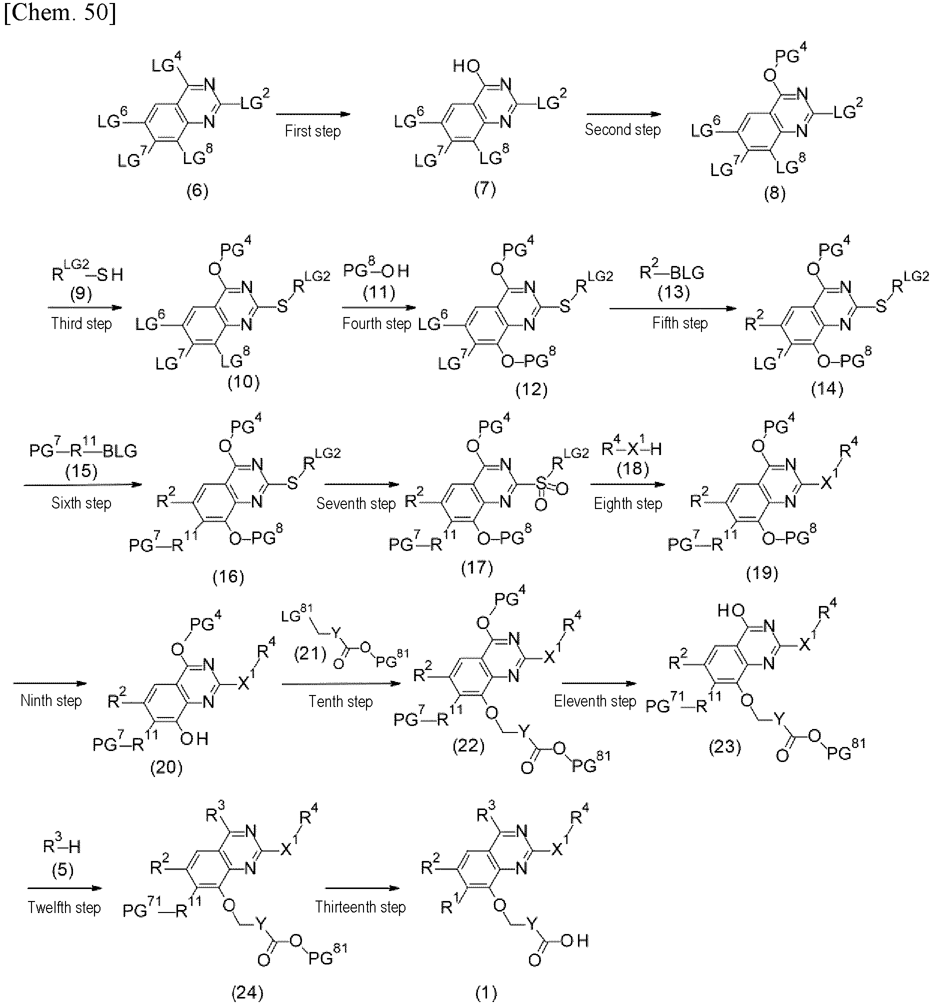

(In the formulae, LG², LG⁴, LG⁶, LG⁷, LG⁸ and LG^{S1}, which are the same as or different from each other, each represent a leaving group. PG⁴ represents a protective group of OH, PG⁷ and PG⁷¹ each represent a protective group of NH contained in R¹, R¹¹ represents a divalent group formed by elimination of H from NH contained in R¹, PG⁸ represents a protective group which can be removed under catalytic hydrogenation reaction conditions, and PG⁸¹ represents a protective group of COOH. Moreover, the deprotection can also be performed in stages by selecting protective groups which can be removed under different deprotection conditions as PG⁴, PG⁷, PG⁷¹, PG⁸ and PG⁸¹. R^{LG2} represents a C₁₋₁₂ alkyl group, and BLG represents a boronic acid group, a boronic acid group protected with a protective group of boronic acid such as a boronic acid pinacol ester group or a trifluoroboric acid salt group (hereinafter sometimes described as a boronic acid group or the like). Examples of the leaving group shown here include Cl, Br, I, a methanesulfonyloxy group, a p-toluenesulfonyloxy group and the like. The same shall apply hereinafter.)

This production method is a first method for producing a compound (1), which is a raw material compound of the Production Method 1.

### (First step)

This step is a method for producing a compound (7) from the compound (6).

This reaction is performed by stirring the compound (6) under cooling to under reflux with heat, generally for 0.1 hours to 5 days. Examples of the solvent used here include, but are not particularly limited to, an alcohol, acetone, DMF, THF and the like. In addition, a mixed solvent of the above solvent and water is sometimes suitable for the reaction. Examples of the reagent used in this reaction include, but are not particularly limited to, an aqueous sodium hydroxide solution, an aqueous potassium hydroxide solution and the like.

For example, the following can be referred as a reference about this reaction.

The Chemical Society of Japan, "Jikken Kagaku Koza (Courses in Experimental Chemistry) (5th edition) ", Vol. 16 (2005) (Maruzen)
Angew. Chem. Int. Ed. 2005, 44, p.1378-1382.

### (Second step)

This step is a method for producing a compound (8) by protection of a hydroxyl group of the compound (7) with the protective group.

In the case of protection with a tert-butyl group, for example, this reaction is performed by stirring the compound (7) in the presence of a tert-butyl protection reagent, from under cooling to under reflux with heat, generally for 0.1 hours to 5 days. Examples of the solvent used here include, but are not particularly limited to, an ether, such as THF or DOX, a halogenated hydrocarbon, such as dichloromethane, tBuOH, DMF and the like. Examples of the tert-butyl protection reagent include, but are not particularly limited to, isobutene, 2-tert-butyl-1,3-diisopropylisourea and the like.

Moreover, the compound (8) can be produced by a dehydration condensation reaction of the compound (7) and tBuOH.

For example, the following can be referred as a reference about this reaction.

P. G. M. Wuts and T. W. Greene, "Greene's Protective Groups in Organic Synthesis", 5th edition, John Wiley & Sons Inc., 2014
Org. Lett., 2012, 14, 17, p.4678-4681

### (Third step)

This step is a method for producing a compound (10) by an ipso substitution reaction of the compound (8) and the compound (9), R^{LG2}-SH.

Examples of the R^{LG2}-SH used here include C₁₋₁₂ alkylthiols, for example, ethanethiol and dodecanethiol.

In this reaction, the compound (8) and the compound (9) are used in an equal amount or with one compound thereof in an excess amount, and the mixture of the compounds is stirred in a solvent inactive for the reaction or with no solvent, from under cooling to under reflux with heat, preferably at 0°C to 80°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, DMF, DMAc, DMSO, ethyl acetate, MeCN and a mixture thereof. Performing the reaction in the presence of an organic base, such as TEA, DIPEA, NMM, 1,4-diazabicyclo[2.2.2]octane (DABCO) or tBuOK, or an inorganic base, such as sodium hydride, potassium carbonate, sodium carbonate or cesium carbonate, is sometimes advantageous for smoothly promoting the reaction.

### (Fourth step)

This step is a method for producing a compound (12) by an ipso substitution reaction of the compound (10) and the compound (11), PG⁸-OH. Examples of the PG⁸-OH used here include benzyl alcohol, p-methoxybenzyl alcohol and 1-phenylethanol.

The reaction conditions are the same as in the third step of the Raw Material Synthesis 1.

### (Fifth step)

This step is a method for producing a compound (14) by a Suzuki-Miyaura coupling reaction of the compound (12) and the compound (13), a boronic acid derivative composed of a R²-boronic acid group or the like.

Examples of the boronic acid group or the like used here include, but are not particularly limited to, a boronic acid group, a boronic acid ester group, a boronic acid pinacol ester group, a triol borate salt group, a trifluoroboric acid salt group and the like.

In this reaction, the compound (12) and the boronic acid derivative composed of the R²-boronic acid group or the like are used in an equal amount or with one compound thereof in an excess amount, and the mixture of the compounds is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include inorganic bases, such as tripotassium phosphate, sodium carbonate, potassium carbonate, sodium hydroxide and barium hydroxide. Examples of the palladium catalyst include tetrakis(triphenylphosphine)palladium, bis(triphenylphosphine)palladium(II) dichloride, PdCl₂(dppf)·CH₂Cl₂, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, palladium(II) acetate and the like. Performing the reaction in the presence of a ligand, such as dicyclohexyl(2',6'-dimethoxybiphenyl-2-yl)phosphine, dicyclohexyl(2',6'-diisopropoxy-[1,1'-biphenyl]-2-yl)phosphine or 1,1'-bis(diphenylphosphino)ferrocene is sometimes advantageous for smoothly promoting the reaction. In addition, heating the mixture by microwave irradiation is sometimes advantageous for smoothly promoting the reaction.

### [Reference]

J. Am. Chem. Soc., 2005, 127, p.4685-4696
Org. Lett. 2011, 13, p.3948-3951
Org. Lett. 2012, 14, p.1278-1281
When LG⁶ is halogen, the compound (14) (where R² is hydrogen) can be produced by a dehydrogenation reaction of the compound (12) with a Pd catalyst and a reducing agent.

### [Reference]

J. Org. Chem., 1977, 42, p.3491-3494
Tetrahedron Letters 2013, 54, 5207-5210

### (Sixth step)

This step is a method for producing a compound (16) by a Suzuki-Miyaura coupling reaction of the compound (14) and a compound (15).

The reaction conditions are the same as in the fifth step of the Raw Material Synthesis 1.

When the compound (16) has an axial chirality, the compound (16) is obtained as a mixture of stereoisomers, and each stereoisomer can be isolated by separation using a common separation operation, for example, ODS column chromatography or silica gel column chromatography.

### (Seventh step)

This step is a method for producing a compound (17) by an oxidation reaction of the compound (16).

In this reaction, the compound (16) is treated with an oxidant in an equal amount or in an excess amount in a solvent inactive for the reaction, from under cooling to under heating, preferably at -20°C to 80°C, generally for 0.1 hours to 3 days. In this reaction, oxidation with m-chloroperbenzoic acid, perbenzoic acid, peracetic acid, sodium hypochlorite or hydrogen peroxide is suitably used. Examples of the solvent include an aromatic hydrocarbon, an ether, a halogenated hydrocarbon, such as dichloromethane, DMF, DMSO, ethyl acetate, MeCN and a mixture thereof. Other examples of the oxidant include cumene hydroperoxide, Oxone, active manganese dioxide, chromic acid, potassium permanganate, sodium periodate and the like.

### [Reference]

The Chemical Society of Japan, "Jikken Kagaku Koza (Courses in Experimental Chemistry)", 5th edition, Vol. 17, Maruzen, 2004
When the compound (17) has an axial chirality, the compound (17) may be obtained as a mixture of stereoisomers, and each stereoisomer can be isolated by separation using a common separation operation, for example, ODS column chromatography or silica gel column chromatography, separation by SFC using a chiral column or the like.

### (Eighth step)

This step is a method for producing a compound (19) by an ipso substitution reaction of the compound (17) and the compound (18).

The reaction conditions are the same as in the third step of the Raw Material Synthesis 1.

### (Ninth step)

This step is a method for producing a compound (20) by deprotection by a catalytic hydrogenation reaction of the compound (19).

This reaction can be performed by stirring the compound (19) under hydrogen atmosphere, from under normal pressure to under increased pressure, in a solvent inactive for the reaction, such as MeOH, EtOH or ethyl acetate, in the presence of a metal catalyst, from under cooling to under heating, preferably at room temperature, for 1 hour to 5 days. As the metal catalyst, a palladium catalyst, such as Pd/C or palladium black, a platinum catalyst, such as a platinum plate or platinum oxide, a nickel catalyst, such as reduced nickel or Raney nickel, or the like is used.

### (Tenth step)

This step is a method for producing a compound (22) from the compound (20) and the compound (21).

This reaction is performed by reacting a mixture of the compound (20) and the compound (21) in an equal amount or with one compound thereof in an excess amount in the presence of a base, in a solvent inactive for the reaction, from under cooling to under reflux with heat, preferably at 0°C to 80°C, generally for 0.1 hours to 5 days. The solvent used here is not particularly limited, and examples thereof include an aromatic hydrocarbon, such as benzene, toluene or xylene, an alcohol, such as MeOH or EtOH, an ether, such as diethyl ether, THF, DOX or 1,2-dimethoxyethane, a halogenated hydrocarbon, such as dichloromethane, 1,2-dichloroethane or chloroform, DMF, DMSO, ethyl acetate, MeCN and a mixture thereof. Examples of the base include, but are not particularly limited to, an organic base, for example, such as TEA, DIPEA, 1,8-diazabicyclo[5.4.0]-7-undecene, n-butyllithium or tBuOK, and an inorganic base, such as sodium hydroxide, sodium carbonate, potassium carbonate, cesium carbonate or sodium hydride. Performing the reaction in the presence of a phase transfer catalyst, such as tetra-n-butylammonium chloride, is sometimes advantageous.

For example, the following can be referred as a reference about this reaction.

The Chemical Society of Japan, "Jikken Kagaku Koza (Courses in Experimental Chemistry)", 5th edition, Vol. 14, Maruzen, 2005
Moreover, the compound (21) in which LG⁸¹ is halogen can be produced by halogenation of a compound in which the moiety corresponding to LG⁸¹ is a hydroxy group. Examples of the halogenating agent used here include, but are not particularly limited to, thionyl chloride, phosphorus oxychloride, hydrobromic acid, phosphorus tribromide and the like.

For example, the following can be referred as a reference about this reaction.

The Chemical Society of Japan, "Jikken Kagaku Koza (Courses in Experimental Chemistry)", 5th edition, Vol. 13, Maruzen, 2004
The compound (21) in which LG⁸¹ is a sulfonyloxy group can be produced by sulfonylation of a compound in which the moiety corresponding to LG⁸¹ is a hydroxy group in the presence of a base. Examples of the sulfonylating reagent used here include, but are not particularly limited to, for example, methanesulfonylchloride, p-toluenesulfonylchloride, methanesulfonic anhydride and the like. Examples of the base include, but are not particularly limited to, for example, TEA, DIPEA, pyridine, tetramethylethylenediamine and the like.

For example, the following can be referred as a reference about this reaction.
Synthesis 1999, 9, p.1633-1636

### (Eleventh step)

This step is a method for producing a compound (23) by performing deprotection of PG⁴ and PG⁷, which are protective groups of the compound (22), and further protecting a deprotected NH group contained in R¹¹ with another protective group PG⁷¹.

The reaction can be performed under the same reaction conditions as in the step described in the Production Method 2 by adding, in addition to the compound (22) and the deprotection reagent, a protection reagent for protecting the NH group with a PG⁷¹ group.

### (Twelfth step)

This step is a method for producing a compound (24) by a reaction of the compound (23) and the compound (5).

The reaction conditions are the same as in the Production Method 3.

### (Thirteenth Step)

This step is a method for producing a compound (1) by subjecting the compound (24) to a deprotection reaction.

The reaction conditions are the same as in the step described in Production Method 2.

### (Raw Material Synthesis 2)

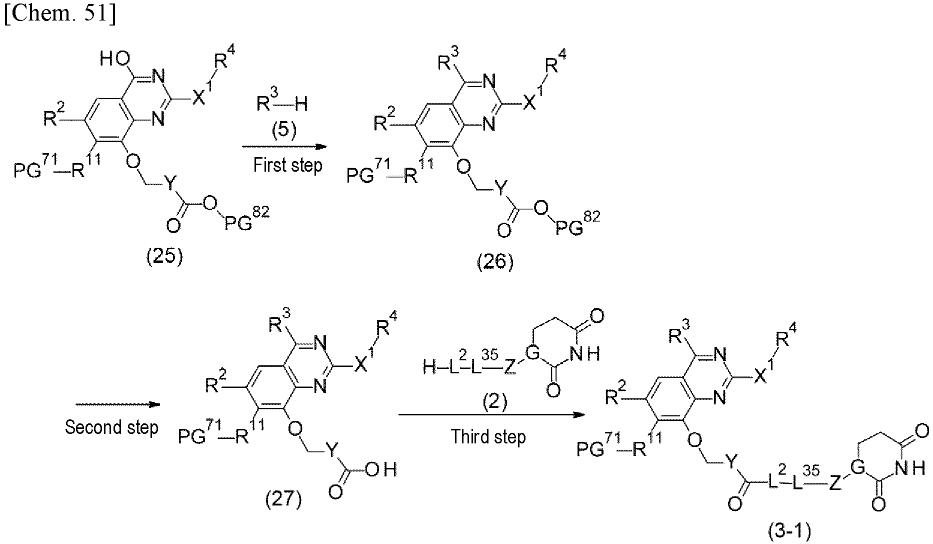

(In the formulae, PG⁸² represents a C₁₋₃ alkyl. The same shall apply hereinafter.)

This production method is a first method for producing a compound (3-1) included in the compound (3), which is a raw material compound of the Production Method 2, in which A is N, -L¹- contained in -L- is CO, -L²- is -N(R^{L2})- or an optionally substituted saturated heterocyclic divalent group containing one or two nitrogen atoms, -L³-L⁴-L⁵- is - L³⁵-, and R^{L2} is H or C₁₋₆ alkyl.

### (First step)

This step is a method for producing a compound (26) by a reaction of the compound (25) and the compound (5).

The reaction conditions are the same as in the Production Method 3.

### (Second step)

This step is a method for producing a compound (27) by hydrolysis of the compound (26) under basic conditions.

This reaction is performed by stirring the compound (26) under cooling to under reflux with heat, generally for 0.1 hours to 5 days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution, trimethyltin hydroxide and the like.

For example, the following can be referred as a reference about this reaction.

The Chemical Society of Japan, "Jikken Kagaku Koza (Courses in Experimental Chemistry) (5th edition)", Vol. 16 (2005) (Maruzen)
Angew. Chem. Int. Ed. 2005, 44, p.1378-1382.

### (Third step)

This step is a method for producing a compound (3-1) by subjecting the compound (27) and the compound (2) to an amidation reaction.

The reaction conditions are the same as in the step described in the Production Method 1.

### (Raw Material Synthesis 3)

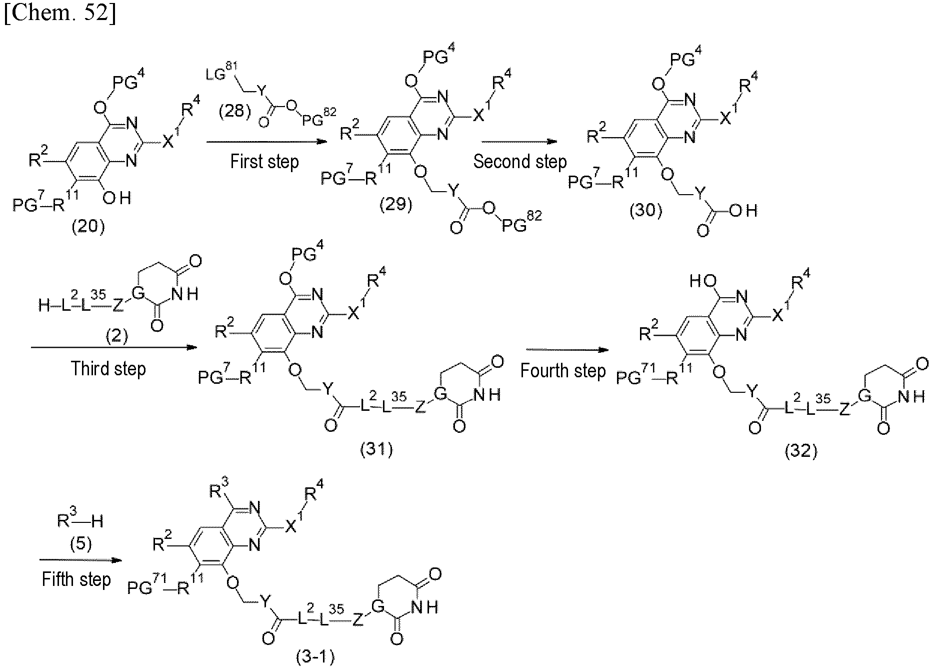

This production method is a second method for producing a compound (3-1) included in the compound (3), which is a raw material compound of the Production Method 2, in which A is N, -L¹- contained in -L- is CO, -L²- is -N(R^{L2})- or an optionally substituted saturated heterocyclic divalent group containing one or two nitrogen atoms, -L³-L⁴-L⁵- is - L³⁵-, and R^{L2} is H or C₁₋₆ alkyl.

### (First step)

This step is a method for producing a compound (29) from the compound (20) and a compound (28).

The reaction conditions are the same as in the tenth step of the Raw Material Synthesis 1.

### (Second step)

This step is a method for producing a compound (30) by subjecting the compound (29) to a deprotection reaction.

The reaction conditions are the same as in the second step of the Raw Material Synthesis 2.

### (Third step)

This step is a method for producing a compound (31) by subjecting the compound (30) and the compound (2) to an amidation reaction.

The reaction conditions are the same as in the step described in the Production Method 1.

### (Fourth step)

This step is a method for producing a compound (32) by performing deprotection of PG⁴ and PG⁷, which are protective groups of the compound (31), and further protecting a deprotected NH group contained in R¹¹ with another protective group PG⁷¹.

The reaction can be performed under the same reaction conditions as in the step described in the Production Method 2 by adding, in addition to the compound (31) and the deprotection reagent, a protection reagent for protecting the NH group with a PG⁷¹ group.

### (Fifth step)

This step is a method for producing a compound (3-1) by a reaction of the compound (32) and the compound (5).

The reaction conditions are the same as in the Production Method 3.

### (Raw Material Synthesis 4)

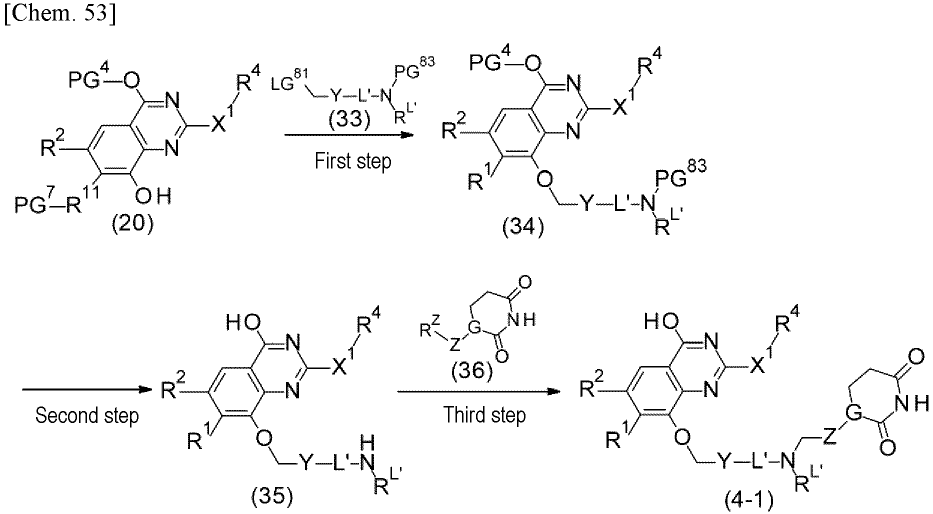

(In the formulae, -L'- represents -L¹-L²-L³- contained in -L-, -R^{Z} represents -CHO or -CH₂-LG⁸¹, and PG⁸³ represents a protective group of NH attached to L', where R^{L'} is H or a C₁₋₃ alkyl, or R^{L'} together with the nitrogen atom and L' adjacent thereto optionally forms a saturated heterocyclic divalent group containing one or two nitrogen atoms.)

This production method is a method for producing a compound (4-1) included in the compound (4), which is a raw material compound of the Production Method 3, in which -L⁴-contained in -L- is -N(R^{L'})-, and -L⁵- is -CH₂-.

### (First step)

This step is a method for producing a compound (34) from the compound (20) and the compound (33).

The reaction conditions are the same as in the tenth step of the Raw Material Synthesis 1.

### (Second step)

This step is a method for producing a compound (35) by subjecting the compound (34) to a deprotection reaction.

The reaction conditions are the same as in the step described in the Production Method 2.

### (Third step)

This step is a step of obtaining a compound (4-1) from the compound (35) and the compound (36) by a reductive amination reaction when -R^{Z} is -CHO or by an alkylation reaction when -R^{Z} is -CH₂-LG⁸¹.

When -R^{Z} is -CHO, the reaction is performed by stirring the compound (35) and the compound (36) in an equivalent amount or with one in an excess equivalent amount in the presence of a reducing agent and acetic acid, in a solvent inactive for the reaction, from under ice-bath cooling to room temperature, generally for 1 hour to 5 days. Examples of the reducing agent used here include, but are not particularly limited to, NaBH(OAc)₃, 2-picoline borane, NaBH₃CN and the like. Examples of the solvent used here include, but are not particularly limited to, a halogenated hydrocarbon, such as dichloromethane, dichloroethane and chloroform, an ether-based solvent, such as THF, diethyl ether and DOX, an alcohol-based solvent, such as MeOH and EtOH, and MeCN.

When -R^{Z} is -CH₂-LG⁸¹, the reaction conditions are the same as in the tenth step of the Raw Material Synthesis 1.

### (Raw Material Synthesis 5)

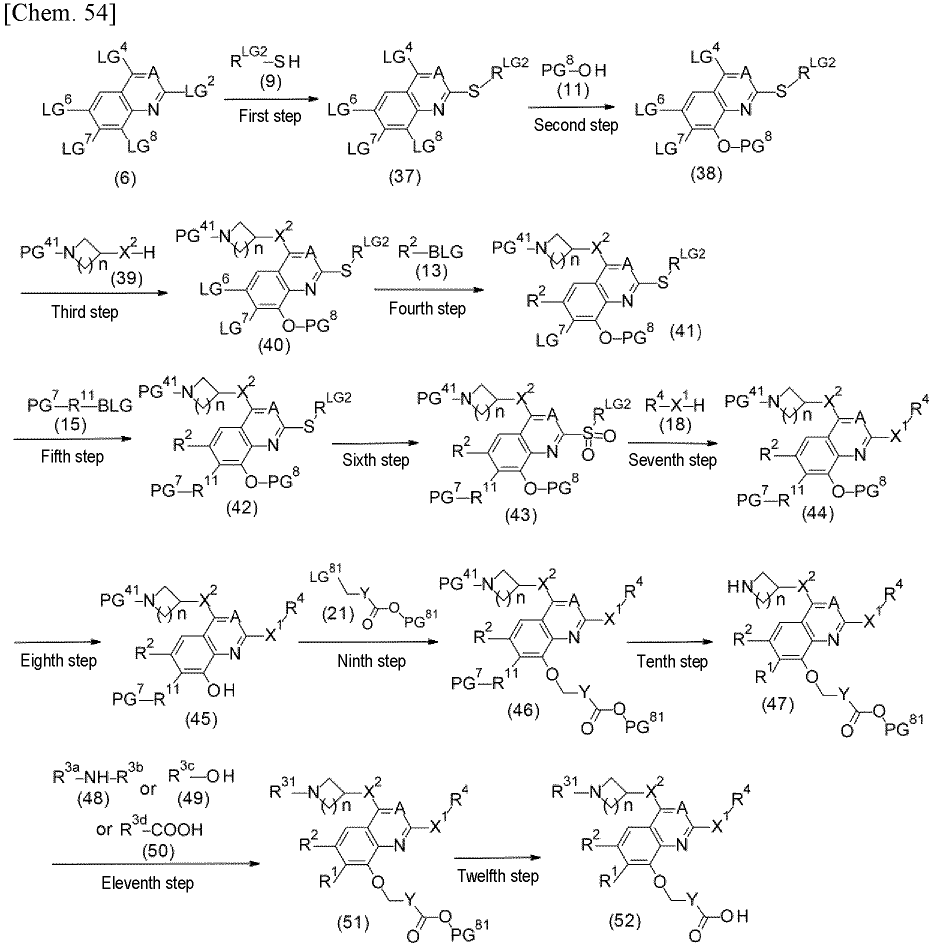

(In the formulae, PG⁴¹ represents a protective group of NH, and R³¹ represents R^{3a}-N(R^{3b})CO-, R^{3c}-OCO- or R^{3d}-CO-.)

This production method is a method for producing a compound (52) included in compound (1), which is a raw material compound of Production Method 1, in which R³ is the formula (IV), the formula (V) or the formula (VI), and X³ is O.

### (First step)

This step is a method for producing a compound (37) by an ipso substitution reaction of the compound (6) and the compound (9), R^{LG2}-SH.

The reaction conditions are the same as in the third step of the Raw Material Synthesis 1.

### (Second Step)

This step is a method for producing a compound (38) by an ipso substitution reaction of the compound (37) and the compound (11).

The reaction conditions are the same as in the fourth step of the Raw Material Synthesis 1.

### (Third step)

This step is a method for producing a compound (40) by an ipso substitution reaction of the compound (38) and a compound (39) having an amino group protected with the protective group PG⁴¹.

In this reaction, the compound (38) and the compound (39) are used in an equal amount or with one compound thereof in an excess amount, and the mixture of the compounds is stirred in a solvent inactive for the reaction, from under cooling to under heating, preferably at -20°C to 60°C, generally for 0.1 hours to 5 days. Examples of the solvent include, but are not particularly limited to, an aromatic hydrocarbon, such as toluene, a halogenated hydrocarbon, such as dichloromethane, DMF, DMSO, ethyl acetate, MeCN and a mixture thereof. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

### (Fourth step)

This step is a method for producing a compound (41) by a Suzuki-Miyaura coupling reaction of the compound (40) and the compound (13), a boronic acid derivative composed of a R²-boronic acid group or the like.

The reaction conditions are the same as in the fifth step of the Raw Material Synthesis 1.

### (Fifth step)

This step is a method for producing a compound (42) by a Suzuki-Miyaura coupling reaction of the compound (41) and the compound (15).

The reaction conditions are the same as in the fifth step of the Raw Material Synthesis 1.

When the compound (42) has an axial chirality, the compound (42) is obtained as a mixture of stereoisomers, and each stereoisomer can be isolated by separation using a common separation operation, for example, ODS column chromatography or silica gel column chromatography.

### (Sixth step)

This step is a method for producing a compound (43) by an oxidation reaction of the compound (42).

The reaction conditions are the same as in the seventh step of the Raw Material Synthesis 1.

When the compound (43) has an axial chirality, the compound (43) may be obtained as a mixture of stereoisomers, and each stereoisomer can be isolated by separation using a common separation operation, for example, ODS column chromatography or silica gel column chromatography, separation by SFC using a chiral column or the like.

### (Seventh step)

This step is a method for producing a compound (44) by an ipso substitution reaction of the compound (43) and the compound (18).

The reaction conditions are the same as in the third step of the Raw Material Synthesis 1.

### (Eighth step)

This step is a method for producing a compound (45) by deprotection by a catalytic hydrogenation reaction of the compound (44).

The reaction conditions are the same as in the ninth step of the Raw Material Synthesis 1.

### (Ninth step)

This step is a method for producing a compound (46) from the compound (45) and the compound (21).

The reaction conditions are the same as in the tenth step of the Raw Material Synthesis 1.

### (Tenth step)

This step is a method for producing a compound (47) by subjecting the compound (46) to a deprotection reaction.

The reaction conditions are the same as in the step described in the Production Method 2.

### (Eleventh step)

This step is a step of obtaining a compound (51) by subjecting the compound (47) to a urea formation reaction using a compound (48), a carbamate formation reaction using a compound (49), and an amidation reaction using a compound (50).

In the urea formation reaction using the compound (48) and the carbamate formation reaction using the compound (49), the compound (48) or the compound (49) is used in an equal amount or with one in an excess amount with respect to the compound (47), and the mixture of the compounds is stirred in the presence of a condensing agent, in a solvent inactive for the reaction, from under cooling to under heating, preferably at -20°C to 60°C, generally for 0.1 hours to 5 days. Examples of the solvent include, but are not particularly limited to, an aromatic hydrocarbon, such as toluene, an ether, such as THF or DOX, a halogenated hydrocarbon, such as dichloromethane, an alcohol, DMF, DMSO, ethyl acetate, MeCN and a mixture thereof. Examples of the condensing agent include CDI, triphosgene, bis(4-nitrophenyl)carbonate, 4-nitrophenyl chloroformate and the like. Use of an additive (for example, 1-hydroxybenzotriazole or dimethylaminopyridine) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

The reaction conditions for the amidation reaction using the compound (50) are the same as in the step described in the Production Method 1.

### (Twelfth step)

This step is a method for producing a compound (52) by subjecting the compound (51) to a deprotection reaction.

The reaction conditions are the same as in the step described in the Production Method 2.

### (Raw Material Synthesis 6)

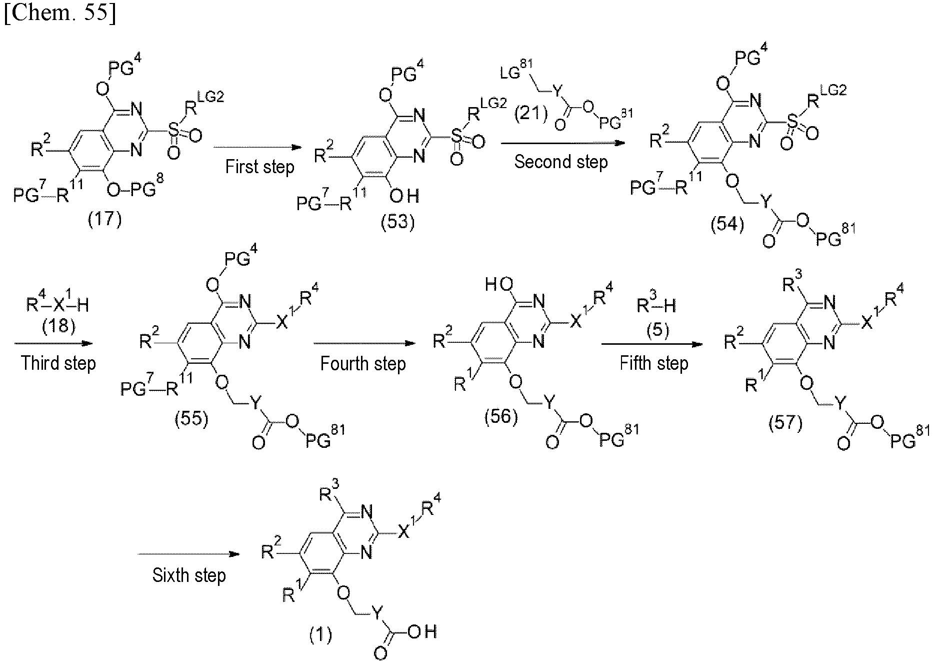

This production method is a second method for producing a compound (1), which is a raw material compound of the Production Method 1.

### (First step)

This step is a method for producing a compound (53) by deprotection by a catalytic hydrogenation reaction of the compound (17).

The reaction conditions are the same as in the ninth step of the Raw Material Synthesis 1.

### (Second step)

This step is a method for producing a compound (54) from the compound (53) and the compound (21).

The reaction conditions are the same as in the tenth step of the Raw Material Synthesis 1.

### (Third step)

This step is a method for producing a compound (55) by an ipso substitution reaction of the compound (54) and the compound (18).

The reaction conditions are the same as in the third step of the Raw Material Synthesis 1.

### (Fourth step)

This step is a method for producing a compound (56) by subjecting the compound (55) to a deprotection reaction.

The reaction conditions are the same as in the step described in the Production Method 2.

### (Fifth step)

This step is a method for producing a compound (57) by a reaction of the compound (56) and the compound (5).

The reaction conditions are the same as in the Production Method 3.

### (Sixth step)

This step is a method for producing a compound (1) by subjecting the compound (57) to a deprotection reaction.

The reaction conditions are the same as in the step described in the Production Method 2.

### (Raw Material Synthesis 7)

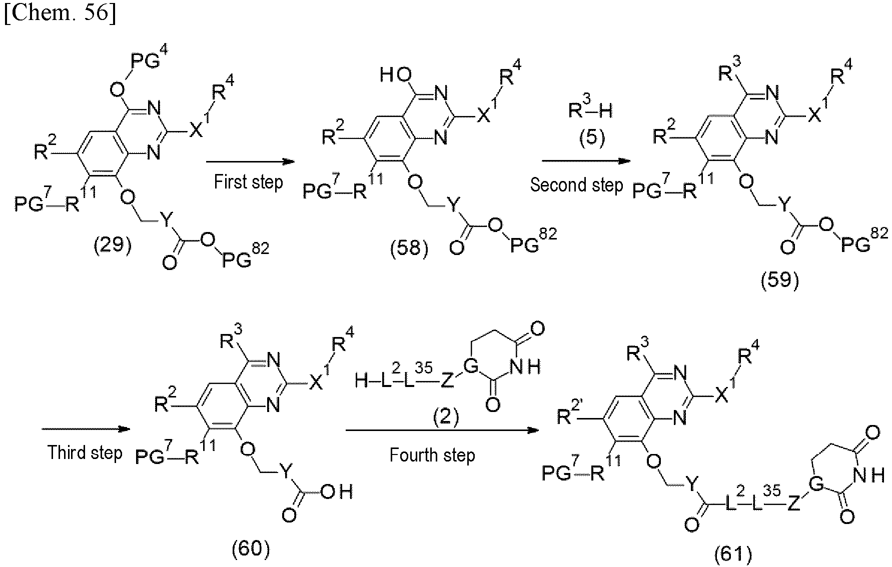

This production method is a method for producing a compound (61) included in the compound (3), which is a raw material compound of the Production Method 2, in which A is N, -L¹- contained in -L- is CO, -L²- is -N(R^{L2})- or an optionally substituted saturated heterocyclic divalent group containing one or two nitrogen atoms, -L³-L⁴-L⁵- is -L³⁵-, and R^{L2} is H or a C₁₋₆ alkyl.

### (First step)

This step is a method for producing a compound (58) by subjecting the compound (29) to a deprotection reaction.

The reaction conditions are the same as in the step described in the Production Method 2.

### (Second step)

This step is a method for producing a compound (59) by a reaction of the compound (58) and the compound (5).

The reaction conditions are the same as in the Production Method 3.

### (Third step)

This step is a method for producing a compound (60) by subjecting the compound (59) to a deprotection reaction.

The reaction conditions are the same as in the second step of the Raw Material Synthesis 2.

### (Fourth step)

This step is a method for producing a compound (61) by subjecting the compound (60) and the compound (2) to an amidation reaction.

The reaction conditions are the same as in the step described in the Production Method 1.

### (Raw Material Synthesis 8)

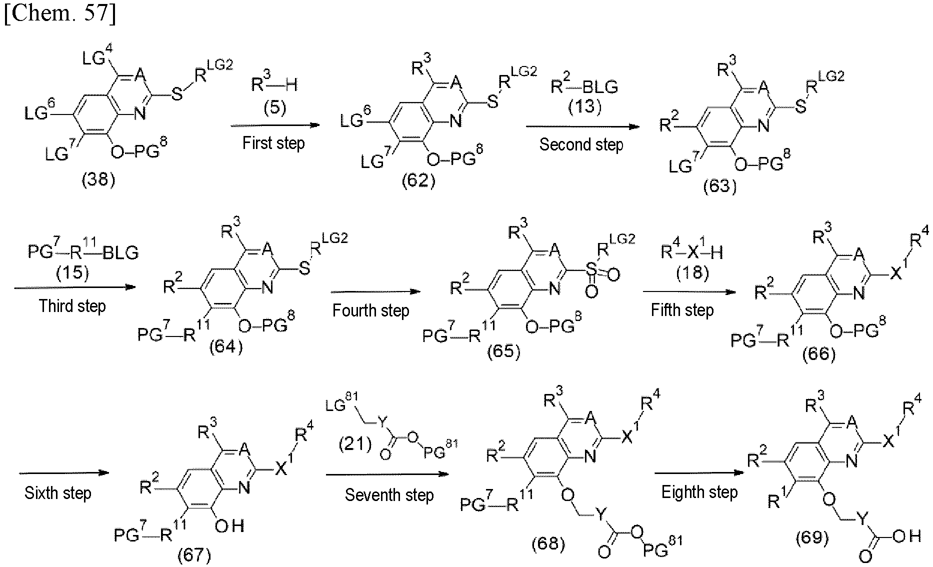

This production method is a method for producing a compound (69), which is a raw material compound of the Production Method 4.

### (First step)

This step is a method for producing a compound (62) by an ipso substitution reaction of the compound (38) and the compound (5).

The reaction conditions are the same as in the third step of the Raw Material Synthesis 5.

### (Second step)

This step is a method for producing a compound (63) by a Suzuki-Miyaura coupling reaction of the compound (62) and the compound (13), a boronic acid derivative composed of a R²-boronic acid group or the like.

The reaction conditions are the same as in the fifth step of the Raw Material Synthesis 1.

### (Third step)

This step is a method for producing a compound (64) by a Suzuki-Miyaura coupling reaction of the compound (63) and the compound (15).

The reaction conditions are the same as in the fifth step of the Raw Material Synthesis 1.

When the compound (64) has an axial chirality, the compound (64) is obtained as a mixture of stereoisomers, and each stereoisomer can be isolated by separation using a common separation operation, for example, ODS column chromatography or silica gel column chromatography.

### (Fourth step)

This step is a method for producing a compound (65) by an oxidation reaction of the compound (64).

The reaction conditions are the same as in the seventh step of the Raw Material Synthesis 1.

When the compound (65) has an axial chirality, the compound (65) may be obtained as a mixture of stereoisomers, and each stereoisomer can be isolated by separation using a common separation operation, for example, ODS column chromatography or silica gel column chromatography, separation by SFC using a chiral column or the like.

### (Fifth step)

This step is a method for producing a compound (66) by an ipso substitution reaction of the compound (65) and the compound (18).

The reaction conditions are the same as in the third step of the Raw Material Synthesis 1.

### (Sixth step)

This step is a method for producing a compound (67) by deprotection by a catalytic hydrogenation reaction of the compound (66).

The reaction conditions are the same as in the ninth step of the Raw Material Synthesis 1.

### (Seventh step)

This step is a method for producing a compound (68) from the compound (67) and the compound (21).

The reaction conditions are the same as in the tenth step of the Raw Material Synthesis 1.

### (Eighth step)

This step is a method for producing a compound (69) by subjecting the compound (68) to a deprotection reaction.

The reaction conditions are the same as in the step described in the Production Method 2.

### (Raw Material Synthesis 9)

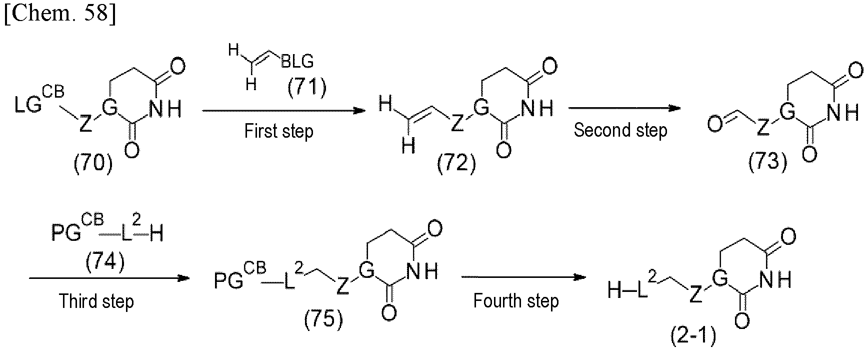

(In the formulae, LG^{CB} represents a leaving group, and PG^{CB} represents a protective group of NH contained in L².)

This production method is a method for producing a compound (2-1) included in the compound (2), which is a raw material compound of the Production Method 1, in which -L²-is an optionally substituted saturated heterocyclic divalent group containing two nitrogen atoms, and -L³⁵- is -CH₂-.

### (First step)

This step is a method for producing a compound (72) by a Suzuki-Miyaura coupling reaction of a compound (70) and a compound (71).

The reaction conditions are the same as in the fifth step of the Raw Material Synthesis 1.

### (Second step)

This step is a method for producing a compound (73) which is an aldehyde from the compound (72).

In this reaction, the compound (72) and osmium tetroxide are reacted in the presence of an organic base, in a solvent inactive for the reaction, from under cooling to room temperature, to obtain a corresponding 1,2-diol compound, and then the 1,2-diol is oxidized by adding periodic acids to the reaction mixture to thus obtain the compound (73), which is an aldehyde. Examples of the solvent used here include an alcohol, such as tBuOH, an ether, such as THF, DOX or 1,2-dimethoxyethane, an aromatic hydrocarbon, such as benzene, toluene or xylene, acetone and a mixed solvent of these compounds and water. Examples of the organic base used here include pyridine and 2,6-lutidine, and examples of the periodic acid used here include sodium periodate and periodic acid.

### (Third step)

This step is a method for producing a compound (75) by a reductive amination reaction using the compound (73) and a compound (74) which is an amine compound.

The reaction conditions are the same as when -R^{Z} is -CHO in the third step of the Raw Material Synthesis 4.

### (Fourth step)

This step is a reaction step of obtaining a compound (2-1) by subjecting the compound (75) to a deprotection reaction.

The reaction conditions are the same as in the Production Method 2.

The pharmacological activities of the compound of the formula (I) were confirmed by the following tests.

### Test Example 1: Evaluation of RAS G12V degradation activity on human KRAS G12V mutant-positive pancreatic cancer line PA-TU-8902

The RAS G12V degradation activity of test compounds was evaluated by measuring the expression levels of RAS G12V by Cell ELISA.

PA-TU-8902 cells (DSMZ, ACC 179) were seeded at 36 µL per well on 384-well plates (from Greiner bio-one) to give 1.5 x 10⁴ cells per well. As for the cell culture conditions, DMEM medium (from Sigma-Aldrich) containing 10% fetal bovine serum (from Cytiva) was used in the presence of 5% CO₂ at 37°C.

The next day, the test compounds (10 points having final concentrations in the range of 3 µM to 0.1 nM), the compound of Example No. 7 having a final concentration of 3 µM as a positive control and dimethyl sulfoxide (DMSO), which was the solvent for the test compounds, as a negative control were diluted 100-fold with a fresh medium and were added at 4 µL per well. The cells were cultured for 24 hours.

The next day, the culture supernatant was removed, and 4% paraformaldehyde phosphate buffer (from FUJIFILM Wako Pure Chemical Corporation) was added at 20 µL per well. The plates were allowed to stand for 30 minutes at room temperature to thus immobilize the cells. Then, the supernatant was removed, and 0.1% Triton X-100 (from Amersham Biosciences)-containing Phosphate buffered saline (PBS) was added at 20 µL per well. After allowing to stand at room temperature for 10 minutes, the supernatant was removed. PBS was added at 25 µL per well, and the supernatant was removed to thus wash each well. The washing was performed twice in total. Subsequent washing operations were performed in the same manner. Next, the supernatant was removed, and 0.5% sodium dodecyl sulfate (SDS; from ThermoFisher Scientific)-containing PBS was added at 20 µL per well. After allowing to stand at room temperature for 10 minutes, the supernatant was removed by centrifugation of the plates. After washing with PBS, the supernatant was removed by centrifugation, and blocking solution (PVDF Blocking Reagent for Can Get Signal [from TOYOBO Co., Ltd.]) was added at 20 µL per well. After allowing to stand for 30 minutes at room temperature, the supernatant was removed by centrifugation and a solution obtained by diluting, as primary antibodies, anti-Ras (G12V Mutant Specific) antibody (Ras (G12V Mutant Specific) (D2H12) Rabbit mAb; from Cell Signaling Technology; 500-fold diluted) and anti-β-actin antibody (Anti-beta Actin antibody; from Abcam; 5,000-fold diluted) with Can Get Signal Solution 2 (from TOYOBO Co., Ltd.) was added at 15 µL per well. The plates were allowed to stand overnight at 4°C.

The next day, the supernatant was removed by centrifugation and washed with PBS. The supernatant was removed by centrifugation, a solution obtained by diluting, as secondary antibodies, anti-rabbit IgG antibody (IRDye 800CW Goat anti-Rabbit IgG; from LI-COR Biosciences) and anti-mouse IgG antibody (IRDye 680RD Donkey anti-Mouse IgG; from LI-COR Biosciences) 1,000-fold with Can Get Signal Solution 2 was added at 15 µL per well. After allowing to stand at room temperature for 1 hour, the supernatant was removed by centrifugation and washed with PBS. After removing the supernatant, the plates were dried with air at room temperature for 2 hours or more, and the 700 nm and 800 nm fluorescent signals were measured with Aerius (from LI-COR Biosciences).

The degradation rate of RAS G12V was calculated with the RAS G12V signaling value at the time of addition of DMSO taken as 0%, which was corrected with the signaling value of β-actin, and with the RAS G12V signaling value at the time of addition of the compound of Example No. 7 at a final concentration of 3 µM taken as 100%. The 50% degradation concentration values (DC₅₀) of RAS G12V were calculated by Sigmoid-Emax nonlinear regression analysis based on the degradation rate up to the concentration of the compound showing Dmax, where Dmax was the degradation rate at the concentration of the compound showing the highest degradation activity. The results for some test compounds of the formula (I) are shown in tables below.

**[Table 1]**

| Ex | DC₅₀ (nM) | Dmax (%) | Ex | DC₅₀ (nM) | Dmax (%) |
|---|---|---|---|---|---|
| 1 | 6 | 77 | 11 | 11 | 88 |
| 2 | 4 | 89 | 12 | 13 | 87 |
| 3 | 29 | 74 | 13 | 27 | 104 |
| 4 | 7 | 83 | 14 | 4 | 100 |
| 5 | 5 | 97 | 15 | 92 | 85 |
| 6 | 14 | 99 | 16 | 162 | 95 |
| 7 | 38 | 103 | 17 | 9 | 70 |
| 8 | 5 | 81 | 18 | 65 | 78 |
| 9 | 6 | 88 | 19 | 26 | 78 |
| 10 | 6 | 88 | 20 | 29 | 85 |

### Test Example 2: Evaluation of ERK phosphorylation inhibitory effect on human KRAS G12V mutant-positive pancreatic cancer line PA-TU-8902

The ERK phosphorylation inhibitory effect of test compounds is evaluated by measuring phosphorylation of 202th threonine (Thr202) and 204th tyrosine (Tyr204) of ERK on the downstream of the KRAS signal by Cell ELISA.

PA-TU-8902 cells are seeded on 384-well plates at 36 µL/well to give 5.0 x 10³ cells per well. The cell culture is performed under the same conditions as in Test Example 1.

The next day, the test compounds (9 points having final concentrations in the range of 3 µM to 0.3 nM), trametinib (MEK inhibitor) having a final concentration of 300 nM as a positive control and DMSO, which is the solvent for the test compounds, as a negative control are diluted 100-fold with a fresh medium and are added at 4 µL per well. The cells are cultured for 24 hours. After culturing, 30% glyoxal solution (40% glyoxal [from Nacalai Tesque] diluted with PBS) is quickly added at 30 µL per well. The plates are allowed to stand for 1 hour and 30 minutes at room temperature to thus immobilize the cells. Then, the supernatant is removed by centrifugation (110 x g for 7 seconds, unless otherwise stated below under the same conditions) of the plates, and 0.1% Triton X-100-containing PBS is added at 20 µL per well. After allowing to stand for 10 minutes at room temperature, the supernatant is removed by centrifugation, and the same operation is repeated. Next, 0.5% SDS-containing PBS is added at 20 µL per well. After allowing to stand at room temperature for 30 minutes, the supernatant is removed by centrifugation. Subsequently, blocking solution (Intercept Blocking Buffer) is added at 20 µL per well, and the plates are allowed to stand for 1 hour at room temperature. The supernatant is removed by centrifugation, and an ERK (Thr202/Tyr204) phosphorylation antibody (Phospho-p44/42 MAPK (Erk 1/2) (Thr202/Tyr204) (D13.14.4E) XP Rabbit mAb; from Cell Signaling Technology) diluted 2,500-fold with blocking solution is added at 15 µL per well as a primary antibody. The plates are allowed to stand at 4°C overnight.

The next day, the supernatant is removed by centrifugation of the plates, and 0.05% Tween-20-containing PBS (from Thermo Scientific; 20 x PBS Tween-20 diluted 20-fold with ion exchange water for use) is added at 50 µL per well, and the supernatant is removed by centrifugation to thus wash each well. The washing is performed three times in total.

After washing, an anti-rabbit antibody diluted 1,000-fold with blocking solution is added at 15 µL per well as a secondary antibody, and the plates are allowed to stand for 1 hour at room temperature. The supernatant is removed by centrifugation of the plates, and each well is washed three times with 0.05% Tween-20-containing PBS in the same manner as after primary antibody reaction. Centrifugation after the third washing is performed at 171 x g for 17 seconds. After removing the supernatant, the plates are dried with air at room temperature for 3 hours or more, and 800 nm fluorescent signals are measured with Aerius.

With the signaling value at the time of addition of DMSO taken as 0% inhibition and with the signaling value at the time of addition of 300 nM trametinib taken as 100% inhibition, the 50% inhibitory concentration values (IC₅₀) are calculated by Sigmoid-Emax nonlinear regression analysis.

### Test Example 3: Evaluation of non-anchorage-dependent cell growth inhibitory effect on human KRAS G12V mutant-positive pancreatic cancer line PA-TU-8902

The non-anchorage-dependent cell growth inhibitory effect of test compounds was evaluated by spheroid 3D cell culture.

PA-TU-8902 cells were seeded on low-cell-adhesive round bottom 384-well plates (PrimeSurface: from Sumitomo Bakelite) at 36 µL/well to give 5 x 10² cells per well. The cell culture was performed under the same conditions as in Test Example 1.

The next day, the test compounds (8 to 10 points having final concentrations in the range of 3 µM to 0.1 nM) and DMSO, which was the solvent for the test compounds, as a negative control were diluted 100-fold with a fresh medium and were added at 4 µL per well. After culturing in the presence of 5% CO₂ at 37°C for 6 days, Cell Titer Glo 2.0 (from Promega) was added at 20 µL per well. After stirring with a plate mixer (from FINEPCR) at normal temperature for 1 hour, the luminescent signals were measured with ARVO X3 (from PerkinElmer).

With the signaling value in treatment with DMSO taken as 0% inhibition and with the signaling value in the medium alone without cells taken as 100% inhibition, the 50% inhibitory concentration values (IC₅₀) were calculated by Sigmoid-Emax nonlinear regression analysis. The results for some test compounds of the formula (I) are shown in tables below. The inhibition rate at 30 nM was calculated for Example No. 17. Note that 50% inhibition @30 nM indicates that the test compound has 50% inhibition activity at a concentration of 30 nM.

**[Table 2-1]**

| Ex | IC₅₀ (nM) | Ex | IC₅₀ (nM) |
|---|---|---|---|
| 1 | 42 | 11 | 18 |
| 2 | 21 | 12 | 33 |
| 3 | 67 | 13 | 55 |
| 4 | 74 | 14 | 12 |
| 5 | 15 | 15 | 139 |
| 6 | 26 | 16 | 255 |
| 7 | 55 | 17 | 50% inhibition @30nM |
| 8 | 48 | 18 | 149 |
| 9 | 47 | 19 | 68 |
| 10 | 22 | 20 | 85 |

**[Table 2-2]**

| Ex | IC₅₀ (nM) |
|---|---|
| 21 | 11 |
| 22 | 5 |

### Test Example 4: Evaluation of non-anchorage-dependent cell growth inhibitory effect on human KRAS G12V mutant-positive lung adenocarcinoma line LCLC-97TM1

The non-anchorage-dependent cell growth inhibitory effect of test compounds was evaluated by spheroid 3D cell culture.

LCLC-97TM1 cells (DSMZ, ACC 388) were seeded on low-cell-adhesive round bottom 384-well plates (Prime Surface: from Sumitomo Bakelite) at 36 µL/well to give 7.5 x 10² cells per well. As for the cell culture conditions, RPMI-1640 medium (from FUJIFILM Wako Pure Chemical Corporation) containing 10% fetal bovine serum was used in the presence of 5% CO2 at 37°C.

The next day, the test compounds (9 points having final concentrations in the range of 10 µM to 0.1 nM) and DMSO, which was the solvent for the test compounds, as a negative control were diluted 100-fold with a fresh medium and were added at 4 µL per well. After culturing in the presence of 5% CO₂ at 37°C for 6 days, Cell Titer Glo 2.0 (from Promega) was added at 20 µL per well. After stirring with a plate mixer (from FINEPCR) at room temperature for 1 hour, the luminescent signals were measured with ARVO X3 (from PerkinElmer).

With the signaling value in treatment with DMSO taken as a survival rate of 100% and with the signaling value in the medium alone without cells taken as a survival rate of 0%, the 50% survival values (IC50) were calculated by Sigmoid-Emax nonlinear regression analysis.

### Test Example 5: Evaluation of anti-tumor activity in mouse bearing human KRAS G12V mutant-positive pancreatic cancer line PA-TU-8902

PA-TU-8902 cells are cultured under the same conditions as in Test Example 1. The PA-TU-8902 cells are recovered and suspended in PBS, and a cell suspension prepared at 1.0 to 3.0 x 10⁷ cells/mL with two equal volumes of VitroGel Hydrogel Matrix (from TheWell Bioscience Inc.) is subcutaneously inoculated into 4- to 6-week-old male nude mice (BALB/c-nu (nu/nu), from Charles River Laboratories Japan, Inc.) in a volume of 100 µL. About two weeks after the inoculation, the mice are divided into groups so that all the groups have approximately the same tumor volume and body weight, and administration of test compounds is started on the next day. The test is conducted for five mice for each of a solvent group and a test compound administration group. The test compounds are dissolved in a solvent containing ethanol (from FUJIFILM Wako Pure Chemical Corporation), a 5% glucose solution (from Otsuka Pharmaceutical), 1 M hydrochloric acid (from Kanto Chemical Co., Inc.), 50% (2-hydroxypropyl)-β-cyclodextrin (HP-βCD) solution (from ROQUETTE), HCO-40 (from Nikko Chemicals Co., Ltd.) and 1 M sodium hydroxide solution (from Kanto Chemical Co., Inc.) at a liquid amount ratio of 4:84.4:1.1:1:9:0.5. The test compound dissolved in a solvent or the solvent is administered into the tail vein. The administration is performed once or twice a week for up to three weeks. The tumor size and the body weight are measured twice a week. The tumor volume is calculated using the following equation. [Tumor volume (mm3)] = [Long diameter of tumor (mm)] x [Short diameter of tumor (mm)]2 x 0.5

The tumor growth inhibition rate (%) by the test compound is calculated with the tumor volume of the test compound administration group on the previous day of the start of the administration taken as 100% inhibition and the tumor volume of the solvent group on the day of the final measurement taken as 0% inhibition. In addition, when the tumor volume of a test compound administration group is smaller than the tumor volume on the previous day of the start of the administration, the tumor regression rate (%) by the test compound is calculated with the tumor volume on the previous day of the start of the administration taken as 0% regression and with the tumor volume 0 taken as 100% regression.

### Test Example 6: Evaluation of anti-tumor activity in mice bearing human G12V mutant KRAS-positive lung adenocarcinoma line LCLC-97TM1

The cell culture was performed under the same conditions as in Test Example 4. The LCLC-97TM1 cells were recovered and suspended in PBS, and a cell suspension prepared at 3.0 x 10⁶ cells/100 µL per mouse with two equal volumes of VitroGel Hydrogel Matrix (from TheWell Bioscience Inc.) was subcutaneously inoculated into 4- to 6-week-old male nude mice (BALB/c-nu (nu/nu), from Charles River Laboratories Japan, Inc.). After about two to three weeks of the inoculation, the mice were divided into groups so that all the groups had approximately the same tumor volume, and administration of a test compound was started. The test was conducted for 5 mice each of a solvent group and a test compound administration group. The solvent and a solution of the test compound dissolved in the solvent were administered to the solvent group and the test compound administration group, respectively, into the tail vein. The same solvent as in Test Example 5 was used. The administration is performed once a week and twice in total. The tumor size and the body weight were measured twice a week. The tumor volume was calculated using the following equation. [Tumor volume (mm3)] = [Long diameter of tumor (mm)] x [Short diameter of tumor (mm)]2 x 0.5

The tumor growth inhibition rate (%) by the test compound was calculated with the tumor volume of the test compound administration group on the previous day of the start of the administration taken as 100% inhibition and the tumor volume of the solvent group on the day of the final measurement taken as 0% inhibition. In addition, when the tumor volume of a test compound administration group was smaller than the tumor volume on the previous day of the start of the administration, the tumor regression rate (%) by the test compound was calculated with the tumor volume on the previous day of the start of the administration taken as 0% regression and with the tumor volume 0 taken as 100% regression.

As a result of the above tests, the degradation-inducing action on G12V mutant KRAS was observed for some compounds of the formula (I). Moreover, cell growth inhibitory effects on human G12V mutant KRAS-positive pancreatic cancer and/or lung cancer lines were observed for some compounds of the formula (I). Anti-tumor activity of some compounds of the formula (I) was observed on mice bearing human G12V mutant KRAS-positive lung cancer lines. Therefore, the compounds of the formula (I) can be used for the treatment of pancreatic cancer and/or lung cancer, in particular, KRAS G12V mutant-positive pancreatic cancer and/or lung cancer, and the like.

A pharmaceutical composition that contains one or two or more compounds of the formula (I) or salts thereof as active ingredients can be prepared by a usually used method using an excipient usually used in the art, namely, a pharmaceutical excipient, a pharmaceutical carrier or the like.

The administration may be either oral administration with a tablet, pill, capsule, granule, powder, liquid or other agent or parenteral administration with an intraarticular, intravenous, intramuscular or other injection, a transmucosal agent, an inhalant or the like.

As a solid composition for oral administration, a tablet, powder, granular or other agent is used. In such a solid composition, one or two or more active ingredients are mixed with at least one inactive excipient. The composition may contain an inactive additive, for example, a lubricant, a disintegrator, a stabilizer or a dissolution aid, according to an ordinary method. A tablet or pill may be coated with a sugar coating or a film soluble in the stomach or intestine, as needed.

Liquid compositions for oral administration include a pharmaceutically acceptable emulsion, solution, suspension, syrup, elixir agent and the like and contain a generally used inactive diluent, for example, purified water or EtOH. The liquid composition may contain, in addition to the inactive diluent, an adjuvant, such as a solubilizer, a wetting agent or a suspending agent, a sweetening agent, a flavor, an aromatic or a preservative.

The injection agents for parenteral administration include a sterile aqueous or nonaqueous solution, suspension or emulsion agent. Examples of the aqueous solvent include distilled water for injection or physiological saline. An example of the nonaqueous solvent is an alcohol, such as EtOH. Such a composition may further contain an isotonizing agent, a preservative, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition, which is dissolved or suspended in sterile water or a sterile solvent for injection before use.

The transmucosal agent, such as an inhalant or a transnasal agent, is used in a solid, liquid or semi-solid form and can be produced according to a conventionally known method. For example, a known excipient and in addition, a pH modifier, a preservative, a surfactant, a lubricant, a stabilizer, a thickener or the like may be appropriately added. The administration can be performed using an appropriate device for inhalation or insufflation. For example, the agent can be administered using a known device, such as a metering and administering inhalation device, or an atomizer, as a compound alone or a powder of a mixture formulated, or as a solution or a suspension in combination with a pharmaceutically acceptable carrier. A dry powder inhaler or the like may be for a single administration or multiple administrations, and dry powder or powder-containing capsule can be used. Alternatively, the agent may be used in a form of a pressurized aerosol spray or the like using an appropriate ejection agent, for example, a suitable gas, such as a chlorofluoroalkane or carbon dioxide.

In the case of a common oral administration, the daily dose is appropriately about 0.001 to 100 mg/kg body weight, preferably 0.1 to 30 mg/kg body weight, further preferably 0.1 to 10 mg/kg body weight, and the dose is given once or is divided into two to four times in a day. In the case of intravenous administration, the daily dose is appropriately about 0.0001 to 10 mg/kg body weight and is given once or is divided into multiple times in a day. In addition, the daily dose of a transmucosal agent is about 0.001 to 100 mg/kg body weight and is given once or is divided into multiple times in a day. The dose is appropriately decided depending on the individual case taking the symptom, age, sex and the like into account.

Depending on the route of administration, dosage form, site of administration and types of excipient and additive, the pharmaceutical composition of the present invention contains 0.01 to 100% by weight, in one embodiment, 0.01 to 50% by weight, of one or more compound of the formula (I) or salts thereof which are active ingredients.

The compounds of the formula (I) can be used in combination with various therapeutic agents or preventive agents for a disease to which the compounds of the formula (I) are considered to have an effectiveness. The combination use may be simultaneous administration or separate administration either sequential or with a desired interval. A simultaneous administration preparation may be a formulated agent or may be separately formulated.

### Examples

The production method of the compounds of the formula (I) will be described in further detail below based on Examples. Note that the present invention is not to be limited to the compounds described in the following Examples. The production methods of raw material compounds are also shown in the Production Examples. The production methods of the compounds of the formula (I) are not limited only to the production methods of specific Examples described below, and the compounds of the formula (I) can also be produced by a combination of the production methods or a method that is obvious to a person skilled in the art.

Note that, in this specification, a compound is sometimes named using naming software, such as ACD/Name (registered trademark, Advanced Chemistry Development, Inc.).

For the purpose of convenience, the concentration mol/L is shown as M. For example, 1 M aqueous sodium hydroxide solution means an aqueous sodium hydroxide solution of 1 mol/L.

### Production Example 1

In THF (310 mL), 7-bromo-2,4-dichloro-8-fluoro-6-iodoquinazoline (30.9 g) was suspended, sodium hydroxide (1M aqueous solution, 147 mL) was added dropwise under ice-bath cooling so that the internal temperature was 12°C or lower, and the mixture was stirred for 2 hours under ice-bath cooling. The reaction solution was poured into an Erlenmeyer flask containing hydrochloric acid (1M aqueous solution, 147 mL) and water (700 mL) under ice-bath cooling, and the mixture was stirred at room temperature for 2 hours. The insoluble materials were filtered while washing with water and were dried under reduced pressure at 40°C overnight, thus obtaining 7-bromo-2-chloro-8-fluoro-6-iodoquinazolin-4-ol (24.6 g) as a solid.

### Production Example 2

Under nitrogen atmosphere, to a mixture of 7-bromo-2-chloro-8-fluoro-6-iodoquinazolin-4-ol (24.6 g) and THF (260 mL), which was heated to 60°C, was added 2-tert-butyl-1,3-diisopropylisourea (73.4 g) dropwise over 15 minutes, and the mixture was stirred at the same temperature for 2.5 hours. After allowing to cool to room temperature, the insoluble materials were separated by filtration while washing with THF (500 mL). The filtrate was concentrated, MeOH (210 mL) was added to the resulting solid, and the mixture was suspended and washed by stirring at room temperature for 1 hour. The insoluble materials were filtered with MeOH (100 mL) to give 7-bromo-4-tert-butoxy-2-chloro-8-fluoro-6-iodoquinazoline (23.2 g) as a solid.

### Production Example 3

To a dichloromethane (300 mL) suspension of 7-bromo-4-tert-butoxy-2-chloro-8-fluoro-6-iodoquinazoline (29 g) were added ethanethiol (5 mL) and DABCO (11 g) at room temperature, and under an argon atmosphere, the mixture was stirred at room temperature overnight. The reaction was quenched with water under ice-bath cooling. Chloroform was added, the organic layer and the aqueous layer were separated by a separation operation, and the aqueous layer was extracted with chloroform three times. The collected organic layer was dried over anhydrous sodium sulfate, was filtered and was concentrated under reduced pressure, thus obtaining 7-bromo-4-tert-butoxy-2-(ethylsulfanyl)-8-fluoro-6-iodoquinazoline (32 g) as a solid.

### Production Example 4

To a THF (400 mL) solution of 7-bromo-4-tert-butoxy-2-(ethylsulfanyl)-8-fluoro-6-iodoquinazoline (32 g) and (1S)-1-phenylethan-1-ol (11 mL) was added tBuOK (10 g) under ice-bath cooling, and the mixture was stirred under ice-bath cooling for 1 hour under an argon atmosphere. The reaction was quenched with saturated aqueous ammonium chloride solution under ice-bath cooling. Water and ethyl acetate were added, the organic layer and the aqueous layer were separated, and the organic layer was washed with saturated aqueous sodium chloride solution, was dried over anhydrous sodium sulfate and was filtered. The filtrate was concentrated under reduced pressure. The residue was purified by silica gel column chromatography (hexane/ethyl acetate), thus obtaining 7-bromo-4-tert-butoxy-2-(ethylsulfanyl)-6-iodo-8-[(1S)-1-phenylethoxy]quinazoline (36.6 g) as an oil.

### Production Example 5

At room temperature, 7-bromo-4-tert-butoxy-2-(ethylsulfanyl)-6-iodo-8-[(1S)-1-phenylethoxy]quinazoline (36.6 g), cyclopropylboronic acid (7.5 g), PdCl₂(dppf)·CH₂Cl₂(7.6 g), tripotassium phosphate (53 g), MeCN (440 mL) and water (80 mL) were mixed and were stirred at 90°C for 4 hours under an argon atmosphere. After the temperature was returned to room temperature, the reaction solution was diluted with ethyl acetate and water. The organic layer and the aqueous layer were separated, and the organic layer was washed with saturated aqueous sodium chloride solution, was dried over anhydrous sodium sulfate and was filtered. The filtrate was concentrated under reduced pressure. The residue was purified by silica gel column chromatography (hexane/ethyl acetate), thus obtaining 7-bromo-4-tert-butoxy-6-cyclopropyl-2-(ethylsulfanyl)-8-[(1S)-1-phenylethoxy]quinazoline (22.9 g) as an oil.

### Production Example 6

To 7-bromo-4-tert-butoxy-6-cyclopropyl-2-(ethylsulfanyl)-8-[(1S)-1-phenylethoxy]quinazoline (14.21 g) were added 6-fluoro-5-methyl-4-(4,4,5,5-tetramethyl-1,3,2-dioxaborolan-2-yl)-2-(triphenylmethyl)-2H-indazole (19.3 g), palladium(II) acetate (0.67 g), dicyclohexyl(2',6'-diisopropoxy-[1,1'-biphenyl]-2-yl)phosphine (2.67 g), anhydrous barium hydroxide (14.6 g), DOX (500 mL) and water (100 mL), degassing and argon gas substitution operations were performed several times. Then, the mixture was heated and stirred at 50°C overnight under an argon atmosphere. The reaction suspension which was allowed to cool was filtered through celite (registered trademark) pad while washing with ethyl acetate, and the gray insoluble materials were removed by filtration. After the filtrate was concentrated to about 1/4 under reduced pressure, water was added, and the mixture was extracted twice with ethyl acetate. The collected organic layer was washed with saturated aqueous sodium chloride solution, was dried over anhydrous magnesium sulfate and was concentrated under reduced pressure. The resulting residue was purified by silica gel column chromatography (hexane/ethyl acetate), thus obtaining 4-tert-butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (about 3.3:1 diastereomeric mixture derived from axial chirality, 16.44 g) as a solid.

### Production Example 7

In dichloromethane (300 mL), 4-tert-butoxy-6-cyclopropyl-2-(ethylsulfanyl)-7-[6-fluoro-5-methyl-2-(triphenylmethyl)-2H-indazol-4-yl]-8-[(1 S)-1-phenylethoxy]quinazoline (about 3.7:1 diastereomeric mixture derived from axial chirality, 22.6 g) was dissolved, and m-chloroperbenzoic acid (about 30% water content, 15 g) was added under ice-bath cooling (internal temperature: 5 to 10°C). Under nitrogen atmosphere, the mixture was stirred for 2 hours at room temperature. Under ice-bath cooling, an aqueous solution (300 mL) of sodium thiosulfate pentahydrate (14 g) and saturated aqueous sodium hydrogen carbonate solution (300 mL) were poured into the reaction mixture, and the mixture was stirred for 30 minutes at room temperature and was then extracted twice with ethyl acetate. The combined organic layer was washed with saturated aqueous sodium hydrogen carbonate solution and saturated aqueous sodium chloride solution, was dried over anhydrous magnesium sulfate and was filtered. The filtrate was concentrated. iPrOH (600 mL) was added to the resulting residue, and the mixture was stirred at room temperature overnight. The insoluble materials were filtered, were washed with iPrOH and were dried under reduced pressure, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (about 1:1 diastereomeric mixture derived from axial chirality, 9.5 g, Production Example 7-2) as a solid. The filtrate was concentrated, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (single diastereomer with undetermined axial chirality configuration, 15.5 g, Production Example 7-1) as a foam-like solid.

### Production Example 8

To a THF (20 mL) solution of 4-tert-butoxy-6-cyclopropyl-2-(ethanesulfonyl)-7-[6-fluoro-5-methyl-2-(triphenylmethyl)-2H-indazol-4-yl]-8-[(1 S)-1-phenylethoxy]quinazoline (1386 mg) and (2S)-2-methoxypropan-1-ol (189 µL) was added tBuOK (254 mg) in an ice-MeOH bath, and under nitrogen atmosphere, the mixture was stirred at the same temperature for 30 minutes. Saturated aqueous ammonium chloride solution was added to the reaction mixture under ice-bath cooling, and the mixture was extracted twice with ethyl acetate. The combined organic layer was washed with saturated aqueous sodium chloride solution, was dried over anhydrous sodium sulfate and was then filtered. The filtrate was concentrated under reduced pressure. The residue was purified by silica gel column chromatography (hexane/ethyl acetate), thus obtaining 4-tert-butoxy-6-cyclopropyl-7-[6-fluoro-5-methyl-2-(triphenylmethyl)-2H-indazol-4-yl]-2-[(2S)-2-methoxypropoxy]-8-[(1S)-1-phenylethoxy]quinazoline (1.25 g) as an oil.

### Production Example 9

To a mixture of 4-bromo-6-fluoro-1H-indazole (235 g), TEA (183 mL) and dichloromethane (1880 mL) was added 1, 1', 1 "-(chloromethanetriyl)tribenzene (335 g), and the mixture was stirred at 25°C for 16 hours. The reaction mixture was poured into ice water (1.5 L), and the organic layer and the aqueous layer were separated. The aqueous layer was extracted with dichloromethane (400 mL) three times. The combined organic layer was dried over anhydrous sodium sulfate. Then, the insoluble materials were removed by filtration, and the filtrate was concentrated under reduced pressure. Petroleum ether (550 mL) was added to the resulting residue for trituration (0°C, 2 hours), and then 4-bromo-6-fluoro-2-(triphenylmethyl)-2H-indazole (508.98 g) was obtained as a solid by collecting by filtration and drying under reduced pressure.

### Production Example 10

To a 2-methyltetrahydrofuran (1000 mL) mixture of 4-bromo-6-fluoro-2-(triphenylmethyl)-2H-indazole (100 g) was added lithium diisopropylamide (2 M THF solution, 214.28 mL) at -78°C under nitrogen atmosphere, and the mixture was stirred at - 78°C for 2.5 hours. Methyl iodide (26.68 mL) was added at -78°C, and the mixture was stirred at 25°C for 2.5 hours. Water (2000 mL) was added to quench the reaction, and the mixture was extracted twice with ethyl acetate (800 mL). The combined organic layer was dried over anhydrous sodium sulfate. Then, the insoluble materials were removed by filtration, and the filtrate was concentrated under reduced pressure. Ethyl acetate (50 mL)/petroleum ether (50 mL) were added to the resulting residue for trituration, and then 4-bromo-6-fluoro-5-methyl-2-(triphenylmethyl)-2H-indazole (81 g) was obtained as a solid by collecting by filtration and drying under reduced pressure.

### Production Example 11

To a mixture of 4-bromo-6-fluoro-5-methyl-2-(triphenylmethyl)-2H-indazole (100 g), 4,4,4',4',5,5,5',5'-octamethyl-2,2'-bi-1,3,2-dioxaborolane (61.42 g), triphenylphosphine (10.57 g), potassium acetate (59.34 g) and DOX (1000 mL) was added palladium(II) acetate (4.52 g) under nitrogen atmosphere at room temperature. After the reaction mixture was degassed and filled with nitrogen gas three times each, the mixture was stirred at 100°C for 12 hours under nitrogen atmosphere. After cooling, water (1500 mL) was added, and the mixture was extracted with ethyl acetate (900 mL) three times. The combined organic layer was dried over anhydrous sodium sulfate, and then the insoluble materials were removed by filtration. Activated carbon (50 g) was added to the resulting solution, and the solution was stirred at 20°C for 1 hour and filtered while washing with ethyl acetate (50 mL) three times. The filtrate was concentrated under reduced pressure. MeOH (200 mL) was added to the resulting residue for trituration, and 6-fluoro-5-methyl-4-(4,4,5,5-tetramethyl-1,3,2-dioxaborolan-2-yl)-2-(triphenylmethyl)-2H-indazole (110 g) was obtained as a solid by collecting by filtration and drying under reduced pressure.

### Production Example 12

To a solution in MeOH (15 mL) and THF (15 mL) of 4-tert-butoxy-6-cyclopropyl-7-[6-fluoro-5-methyl-2-(triphenylmethyl)-2H-indazol-4-yl]-2-[(2S)-2-methoxypropoxy]-8-[(1S)-1-phenylethoxy]quinazoline (1.25 g) were added sodium hydrogen carbonate (1.62 g) and 10% Pd/C (about 50% water content, 609 mg), and the mixture was stirred under a hydrogen atmosphere at normal temperature and normal pressure for 3 hours. Another 10% Pd/C (about 50% water content, 304 mg) was added, and the mixture was stirred under a hydrogen atmosphere at normal temperature and normal pressure for 3 hours. The mixture was filtered through celite (registered trademark) pad using chloroform/iPrOH (4/1) and EtOH/water (10/1), and the filtrate was concentrated. The residue was purified by silica gel column chromatography (hexane/ethyl acetate), thus obtaining 4-tert-butoxy-6-cyclopropyl-7-[6-fluoro-5-methyl-2-(triphenylmethyl)-2H-indazol-4-yl]-2-[(2S)-2-methoxypropoxy]quinazolin-8-ol (923 mg) as a solid.

### Production Example 13

To a DMF (10 mL) solution of 4-tert-butoxy-6-cyclopropyl-7-[6-fluoro-5-methyl-2-(triphenylmethyl)-2H-indazol-4-yl]-2-[(2S)-2-methoxypropoxy]quinazolin-8-ol (1115 mg) were added DIPEA (1036 µL), tert-butyl 4-(chloromethyl)benzoate (515 mg) and cesium carbonate (2220 mg) at room temperature in this order, and under nitrogen atmosphere, the mixture was stirred at room temperature for 4 hours. Water and ethyl acetate were added to the reaction solution, and the mixture was extracted twice with ethyl acetate. The organic layer was washed with saturated aqueous sodium chloride solution, was dried over anhydrous sodium sulfate and was then filtered. The filtrate was concentrated under reduced pressure, and the residue was purified by silica gel column chromatography (hexane/ethyl acetate), thus obtaining tert-butyl 4-[({(7M)-4-tert-butoxy-6-cyclopropyl-7-[6-fluoro-5-methyl-2-(triphenylmethyl)-2H-indazol-4-yl]-2-[(2S)-2-methoxypropoxy]quinazolin-8-yl}oxy)methyl]benzoate (1347 mg) as a solid.

### Production Example 14

To a THF (20 mL) solution of tert-butyl 4-[({(7M)-4-tert-butoxy-6-cyclopropyl-7-[6-fluoro-5-methyl-2-(triphenylmethyl)-2H-indazol-4-yl]-2-[(2S)-2-methoxypropoxy]quinazolin-8-yl}oxy)methyl]benzoate (1347 mg) were added 4-methylbenzene-1-sulfonic acid monohydrate (216 mg), 3,4-dihydro-2H-pyran (1.08 mL) at room temperature, and under nitrogen atmosphere, the mixture was stirred at room temperature overnight. Saturated aqueous sodium hydrogen carbonate solution was added to the reaction mixture, and the mixture was extracted with ethyl acetate. The organic layer was dried over anhydrous sodium sulfate, was filtered and was concentrated. The residue was purified by silica gel column chromatography (hexane/ethyl acetate), thus obtaining tert-butyl 4-[({(7M)-6-cyclopropyl-7-[6-fluoro-5-methyl-1-(oxan-2-yl)-1H-indazol-4-yl]-4-hydroxy-2-[(2S)-2-methoxypropoxy]quinazolin-8-yl}oxy)methyl]benzoate (966 mg) as a solid.

### Production Example 17

To a solution in THF (1 mL) and MeCN (1 mL) of methyl 4-[({4-tert-butoxy-6-cyclopropyl-7-[6-fluoro-5-methyl-2-(triphenylmethyl)-2H-indazol-4-yl]-2-[(2S)-2-methoxypropoxy]quinazolin-8-yl}oxy)methyl]benzoate (100 mg) was added sodium hydroxide (1 M aqueous solution, 1000 µL) at room temperature, and the mixture was stirred at 50°C for 5 hours. Hydrochloric acid (1 M aqueous solution, 1000 µL) and water were added, and the mixture was extracted with chloroform/iPrOH (4/1). Then, the organic layer was dried over anhydrous sodium sulfate, was filtered and was concentrated, thus obtaining 4-[({4-tert-butoxy-6-cyclopropyl-7-[6-fluoro-5-methyl-2-(triphenylmethyl)-2H-indazol-4-yl]-2-[(2S)-2-methoxypropoxy]quinazolin-8-yl}oxy)methyl]benzoic acid (111 mg) as an oil.

### Production Example 20

Under ice-bath cooling, to a THF (50 mL) solution of N,N-dimethylethylenediamine (6 mL) was added CDI (8.9 g) under ice-bath cooling, and under nitrogen atmosphere, the mixture was stirred at room temperature for 2 hours. The reaction mixture was concentrated under reduced pressure, iPrOH (50 mL) and azetidin-3-ol hydrochloride (5 g) were added to the residue at room temperature. Under an argon atmosphere, the mixture was stirred at 80°C for 3 hours. The reaction mixture was concentrated under reduced pressure, and the residue was purified by silica gel column chromatography (chloroform/MeOH/28% ammonia water), thus obtaining N-[2-(dimethylamino)ethyl]-3-hydroxyazetidine-1-carboxyamide (9.67 g) as an oil.

### Production Example 21

To a THF (2 mL) solution of tert-butyl 4-[({(7M)-6-cyclopropyl-7-[6-fluoro-5-methyl-1-(oxan-2-yl)-1H-indazol-4-yl]-4-hydroxy-2-[(2S)-2-methoxypropoxy]quinazolin-8-yl}oxy)methyl]benzoate (100 mg) were added PyBOP (180 mg) and cesium carbonate (120 mg) at room temperature, and under an argon atmosphere, the mixture was stirred at room temperature for 1 hour. A solution in THF (2 mL) and DIPEA (0.5 mL) of azetidine-3-carbonitrile monotrifluoroacetate (150 mg) and cesium carbonate (140 mg) were added at room temperature, and under an argon atmosphere, the mixture was stirred at room temperature for 2 hours. Ethyl acetate was added to the reaction mixture, and the insoluble materials were filtered. The filtrate was concentrated under reduced pressure, and the residue was purified by silica gel column chromatography (hexane/ethyl acetate), thus obtaining tert-butyl 4-[({(7M)-4-(3-cyanoazetidin-1-yl)-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoate (73.1 mg) as a solid.

### Production Example 22

To a mixture of tert-butyl 4-[({(7M)-4-(3-cyanoazetidin-1-yl)-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoate (72 mg) and dichloromethane (2 mL) was added TFA (0.4 mL) at room temperature, and the mixture was stirred at the same temperature overnight. The reaction mixture was concentrated under reduced pressure, chloroform and saturated aqueous sodium hydrogen carbonate solution were added to the residue, and the mixture was extracted with chloroform/iPrOH (4/1). The organic layer was washed with saturated aqueous sodium chloride solution, was dried over anhydrous sodium sulfate, and was filtered. The filtrate was concentrated under reduced pressure. Ethyl acetate and water were added to the residue, and the mixture was extracted with ethyl acetate. The organic layer was dried over anhydrous sodium sulfate, was filtered and was concentrated under reduced pressure, thus obtaining 4-[({(7M)-4-(3-cyanoazetidin-1-yl)-6-cyclopropyl-7-(6-fluoro-5-methyl-1H-indazol-4-yl)-2-[(2S)-2-methoxypropoxy]quinazolin-8-yl}oxy)methyl]benzoic acid (67.1 mg) as a solid.

### Production Example 29

To a THF (5 mL) solution of tert-butyl 4-[({(7M)-6-cyclopropyl-7-[6-fluoro-5-methyl-1-(oxan-2-yl)-1H-indazol-4-yl]-4-hydroxy-2-[(2S)-2-methoxypropoxy]quinazolin-8-yl}oxy)methyl]benzoate (50 mg) were added PyBOP (80 mg) and cesium carbonate (50 mg) at room temperature, and under nitrogen atmosphere, the mixture was stirred at room temperature for 1 hour. (S)-Azetidine-2-carboxamide (45 mg) and cesium carbonate (68 mg) were added at room temperature, and under nitrogen atmosphere, the mixture was stirred at room temperature overnight. Water was added to the reaction mixture at room temperature, and the mixture was extracted twice with ethyl acetate. The organic layer was dried over anhydrous sodium sulfate, was filtered and was concentrated. The residue was purified by silica gel column chromatography (hexane/ethyl acetate to chloroform/MeOH), thus obtaining tert-butyl 4-[({(7M)-4-[(2S)-2-carbamoylazetidin-1-yl]-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoate (80 mg) as an oil.

### Production Example 30

To a dichloromethane (5 mL) solution of tert-butyl 4-[({(7M)-4-[(2S)-2-carbamoylazetidin-1-yl]-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoate (80 mg) was added TFA (1 mL) at room temperature, and the mixture was stirred at room temperature for 5 hours. The reaction mixture was concentrated, and the residue was purified by silica gel column chromatography (chloroform/MeOH), thus obtaining 4-[({(7M)-4-[(2S)-2-carbamoylazetidin-1-yl]-6-cyclopropyl-7-(6-fluoro-5-methyl-1H-indazol-4-yl)-2-[(2S)-2-methoxypropoxy]quinazolin-8-yl}oxy)methyl]benzoic acid (45 mg) as a solid.

### Production Example 38

Tert-butyl 4-[({4-tert-butoxy-6-cyclopropyl-2-[3-(dimethylamino)-2,2-dimethylpropoxy]-7-[6-fluoro-5-methyl-2-(triphenylmethyl)-2H-indazol-4-yl]quinazolin-8-yl}oxy)methyl]benzoate (320 mg) was dissolved in a THF (6.2 mL), 4-methylbenzene-1-sulfonic acid monohydrate (66 mg) was added at room temperature, and under an argon atmosphere, the mixture was stirred at room temperature overnight. Saturated aqueous sodium hydrogen carbonate solution was added at room temperature, and the mixture was extracted twice with chloroform/iPrOH (4/1). The organic layer was dried over anhydrous sodium sulfate, was filtered and was concentrated under reduced pressure, thus obtaining tert-butyl 4-[({6-cyclopropyl-2-[3-(dimethylamino)-2,2-dimethylpropoxy]-7-(6-fluoro-5-methyl-1H-indazol-4-yl)-4-hydroxyquinazolin-8-yl}oxy)methyl]benzoate (222 mg) as an oil.

### Production Example 40

To a mixture of tert-butyl4-[({4-[(2S)-2-carbamoylazetidin-1-yl]-6-cyclopropyl-2-[3-(dimethylamino)-2,2-dimethylpropoxy]-7-(6-fluoro-5-methyl-1H-indazol-4-yl)quinazolin-8-yl}oxy)methyl]benzoate (245 mg) and dichloromethane (5 mL) was added TFA (1.5 mL) at room temperature, and the mixture was stirred at the same temperature for 5 hours. The reaction mixture was concentrated under reduced pressure. The residue was purified by ODS column chromatography (MeCN/0.1% aqueous formic acid solution), saturated aqueous sodium hydrogen carbonate solution was added to a fraction containing the peak at the lower polarity, and the mixture was extracted twice with chloroform/iPrOH (4/1). The organic layer was dried over anhydrous sodium sulfate, was filtered and was concentrated under reduced pressure, thus obtaining 4-({[(7M)-4-[(2S)-2-carbamoylazetidin-1-yl]-6-cyclopropyl-2-[3-(dimethylamino)-2,2-dimethylpropoxy]-7-(6-fluoro-5-methyl-1H-indazol-4-yl)quinazolin-8-yl]oxy}methyl)benzoic acid (single diastereomer, 36 mg) as a solid.

### Production Example 43

Tert-butyl 4-({[6-cyclopropyl-7-(6-fluoro-5-methyl-1H-indazol-4-yl)-4-hydroxy-2-{[(3R)-1-methylpyrrolidin-3-yl]methoxy}quinazolin-8-yl]oxy}methyl)benzoate (147 mg) was dissolved in THF (6 mL). Cesium carbonate (130 mg) and PyBOP (210 mg) were added at room temperature, and under nitrogen atmosphere, the mixture was stirred at room temperature for 1 hour. (S)-Azetidine-2-carboxamide (67 mg) and cesium carbonate (220 mg) were added, and under nitrogen atmosphere, the mixture was stirred at room temperature for 1 hour. Water and chloroform were added to the reaction mixture, and the mixture was extracted with chloroform three times. The combined organic layer was dried over anhydrous sodium sulfate, was filtered and was concentrated. The residue was purified by ODS column chromatography (MeCN/0.1% aqueous formic acid solution), and a fraction containing the peak at the lower polarity was collected and concentrated. The residue was dissolved in MeCN/water, and saturated aqueous sodium hydrogen carbonate solution was added. Then, the mixture was extracted twice with chloroform/iPrOH (9/1). The combined organic layer was dried over anhydrous sodium sulfate, was filtered and was concentrated, thus obtaining tert-butyl 4-({[(7M)-4-[(2S)-2-carbamoylazetidin-1-yl]-6-cyclopropyl-7-(6-fluoro-5-methyl-1H-indazol-4-yl)-2-{[(3R)-1-methylpyrrolidin-3-yl]methoxy}quinazolin-8-yl]oxy}methyl)benzoate (single diastereomer, 66 mg) as a solid.

### Production Example 51

Acetic acid (60 µL) was added to a mixture in dichloromethane (2.5 mL) and N-methyl-2-pyrrolidone (5 mL) of 1-(2,6-dioxopiperidin-3-yl)-3-methyl-2-oxo-2,3-dihydro-1H-benzimidazole-5-carbaldehyde (286 mg) and (S)-1-(tert-butoxycarbonyl)-2-methylpiperazine (405 mg), and the mixture was stirred at room temperature for 30 minutes. Sodium triacetoxyborohydride (424 mg) was added, and the mixture was stirred at room temperature for 16 hours. Water and saturated aqueous sodium hydrogen carbonate solution were added, and the mixture was stirred for a while and was extracted twice with ethyl acetate. The combined organic layer was washed with water and saturated aqueous sodium chloride solution and was dried over anhydrous magnesium sulfate. After separating the drying agent by filtration, the filtrate was concentrated under reduced pressure. The residue was purified by silica gel column chromatography (chloroform/iPrOH), thus obtaining tert-butyl (2S)-4-{[1-(2,6-dioxopiperidin-3-yl)-3-methyl-2-oxo-2,3-dihydro-1H-benzimidazol-5-yl]methyl}-2-methylpiperazine-1-carboxylate (470 mg) as a foam-like solid.

### Production Example 52

To a mixture of tert-butyl (2S)-4-{[1-(2,6-dioxopiperidin-3-yl)-3-methyl-2-oxo-2,3-dihydro-1H-benzimidazol-5-yl]methyl}-2-methylpiperazine-1-carboxylate (490 mg) and dichloromethane (10 mL) was added hydrochloric acid (4 M DOX solution, 8 mL), and under an argon atmosphere, the mixture was stirred at room temperature overnight. Isopropyl ether was added, and the mixture was suspended. The insoluble materials were filtered and were dried under reduced pressure, thus obtaining 3-(3-methyl-5-{[(3S)-3-methylpiperazin-1-yl]methyl}-2-oxo-2,3-dihydro-1H-benzimidazol-1-yl)piperidine-2,6-dione n hydrochloride (450 mg) as a solid.

### Production Example 53

In DOX (90 mL) and water (21 mL), 1-(6-bromo-1-methyl-1H-indazol-3-yl)-1,3-diazinane-2,4-dione (4143 mg), potassium vinyltrifluoroborate (6869 mg), PdCl₂(dppf)·CH₂Cl₂ (1047 mg) and cesium carbonate (8355 mg) were suspended, and under an argon atmosphere, the suspension was stirred at 80°C for 3 hours. After the reaction mixture was allowed to cool to room temperature, water was added, and the aqueous layer was extracted with ethyl acetate three times. The organic layer was washed with saturated aqueous sodium chloride solution, was dried over anhydrous magnesium sulfate and was filtered. The filtrate was concentrated under reduced pressure. The residue was purified by silica gel column chromatography (hexane/ethyl acetate to chloroform/MeOH), thus obtaining 1-(6-ethenyl-1-methyl-1H-indazol-3-yl)-1,3-diazinane-2,4-dione (3565 mg) as a solid.

### Production Example 54

In DOX (200 mL) and water (30 mL), 1-(6-ethenyl-1-methyl-1H-indazol-3-yl)-1,3-diazinane-2,4-dione (1590 mg) was dissolved, and to the mixture osmium(VIII) tetroxide (2.5 wt% tBuOH solution, 18.3 g) and 2,6-lutidine (1369 µL) were added. Sodium periodate (5023 mg) was gradually added to the reaction mixture under ice-bath cooling, and the mixture was stirred at room temperature for 2 hours. Under ice-bath cooling, an aqueous solution of sodium thiosulfate pentahydrate (30 g) was added, and the mixture was stirred at room temperature for 30 minutes. Water and saturated aqueous ammonium chloride solution were added, and the mixture was extracted with ethyl acetate five times. The organic layer was washed with saturated aqueous sodium chloride solution, was dried over anhydrous magnesium sulfate and was filtered. The filtrate was concentrated under reduced pressure, and the residue was purified by silica gel column chromatography (hexane/ethyl acetate to chloroform/MeOH). The resulting solid was filtered using hexane/ethyl acetate (2/1) to give 3-(2,4-dioxo-1,3-diazinan-1-yl)-1-methyl-1H-indazole-6-carbaldehyde (1344 mg) as a solid.

### Production Example 62

To a mixture of tert-butyl 6-{[3-(2,4-dioxo-1,3-diazinan-1-yl)-1-methyl-1H-indazol-6-yl]methyl}-2,6-diazaspiro[3.3]heptan-2-carboxylate (68 mg) and dichloromethane (1 mL) was added TFA (1 mL) under ice-bath cooling, and the mixture was stirred for 1 hour. The reaction mixture was concentrated under reduced pressure, and the residue was purified by basic silica gel column chromatography (chloroform/MeOH), thus obtaining 1-{6-[(2,6-diazaspiro[3.3]heptan-2-yl)methyl]-1-methyl-1H-indazol-3-yl}-1,3-diazinane-2,4-dione (68 mg) as a foam-like solid.

### Production Example 65

To an N-methyl-2-pyrrolidone (60 mL) suspension of 2-amino-4-bromo-3-fluoro-5-iodobenzoic acid (30 g) was added trimethyl orthoacetate (32 mL) at room temperature, and under an argon atmosphere, the mixture was stirred at 110°C overnight. After the temperature was returned to room temperature, the reaction mixture was suspended by the addition of MeOH. The insoluble materials were filtered and were dried under reduced pressure at 50°C overnight, thus obtaining methyl 2-acetamide-4-bromo-3-fluoro-5-iodobenzoate (21.5 g) as a solid.

### Production Example 66

Under an argon atmosphere, to a THF (250 mL) suspension of methyl 2-acetamide-4-bromo-3-fluoro-5-iodobenzoate (21.5 g) was added lithium bis(trimethylsilyl)amide (1 M THF solution, 160 mL) over 20 minutes using a dropping funnel under ice-bath cooling. Then, the mixture was stirred at 40°C for 1 hour under an argon atmosphere. Under ice-bath cooling, water was added to quench the reaction, and the mixture was diluted with ethyl acetate and water. The organic layer and the aqueous layer were separated by a separation operation, and the organic layer was extracted twice with water. Hydrochloric acid (1 M aqueous solution, 200 mL) was slowly added to the collected aqueous layer under ice-bath cooling to precipitate a solid. The insoluble materials were filtered, were washed with water and MeOH and were then dried under reduced pressure at 50°C overnight, thus obtaining 7-bromo-8-fluoro-6-iodoquinoline-2,4-diol (17.7 g) as a solid.

### Production Example 67

Under a nitrogen atmosphere, DIPEA (30 mL) was added dropwise to a phosphoryl chloride (95.2 mL) suspension of 7-bromo-8-fluoro-6-iodoquinoline-2,4-diol (21.24 g) under ice-bath cooling over 5 minutes, and the mixture was stirred at 110°C for 2 hours. After the reaction mixture was allowed to cool to room temperature, MeCN (100 mL) was added, and the mixture was stirred under ice-bath cooling for 30 minutes. The insoluble materials were filtered using MeCN (100 mL). The resulting solid was suspended in MeCN (50 mL) and ice water (200 mL), and the suspension was stirred for 30 minutes. The insoluble materials were filtered with water/MeCN (5/1, 250 mL) to give 7-bromo-2,4-dichloro-8-fluoro-6-iodoquinoline (18.95 g) as a solid.

### Production Example 68

Under an argon atmosphere, to an N-methyl-2-pyrrolidone (45 mL) suspension of 7-bromo-2,4-dichloro-8-fluoro-6-iodoquinoline (4.4 g) was added DABCO (1.3 g), and the mixture was stirred at 40°C for 2 hours. Ethanethiol (850 µL) was added, and the mixture was stirred at 60°C for 4 hours. After the mixture was cooled to room temperature, water was added (200 mL), and the mixture was stirred at room temperature for 30 minutes. The insoluble materials were filtered and were dried under reduced pressure, thus obtaining 7-bromo-4-chloro-2-(ethylsulfanyl)-8-fluoro-6-iodoquinoline (4.33 g) as a solid.

### Production Example 70

In DMAc (10 mL), 1-(tert-butoxycarbonyl)-3-hydroxyazetidine (1 g) was dissolved. Under an argon atmosphere, tBuOK (600 mg) was added, and the mixture was stirred at room temperature for 10 minutes to prepare an alkoxide. In DMAc (10 mL), 7-bromo-4-chloro-2-(ethylsulfanyl)-6-iodo-8-[(1S)-1-phenylethoxy]quinoline (2.12 g) was dissolved. Under an argon atmosphere, the prepared alkoxide solution was added dropwise in an ice-salt bath, and the mixture was stirred at the same temperature for 30 minutes. Water was added to the reaction mixture under ice-bath cooling, and the mixture was stirred at the same temperature for 30 minutes. After the insoluble materials were filtered, the resulting solid was purified by silica gel column chromatography (hexane/chloroform/MeOH), thus obtaining tert-butyl 3-({7-bromo-2-(ethylsulfanyl)-6-iodo-8-[(1S)-1-phenylethoxy]quinolin-4-yl}oxy)azetidine-1-carboxylate (2.48 g) as a foam-like solid.

### Production Example 73

MeOH (120 mL) was added to tert-butyl 3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (about 3.5:1 diastereomeric mixture derived from axial chirality, 5.98 g), and the mixture was stirred at 50°C for 1 hour and then at room temperature overnight. Tert-butyl 3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (about 1:1 diastereomeric mixture derived from axial chirality, 1.82 g), which was an insoluble material, was separated by filtration while washing with MeOH. The filtrate was concentrated under reduced pressure, thus obtaining the desired tert-butyl 3-({(7M)-6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (single diastereomer, 3.43 g) as a foam-like solid.

### Production Example 74

Tert-butyl 3-({(7M)-6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (950 mg) was dissolved in dichloromethane (25 mL), and m-chloroperbenzoic acid (about 30% water content, 600 mg) was added under ice-bath cooling. The mixture was stirred at the same temperature for 2 hours. Aqueous sodium thiosulfate solution was added under ice-bath cooling, and the mixture was stirred at room temperature for 30 minutes. Then, the mixture was extracted twice with chloroform. The combined organic layer was washed with saturated aqueous sodium chloride solution, was dried over anhydrous sodium sulfate and was filtered. The filtrate was concentrated, and the residue was purified by silica gel column chromatography (chloroform/MeOH), thus obtaining tert-butyl 3-({(7M)-6-cyclopropyl-2-(ethanesulfonyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (752 mg) as a foam-like solid.

### Production Example 79

Under ice-bath cooling, to a THF (3 mL) solution of N,N-dimethylethylenediamine (90 µL) was added CDI (130 mg), and under nitrogen atmosphere, the mixture was stirred for 1 hour. The reaction mixture was concentrated under reduced pressure. An iPrOH (3 mL) solution of methyl 4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]benzoate (100 mg) was added, and under nitrogen atmosphere, the mixture was stirred at 90°C for 30 minutes. The reaction mixture was concentrated, and the residue was purified by basic silica gel column chromatography (chloroform/MeOH), thus obtaining methyl 4-[({(7M)-6-cyclopropyl-4-[(1-{[2-(dimethylamino)ethyl]carbamoyl }azetidin-3-yl)oxy]-7-(6-fluoro-5-methyl-1H-indazol-4-yl)-2-[(2S)-2-methoxypropoxy]quinolin-8-yl}oxy)methyl]benzoate (89 mg) as a foam-like solid.

### Production Example 88

To a dichloromethane (20 mL) solution of tert-butyl (3S)-4-{[4-(hydroxymethyl)phenyl]methyl}-3-methylpiperazine-1-carboxylate (1730 mg) were added DIPEA (2 mL) and methanesulfonylchloride (650 µL) under ice-bath cooling, and under nitrogen atmosphere, the mixture was stirred under ice-bath cooling for 1 hour and at room temperature overnight. Water was added to the reaction mixture, and the mixture was extracted twice with ethyl acetate. The organic layers were combined and washed with water and saturated aqueous sodium chloride solution, were then dried over anhydrous magnesium sulfate and were filtered. The filtrate was concentrated under reduced pressure, and the resulting residue was purified by silica gel column chromatography (hexane/ethyl acetate), thus obtaining tert-butyl (3 S)-4-{[4-(chloromethyl)phenyl]methyl}-3-methylpiperazine-1-carboxylate (1550 mg) as a solid.

### Production Example 89

To a suspension in DMF (25 mL) and THF (25 mL) of 7-bromo-4-tert-butoxy-2-chloro-8-fluoro-6-iodoquinazoline (5 g) were added 4-hydroxytetrahydropyran (1.45 mL), DABCO (120 mg) and cesium carbonate (7 g) at room temperature, and under nitrogen atmosphere, the mixture was stirred at room temperature overnight. The reaction mixture was diluted by addition of ethyl acetate. The insoluble materials were filtered through celite (registered trademark) pad and were removed by filtration. Saturated aqueous ammonium chloride solution was added to the filtrate, and the mixture was extracted with ethyl acetate. The organic layer was washed with water and saturated aqueous sodium chloride solution, was then dried over anhydrous magnesium sulfate and was filtered. The filtrate was concentrated under reduced pressure, and the resulting residue was purified by silica gel column chromatography (chloroform/ethyl acetate), thus obtaining 7-bromo-4-tert-butoxy-8-fluoro-6-iodo-2-[oxan-4-yl)oxy]quinazoline (3.9 g) as a solid.

### Production Example 97

To a dichloromethane (10 mL) solution of tert-butyl 3-cyanoazetidine-1-carboxylate (330 mg) was added TFA (2 mL) under ice-bath cooling, and under nitrogen atmosphere, the mixture was stirred at room temperature for 1 hour. Then, the reaction mixture was concentrated under reduced pressure to prepare an amine. In another flask, to a MeCN (10 mL) solution of tert-butyl 4-[({(7M)-6-cyclopropyl-7-[6-fluoro-5-methyl-1-(oxan-2-yl)-1H-indazol-4-yl]-4-hydroxy-2-[oxan-4-yl)oxy]quinazolin-8-yl}oxy)methyl]benzoate (300 mg) were added TEA (120 µL) and PyAOP (440 mg) at room temperature, and under nitrogen atmosphere, the mixture was stirred at room temperature for 2 hours. A MeCN (5 mL) solution of the prepared amine and TEA (1.1 mL) was added at room temperature, and under nitrogen atmosphere, the mixture was stirred at room temperature for 2 hours. The reaction mixture was concentrated under reduced pressure, and the residue was purified by silica gel column chromatography (hexane/ethyl acetate), thus obtaining tert-butyl 4-[({(7M)-4-(3-cyanoazetidin-1-yl)-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoate (222 mg) as a solid.

### Production Example 99

To a 1,4-dioxane (200 mL) solution of 3-[(4-methoxyphenyl)methyl]-1,3-diazinane-2,4-dione (5 g) and tert-butyl (3-iodophenoxy)di(methyl)silane (7.85 g) were added copper(I) iodide (4.07 g), N,N'-dimethylethylenediamine (4.59 mL) and tripotassium phosphate (13.59 g) under nitrogen atmosphere, and the mixture was stirred at 120°C for 16 hours. Water was added to the reaction mixture, and the mixture was extracted twice with ethyl acetate. The collected organic layer was dried over anhydrous sodium sulfate, was filtered and was concentrated under reduced pressure. The residue was purified by silica gel column chromatography (petroleum ether/ethyl acetate), thus obtaining 1-(3-{[tert-butyldi(methyl)silyl]oxy}phenyl)-3-[(4-methoxyphenyl)methyl]-1,3-diazinane-2,4-dione (8 g) as an oil.

### Production Example 100

To a THF (90 mL) solution of 1-(3-{[tert-butyldi(methyl)silyl]oxy}phenyl)-3-[(4-methoxyphenyl)methyl]-1,3-diazinane-2,4-dione (8.9 g) was added TBAF (1 M THF solution, 30.3 mL). After nitrogen gas substitution operations were performed three times, the mixture was stirred under a nitrogen atmosphere at room temperature for 16 hours. The reaction mixture was diluted with water and extracted twice with ethyl acetate. The collected organic layer was washed with saturated aqueous sodium chloride solution four times, was dried over anhydrous sodium sulfate and was filtered. The filtrate was concentrated under reduced pressure. Then, the residue was suspended in ethyl acetate (30 mL) and stirred. The insoluble materials were filtered and were dried under reduced pressure. The resulting solid was suspended in MeCN/water (2/1, 200 mL) and was lyophilized, thus obtaining 1-(3-hydroxyphenyl)-3-[(4-methoxyphenyl)methyl]-1,3-diazinane-2,4-dione (6.2 g) as a solid.

### Production Example 101

To a DMF (10 mL) solution of 1-(3-hydroxyphenyl)-3-[(4-methoxyphenyl)methyl]-1,3-diazinane-2,4-dione (1 g) were added potassium iodide (560 mg) and potassium carbonate (635 mg) under nitrogen atmosphere at room temperature. After the reaction mixture was heated to 115°C, 2-bromo-1,1-dimethoxyethane (468 µL) was added, and the mixture was stirred at the same temperature for 18 hours. The reaction mixture was filtered using MeCN and the filtrate was concentrated under reduced pressure. The residue was purified by reverse phase chromatography (MeCN/0.05% aqueous TFA solution), thus obtaining 1-[3-(2,2-dimethoxyethoxy)phenyl]-3-[(4-methoxyphenyl)methyl]-1,3-diazinane-2,4-dione (940 mg) as a solid.

### Production Example 102

To an acetone (9.2 mL) solution of 1-[3-(2,2-dimethoxyethoxy)phenyl]-3-[(4-methoxyphenyl)methyl]-1,3-diazinane-2,4-dione (920 mg) was added hydrochloric acid (2 M aqueous solution, 5.55 mL), and the mixture was stirred at 50°C for 5 hours. The reaction mixture was concentrated under reduced pressure and was lyophilized, thus obtaining (3-{3-[(4-methoxyphenyl)methyl]-2,4-dioxo-1,3-diazinan-1-yl}phenoxy)acetaldehyde (889 mg) as a solid.

### Production Example 104

A mixture of tert-butyl 9-[2-(3-{3-[(4-methoxyphenyl)methyl]-2,4-dioxo-1,3-diazinan-1-yl}phenoxy)ethyl]-3,9-diazaspiro[5.5]undecane-3-carboxylate (180 mg) and trifluoromethanesulfonic acid (180 µL) was purged with nitrogen gas three times, and under nitrogen atmosphere, the mixture was stirred at 65°C for 2 hours. The reaction mixture was concentrated under reduced pressure, and the resulting crude product, which was combined with a crude product obtained by the reaction performed in the same manner (using 270 mg of raw materials), was dissolved in MeCN (5 mL), was purified by reverse phase chromatography (MeCN/0.05% aqueous TFA solution), thus obtaining 1-{3-[2-(3,9-diazaspiro[5.5]undecan-3-yl)ethoxy]phenyl}-1,3-diazinane-2,4-dione n trifluoromethanesulfonate (350 mg) as a solid.

In the same manner as in the production methods of the Production Examples described above, the compounds shown in the tables presented below were produced. In addition, the production method, the structure and the physiochemical data of the compound of each Production Example are shown in the tables presented below.

### Example 2

HATU (38 mg) was added to a mixture of 4-[({(7M)-4-(3-cyanoazetidin-1-yl]-6-cyclopropyl-7-(6-fluoro-5-methyl-1H-indazol-4-yl)-2-[(2S)-2-methoxypropoxy]quinazolin-8-yl}oxy)methyl]benzoic acid (57 mg), 3-(3-methyl-5-{[(3S)-3-methylpiperazin-1-yl]methyl}-2-oxo-2,3-dihydro-1H-benzimidazol-1-yl)piperidine-2,6-dione n hydrochloride (40 mg), DIPEA (100 µL) and DMF (2 mL), and the mixture was stirred at room temperature overnight. Ethyl acetate, water and saturated aqueous sodium chloride solution were added, and the mixture was extracted with ethyl acetate. The resulting organic layer was dried over anhydrous sodium sulfate and was separated by filtration. Then, the filtrate was concentrated under reduced pressure. The residue was purified by ODS column chromatography (MeCN/0.1% aqueous formic acid solution). Saturated aqueous sodium hydrogen carbonate solution was added to the desired fraction, and the mixture was extracted twice with chloroform/iPrOH (4/1). The organic layer was dried over anhydrous sodium sulfate, was filtered and was concentrated under reduced pressure. The residue was washed with hexane and was dried under reduced pressure, thus obtaining 1-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-3-carbonitrile (44 mg) as a solid.

### Example 3

TFA (0.4 mL) was added to a mixture of tert-butyl [(3 S,4R)-1- { 6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-[6-fluoro-5-methyl-1-(oxan-2-yl)-1H-indazol-4-yl]-2-[(2S)-2-methoxypropoxy]quinazolin-4-yl}-3-fluoropiperidin-4-yl]carbamate (58 mg) and dichloromethane (1.2 mL), and the mixture was stirred at room temperature overnight. The reaction mixture was concentrated under reduced pressure, and the residue was purified by ODS column chromatography (MeCN/0.1% aqueous formic acid solution). Saturated aqueous sodium hydrogen carbonate solution was added to the desired fraction. The mixture was extracted twice with chloroform/iPrOH (4/1). The organic layer was dried over anhydrous sodium sulfate, was filtered and was concentrated under reduced pressure. The resulting solid was washed with hexane and was concentrated under reduced pressure, thus obtaining 3-(5-{[(3 S)-4-{4-[({4-[(3 S,4R)-4-amino-3-fluoropiperidin-1-yl]-6-cyclopropyl-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione (29.1 mg) as a solid.

### Example 5

HATU (39 mg) was added to a mixture of 4-[({(7M)-4-[(2S)-2-carbamoylazetidin-1-yl]-6-cyclopropyl-7-(6-fluoro-5-methyl-1H-indazol-4-yl)-2-[(2S)-2-methoxypropoxy]quinazolin-8-yl}oxy)methyl]benzoic acid (45 mg), 1-(1-methyl-6-{[(3S)-3-methylpiperazin-1-yl]methyl}-1H-indazol-3-yl)-1,3-diazinane-2,4-dione n hydrochloride (34 mg), DIPEA (82 µL) and DMF (3 mL), and the mixture was stirred at room temperature overnight. Ethyl acetate, water and saturated aqueous sodium chloride solution were added, and the aqueous layer was separated. The aqueous layer was extracted with ethyl acetate, and the combined organic layer was dried over anhydrous sodium sulfate and was separated by filtration. Then, the filtrate was concentrated under reduced pressure. The residue was purified by ODS column chromatography (MeCN/0.1% aqueous formic acid solution), and saturated aqueous sodium hydrogen carbonate solution was added to the desired fraction. The mixture was extracted with chloroform/iPrOH (4/1) three times. The organic layer was dried over anhydrous sodium sulfate, was filtered and was concentrated under reduced pressure. The residue was dissolved in MeCN/water and lyophilized, thus obtaining (2S)-1-{(7M)-6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl]phenyl}methoxy)-7-(6-fluoro-5-methyl-1H-indazol-4-yl)-2-[(2S)-2-methoxypropoxy]quinazolin-4-yl}azetidine-2-carboxyamide (20 mg) as a solid.

### Example 20

To a THF (5 mL) solution of 1-(6-{[4-({4-[({(7M)-6-cyclopropyl-7-[6-fluoro-5-methyl-1H-indazol-4-yl]-4-hydroxy-2-[(2S)-2-methoxypropoxy]quinazolin-8-yl}oxy)methyl]phenyl}methyl)-3-oxopiperazin-1-yl]methyl}-1-methyl-1H-indazol-3-yl)-1,3-diazinane-2,4-dione (45 mg) were added PyBOP (63 mg) and cesium carbonate (40 mg) at room temperature, and under nitrogen atmosphere, the mixture was stirred at room temperature for 1 hour. At room temperature, (S)-azetidine-2-carboxamide (25 mg) and DIPEA (90 µL) were added, and under nitrogen atmosphere, the mixture was stirred at room temperature for 2 hours. Water was added to the reaction mixture, and the mixture was extracted twice with ethyl acetate. The organic layer was washed with saturated aqueous sodium chloride solution, was dried over anhydrous magnesium sulfate and was filtered. The filtrate was concentrated under reduced pressure. The residue was purified by silica gel column chromatography (chloroform/MeOH), thus obtaining (2S)-1-{(7M)-6-cyclopropyl-8-({4-[(4-{[3-(2,4-dioxo-1,3-diazinan-1-yl)-1-methyl-1H-indazol-6-yl]methyl}-2-oxopiperazin-1-yl)methyl]phenyl}methoxy)-7-(6-fluoro-5-methyl-1H-indazol-4-yl)-2-[(2S)-2-methoxypropoxy]quinazolin-4-yl}azetidine-2-carboxyamide (31 mg) as a solid.

In the same manner as in the production methods of the Examples described above, the Example compounds shown in the tables presented below were produced. In addition, the production method and the physiochemical data of the compound of each Example are shown in tables presented below.

In the tables presented below, the following abbreviations are sometimes used.

PEx: Production Example No., Ex: Example No., PSyn: Production Example No. produced in the same method, Syn: Example No. produced in the same method (for example, Syn 1 represents that it was produced by the same method as for Example 1), Str: chemical structural formula (a compound with "#" in the chemical structural formula represents that the compound is a diastereomeric mixture having an axial chirality of 3:1 to 5:1, a compound with "##" in the chemical structural formula represents that the compound is single structure with regard to the axial chirality but has an undetermined configuration, and a compound with "###" in the chemical structural formula represents that the compound is a diastereomeric mixture having an axial chirality of 3:1 to 5:1 but has an undetermined configuration), n HCl: n hydrochloride (the compound with Production Example No. shows that the compound is monohydrochloride to trihydrochloride), n TfOH: n trifluoromethanesulfonic acid (the compound with Production Example No. shows that the compound is monotrifluoromethanesulfonate to tritrifluoromethanesulfonate), DAT: physiochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H-NMR (500 MHz) in DMSO-d₆ at 27°C, NMR (90°C): δ value (ppm) of peak in ¹H-NMR (500 MHz) in DMSO-d₆ at 90°C, s: singlet (spectrum), d: doublet (spectrum), dd: double doublet (spectrum), ddd: double double doublet (spectrum), t: triplet (spectrum), dt: double triplet (spectrum), q: quartet (spectrum), m: multiplet (spectrum), br: broad (spectrum).

**[Table 3-1]**

| PEx | Str |
|---|---|
| 1 | 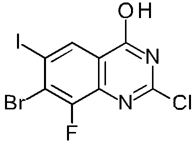 |
| 2 | 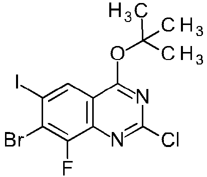 |
| 3 | 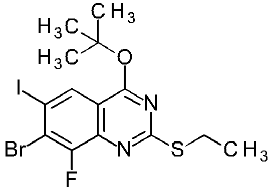 |
| 4 | 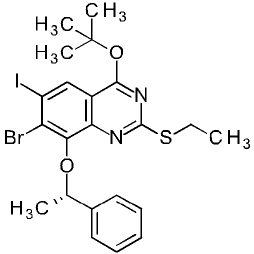 |
| 5 | 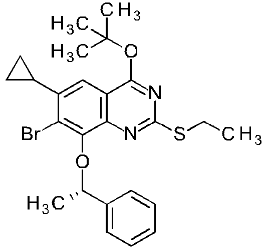 |

**[Table 3-2]**

| PEx | Str |
|---|---|
| 6 | 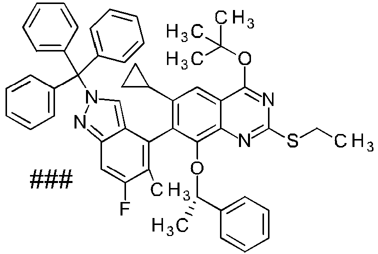 |
| 7-1 | 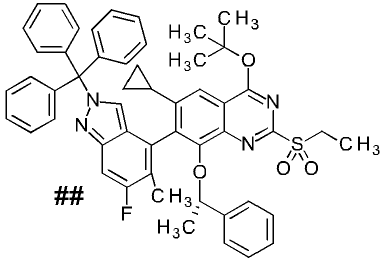 |
| 7-2 | 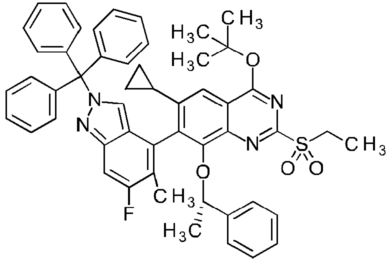 |
| 8 | 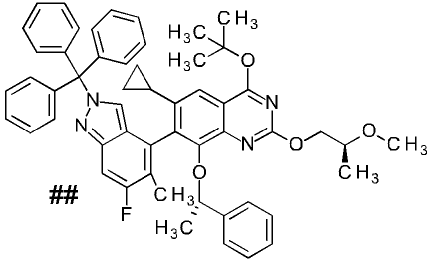 |

**[Table 3-3]**

| PEx | Str |
|---|---|
| 9 | 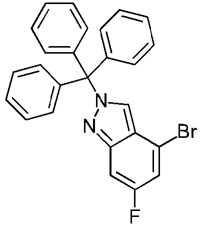 |
| 10 | 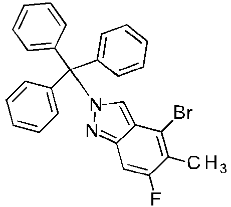 |
| 11 | 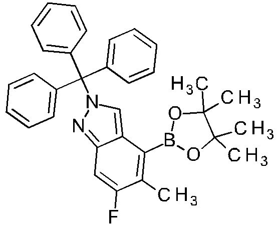 |
| 12 | 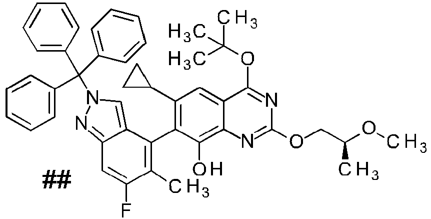 |

**[Table 3-4]**

| PEx | Str |
|---|---|
| 13 | 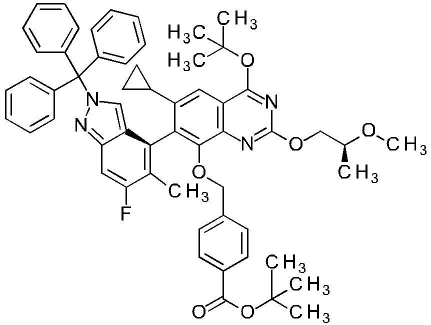 |
| 14 | 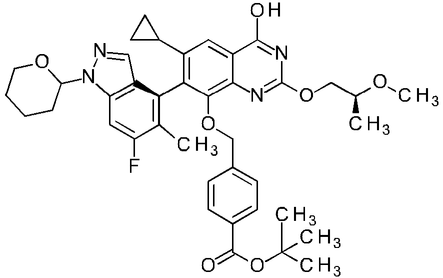 |
| 15 | 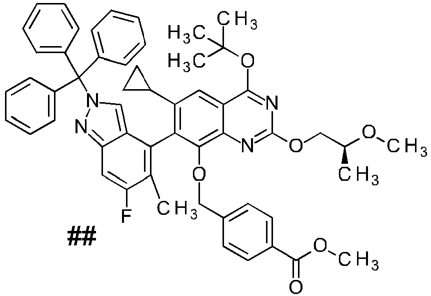 |
| 16 | 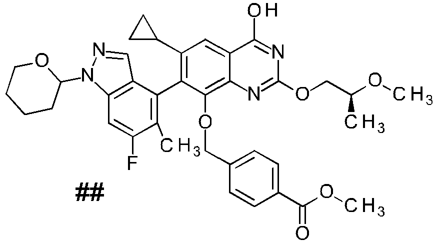 |

**[Table 3-5]**

| PEx | Str |
|---|---|
| 17 | 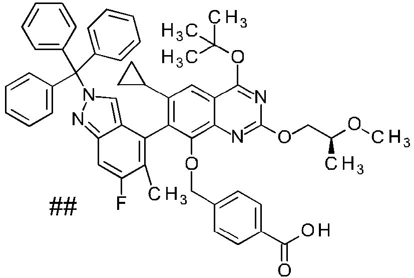 |
| 18 | 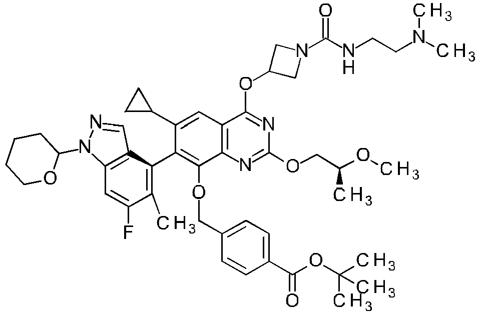 |
| 19 | 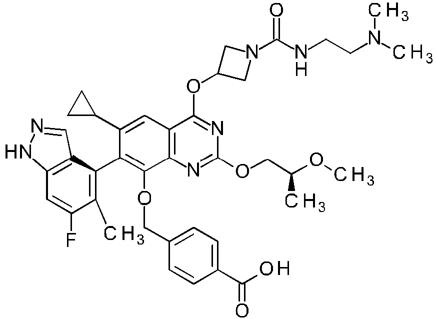 |
| 20 | 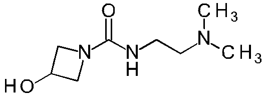 |

**[Table 3-6]**

| PEx | Str |
|---|---|
| 21 | 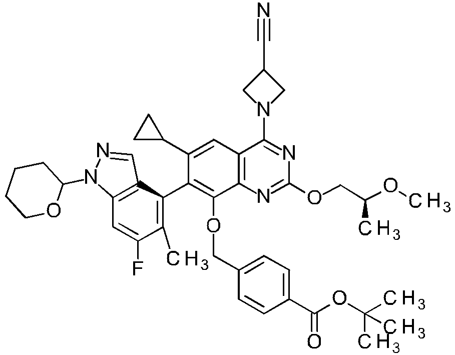 |
| 22 | 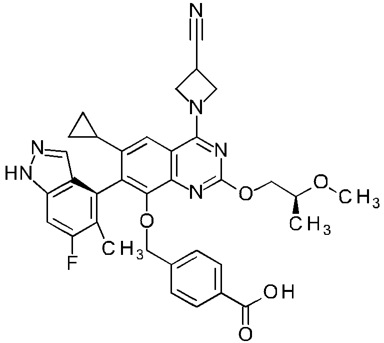 |
| 23 | 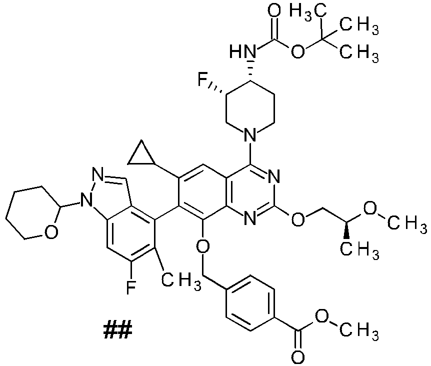 |

**[Table 3-7]**

| PEx | Str |
|---|---|
| 24 | 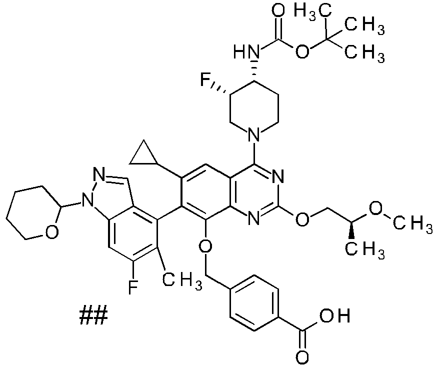 |
| 25 | 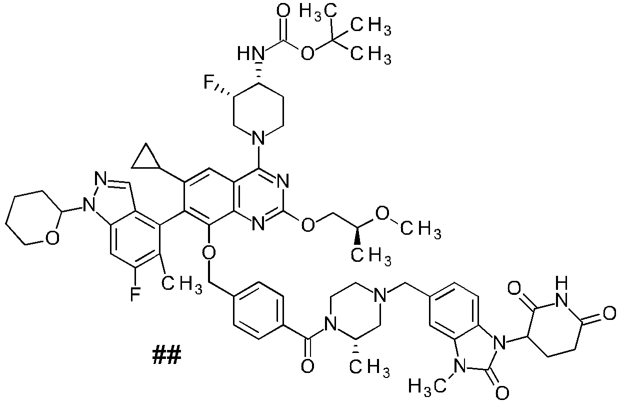 |
| 26 | 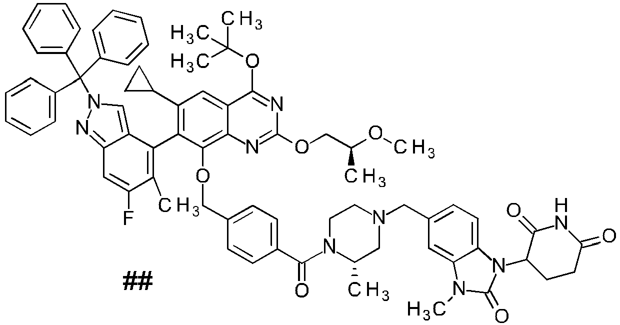 |

**[Table 3-8]**

| PEx | Str |
|---|---|
| 27 | 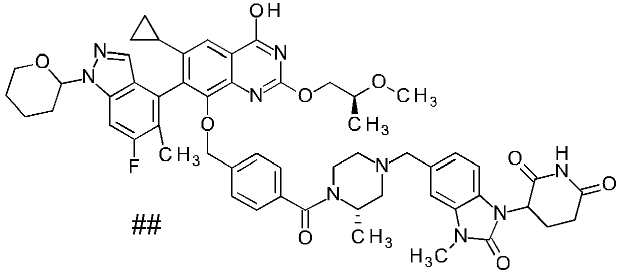 |
| 28 | 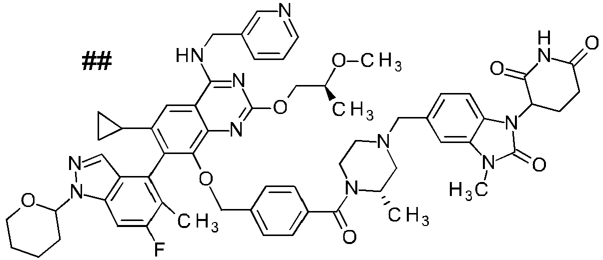 |
| 29 | 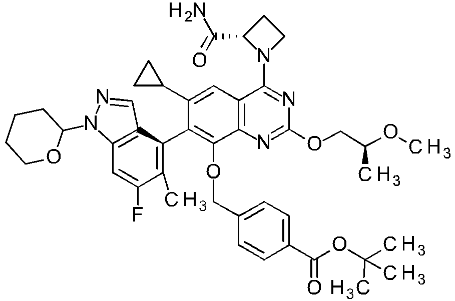 |
| 30 | 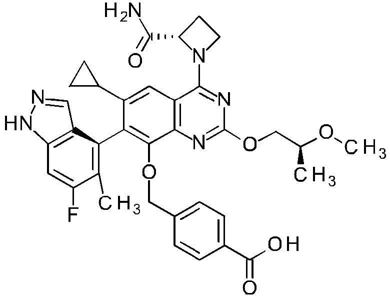 |

**[Table 3-9]**

| PEx | Str |
|---|---|
| 31 | 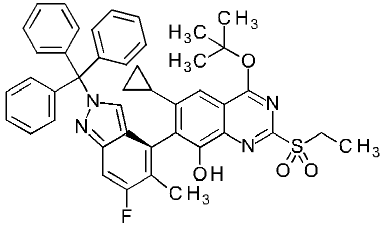 |
| 32 | 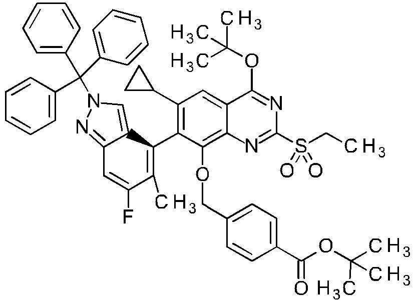 |
| 33 | 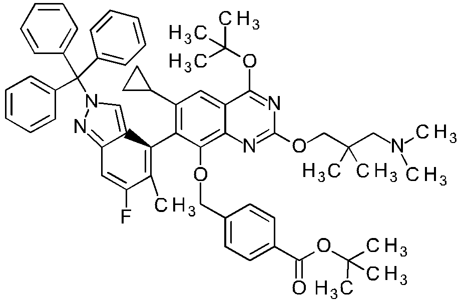 |
| 34 | 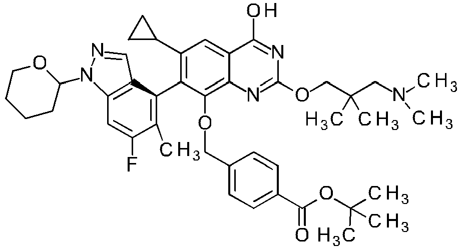 |

**[Table 3-10]**

| PEx | Str |
|---|---|
| 35 | 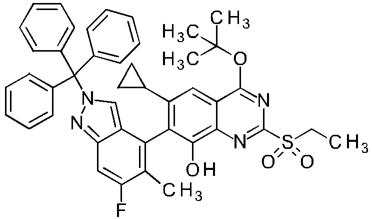 |
| 36 | 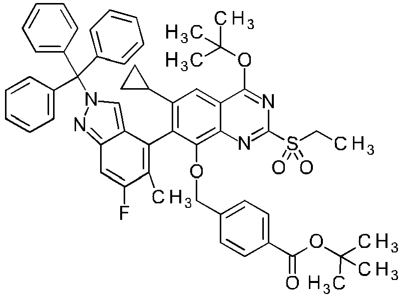 |
| 37 | 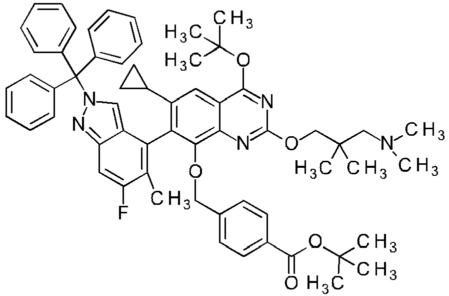 |
| 38 | 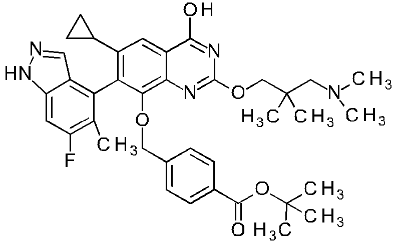 |

**[Table 3-11]**

| PEx | Str |
|---|---|
| 39 | 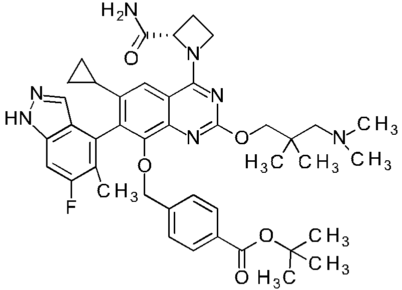 |
| 40 | 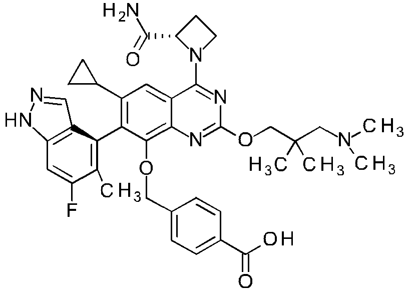 |
| 41 | 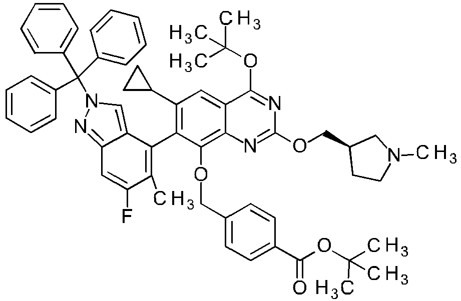 |
| 42 | 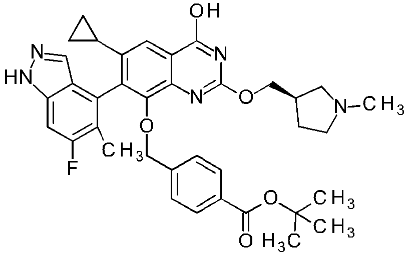 |

**[Table 3-12]**

| PEx | Str |
|---|---|
| 43 | 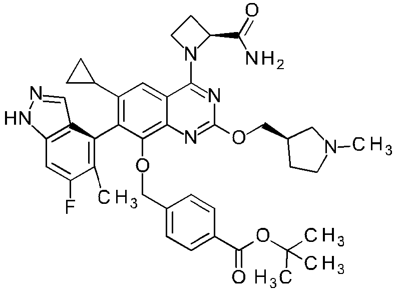 |
| 44 | 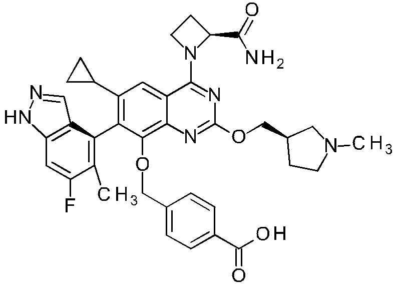 |
| 45 | 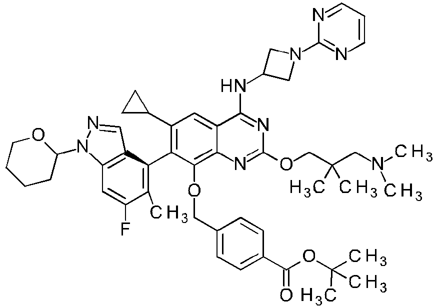 |
| 46 | 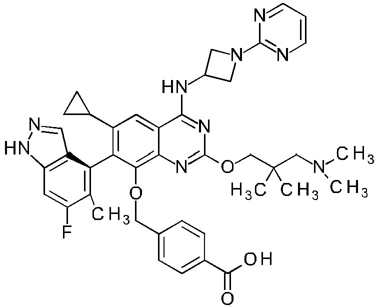 |

**[Table 3-13]**

| PEx | Str |
|---|---|
| 47 | 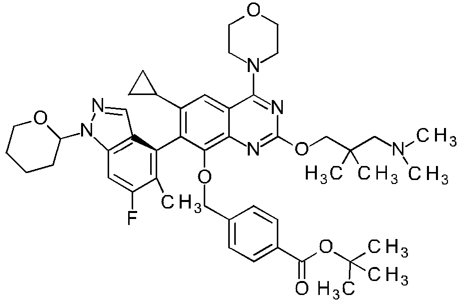 |
| 48 | 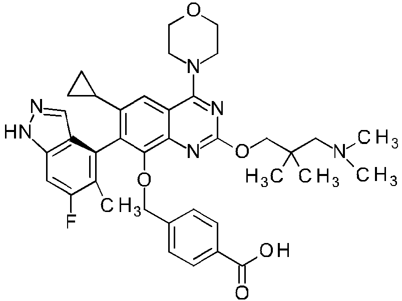 |
| 49 | 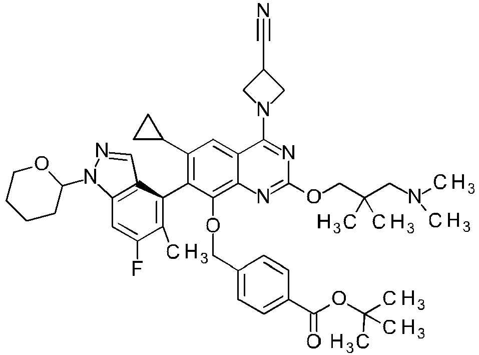 |

**[Table 3-14]**

| PEx | Str |
|---|---|
| 50 | 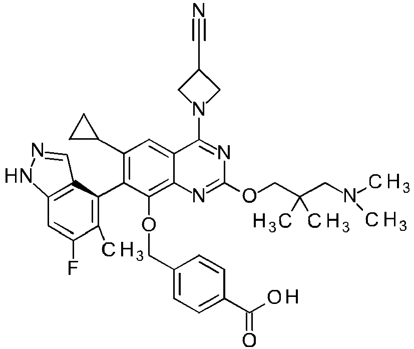 |
| 51 | 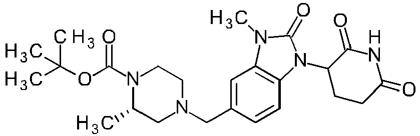 |
| 52 | 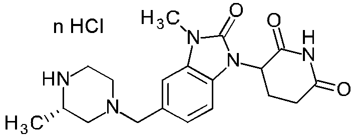 |
| 53 | 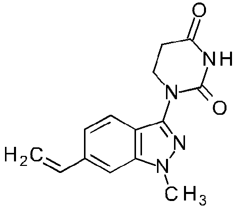 |
| 54 | 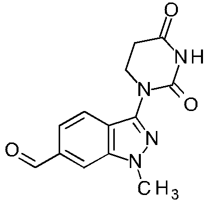 |

**[Table 3-15]**

| PEx | Str |
|---|---|
| 55 | 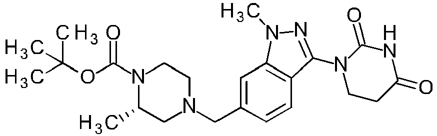 |
| 56 | 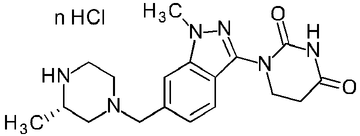 |
| 57 | 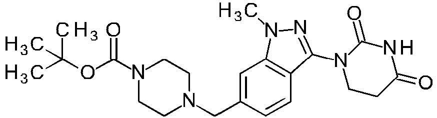 |
| 58 | 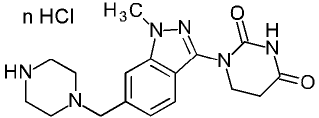 |
| 59 | 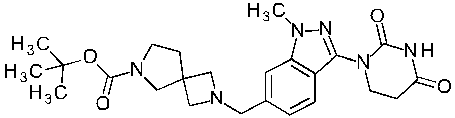 |
| 60 | 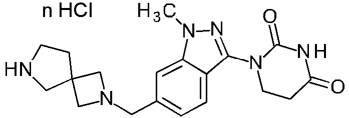 |
| 61 | 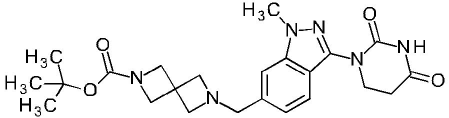 |
| 62 | 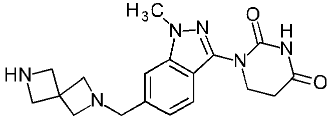 |

**[Table 3-16]**

| PEx | Str |
|---|---|
| 63 | 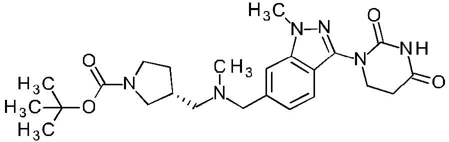 |
| 64 | 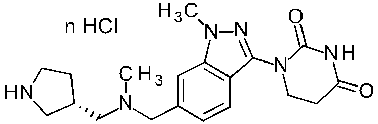 |
| 65 | 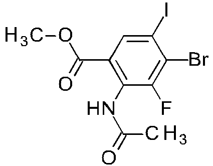 |
| 66 | 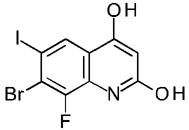 |
| 67 | 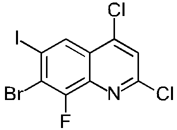 |
| 68 | 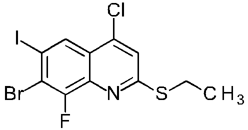 |
| 69 | 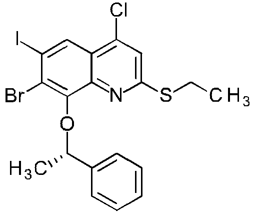 |

**[Table 3-17]**

| PEx | Str |
|---|---|
| 70 | 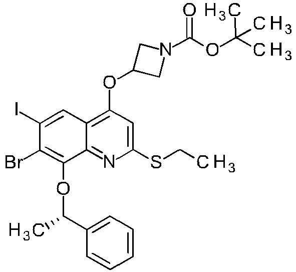 |
| 71 | 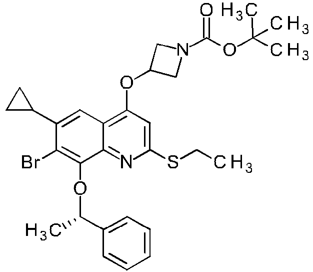 |
| 72 | 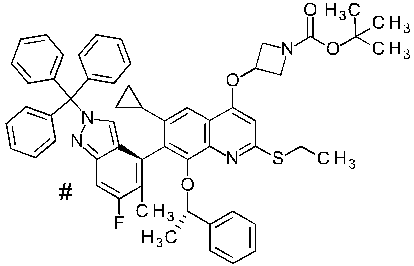 |
| 73 | 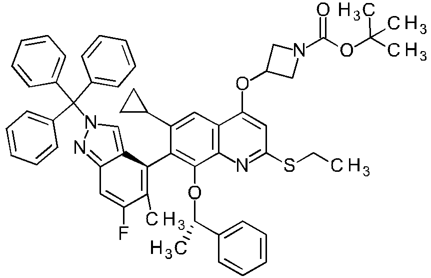 |

**[Table 3-18]**

| PEx | Str |
|---|---|
| 74 | 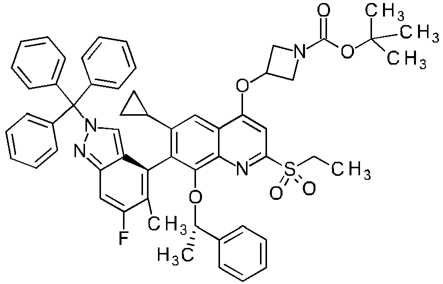 |
| 75 | 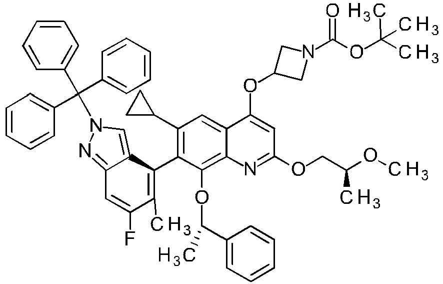 |
| 76 | 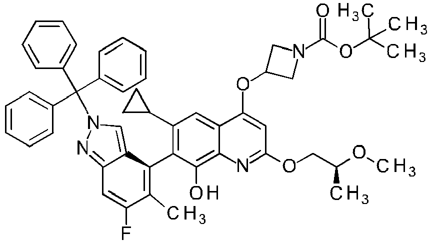 |
| 77 | 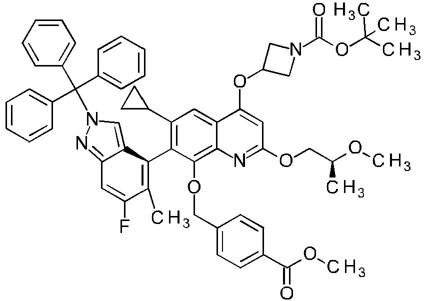 |

**[Table 3-19]**

| PEx | Str |
|---|---|
| 78 | 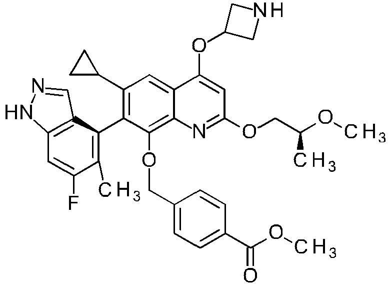 |
| 79 | 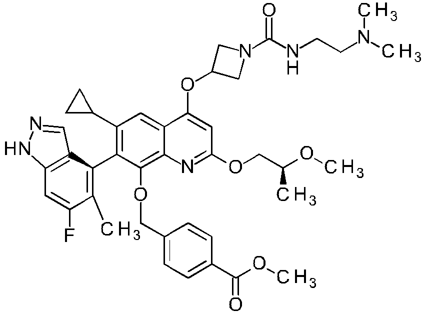 |
| 80 | 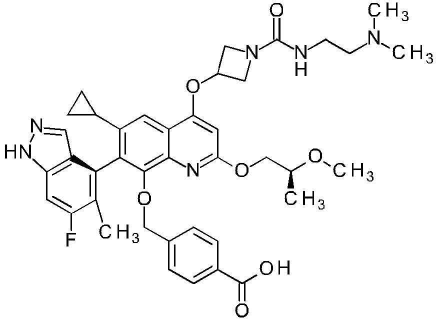 |
| 81 | 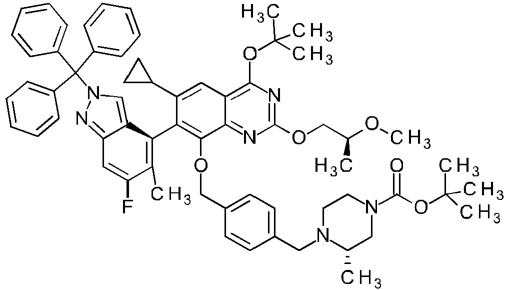 |

**[Table 3-20]**

| PEx | Str |
|---|---|
| 82 | 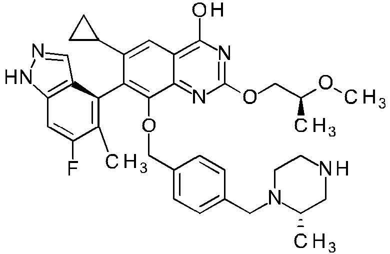 |
| 83 | 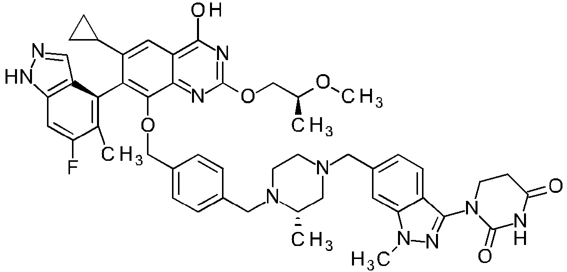 |
| 84 | 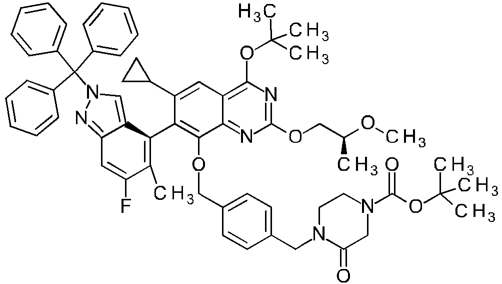 |
| 85 | 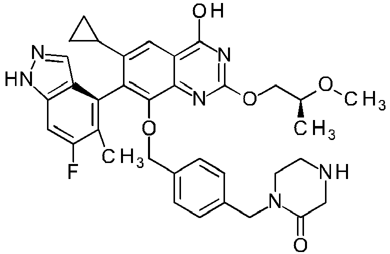 |

**[Table 3-21]**

| PEx | Str |
|---|---|
| 86 | 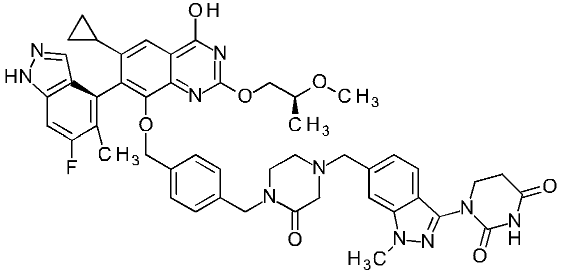 |
| 87 | 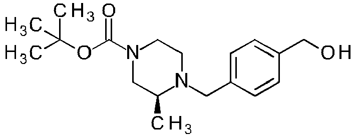 |
| 88 | 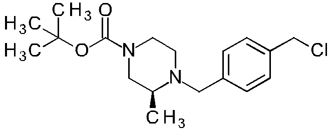 |

**[Table 3-22]**

| PEx | Str |
|---|---|
| 89 | 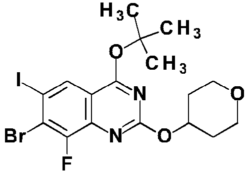 |
| 90 | 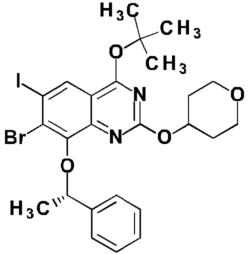 |
| 91 | 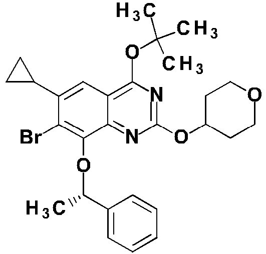 |
| 92 | 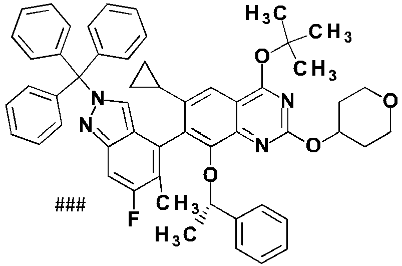 |
| 93 | 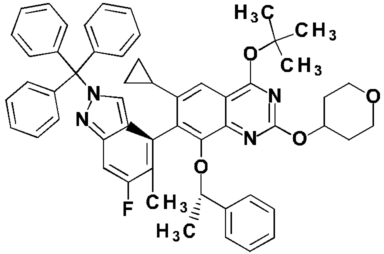 |

**[Table 3-23]**

| PEx | Str |
|---|---|
| 94 | 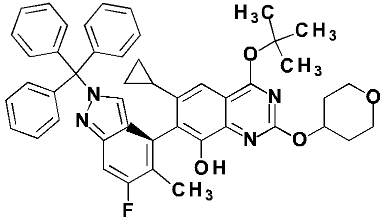 |
| 95 | 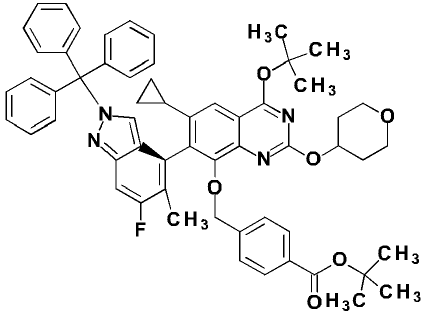 |
| 96 | 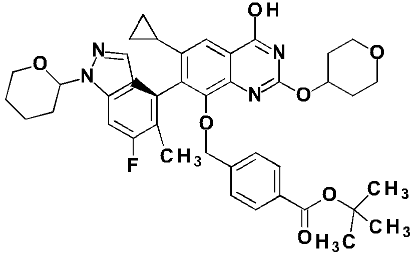 |
| 97 | 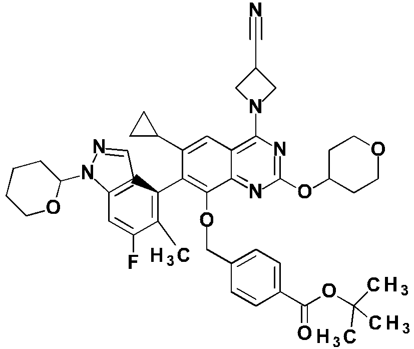 |

**[Table 3-24]**

| PEx | Str |
|---|---|
| 98 | 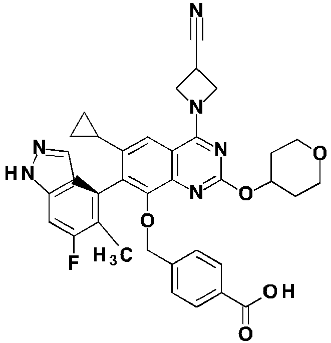 |
| 99 | 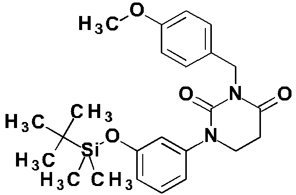 |
| 100 | 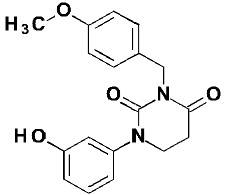 |
| 101 | 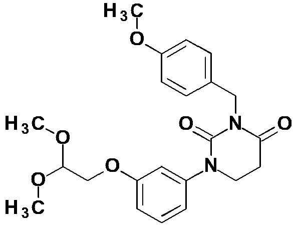 |
| 102 | 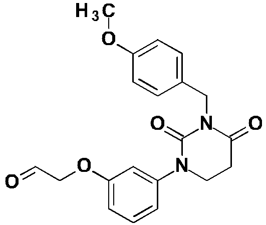 |

**[Table 3-25]**

| PEx | Str |
|---|---|
| 103 | 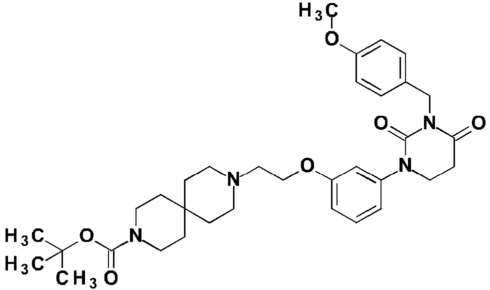 |
| 104 | 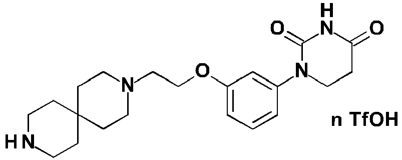 |

**[Table 4-1]**

| PEx | PSyn | DAT |
|---|---|---|
| 1 | 1 | ESI+: 402.9, 404.9 |
| 2 | 2 | ESI+: 480.9, 482.8 [M+Na]+ |
| 3 | 3 | ESI+: 509.0 [M+Na]+ |
| 4 | 4 | ESI+: 587.2 |
| 5 | 5 | ESI+: 503.4 |
| 6 | 6 | ESI+: 813.4 |
| 7-1 | 7 | ESI+: 867.5 [M+Na]+ |
| 7-2 | 7 | ESI+: 867.5 [M+Na]+ |
| 8 | 8 | ESI+: 841.6 |
| 9 | 9 | NMR: 7.08-7.15 (m, 6H), 7.36-7.44 (m, 10H), 7.49-7.54 (m, 1H), 7.89 (d, 1H) |
| 10 | 10 | NMR: 2.34 (d, 3H), 7.07-7.13 (m, 6H), 7.36-7.43 (m, 9H), 7.51 (d, 1H), 7.79 (d, 1H) |
| 11 | 11 | NMR: 1.21 (s, 12H), 2.44 (d, 3H), 7.04-7.11 (m, 6H), 7.34-7.44 (m, 9H), 7.49 (d, 1H), 8.09 (d, 1H) |
| 12 | 12 | ESI+: 737.7 |
| 13 | 13 | ESI+: 949.6 [M+Na]+ |
| 14 | 14 | ESI+: 713.5 |
| 15 | 13 | ESI+: 885.7 |
| 16 | 14 | ESI+: 671.5 |
| 17 | 17 | ESI+: 871.6 |
| 18 | 21 | ESI+: 882.6 |
| 19 | 22 | ESI+: 742.4 |
| 20 | 20 | ESI+: 188.1 |
| 21 | 21 | ESI+: 777.7 |
| 22 | 22 | ESI+: 637.4 |
| 23 | 21 | ESI+: 871.7 |
| 24 | 17 | ESI+: 857.8 |
| 25 | Syn2 | ESI+: 1211.1 |
| 26 | Syn2 | ESI+: 1224.9 |
| 27 | 14 | ESI+: 1010.5 |
| 28 | 21 | ESI-: 1098.8 |
| 29 | 29 | ESI+: 795.7 |
| 30 | 30 | ESI+: 655.5 |
| 31 | 12 | ESI+: 763.6 [M+Na]+ |
| 32 | 13 | ESI+: 953.6 [M+Na]+ |
| 33 | 8 | ESI+: 968.6 |
| 34 | 14 | ESI+: 754.4 |
| 35 | 12 | ESI+: 763.5 [M+Na]+ |

**[Table 4-2]**

| PEx | PSyn | DAT |
|---|---|---|
| 36 | 13 | ESI+: 953.6 [M+Na]+ |
| 37 | 8 | ESI+: 968.6 |
| 38 | 38 | ESI+: 670.7 |
| 39 | 21 | ESI+: 752.4 |
| 40 | 40 | ESI+: 696.5 |
| 41 | 8 | ESI+: 952.8 |
| 42 | 38 | ESI+: 654.6 |
| 43 | 43 | ESI+: 736.7 |
| 44 | 22 | ESI+: 680.5 |
| 45 | 21 | ESI+: 908.6 [M+Na]+ |
| 46 | 22 | ESI+: 746.4 |
| 47 | 21 | ESI+: 823.6 |
| 48 | 22 | ESI+: 683.5 |
| 49 | 21 | ESI+: 818.7 |
| 50 | 22 | ESI+: 678.6 |
| 51 | 51 | ESI+: 472.5 |
| 52 | 52 | ESI+: 372.4 |
| 53 | 53 | ESI+: 271.3 |
| 54 | 54 | ESI+: 273.2 |
| 55 | 51 | ESI+: 457.4 |
| 56 | 52 | ESI+: 357.3 |
| 57 | 51 | ESI+: 443.5 |
| 58 | 52 | ESI+: 343.4 |
| 59 | 51 | ESI+: 469.3 |
| 60 | 52 | ESI+: 369.2 |
| 61 | 51 | ESI+: 455.4 |
| 62 | 62 | ESI+: 355.3 |
| 63 | 51 | ESI+: 471.3 |
| 64 | 52 | ESI+: 371.3 |
| 65 | 65 | ESI+: 416.1 |
| 66 | 66 | ESI+: 383.9 |
| 67 | 67 | ESI+: 419.9, 421.8 |
| 68 | 68 | ESI+: 446.0, 447.9 |
| 69 | 4 | ESI+: 548.0, 550.1 |
| 70 | 70 | ESI+: 707.1, 709.2 [M+Na]+ |
| 71 | 5 | ESI+: 601.5 |

**[Table 4-3]**

| PEx | PSyn | DAT |
|---|---|---|
| 72 | 6 | ESI+: 911.5 |
| 73 | 73 | ESI+: 911.5 |
| 74 | 74 | ESI+: 965.5 [M+Na]+ |
| 75 | 8 | ESI+: 939.6 |
| 76 | 12 | ESI+: 835.7 |
| 77 | 13 | ESI+: 1005.7 [M+Na]+ |
| 78 | 22 | ESI+: 641.4 |
| 79 | 79 | ESI+: 755.7 |
| 80 | 17 | ESI+: 741.5 |
| 81 | 13 | ESI+: 1039.7 |
| 82 | 22 | ESI+: 641.5 |
| 83 | 51 | ESI+: 897.6 |
| 84 | 13 | ESI+: 1061.6 [M+Na]+ |
| 85 | 22 | ESI+: 641.6 |
| 86 | 51 | ESI+: 897.8 |
| 87 | 51 | ESI+: 321.4 |
| 88 | 88 | ESI+: 339.2 |

**[Table 4-4]**

| PEx | PSyn | DAT |
|---|---|---|
| 89 | 89 | ESI+: 527.0 |
| 90 | 4 | ESI+: 629.0 |
| 91 | 5 | ESI+: 543.2 |
| 92 | 6 | ESI+: 853.6 |
| 93 | 73 | ESI+: 853.4 |
| 94 | 12 | ESI+: 749.4 |
| 95 | 13 | ESI+: 939.6 |
| 96 | 14 | ESI+: 725.7 |
| 97 | 97 | ESI+: 789.8 |
| 98 | 22 | ESI+: 649.6 |
| 99 | 99 | ESI+: 441.3 |
| 100 | 100 | ESI+: 327.2 |
| 101 | 101 | ESI+: 415.2 |
| 102 | 102 | ESI+: 369.4 |
| 103 | 51 | ESI+: 607.6 |
| 104 | 104 | ESI+: 387.2 |

**[Table 5-1]**

| Ex | Str |
|---|---|
| 1 | 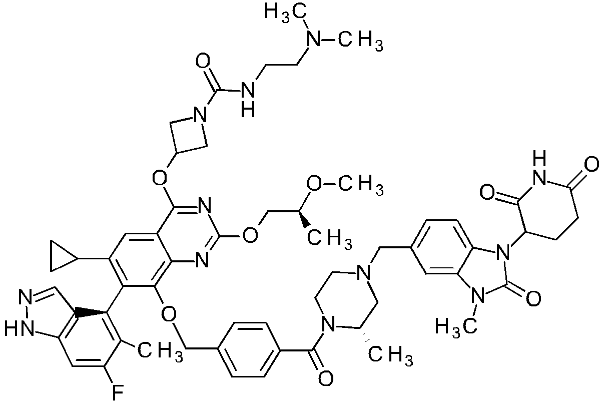 |
| 2 | 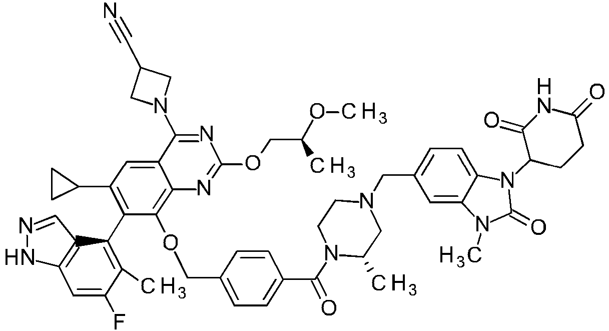 |
| 3 | 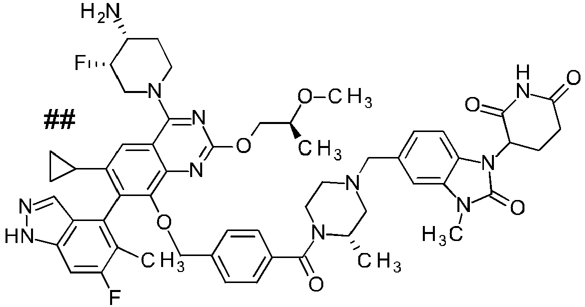 |

**[Table 5-2]**

| Ex | Str |
|---|---|
| 4 | 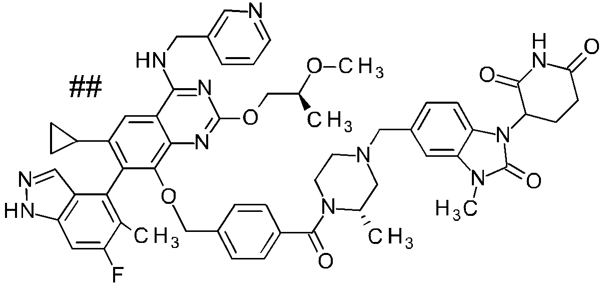 |
| 5 | 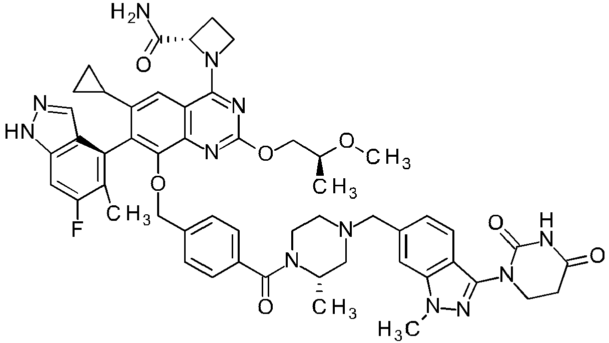 |
| 6 | 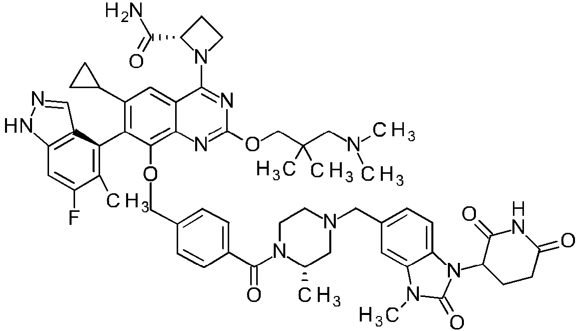 |

**[Table 5-3]**

| Ex | Str |
|---|---|
| 7 | 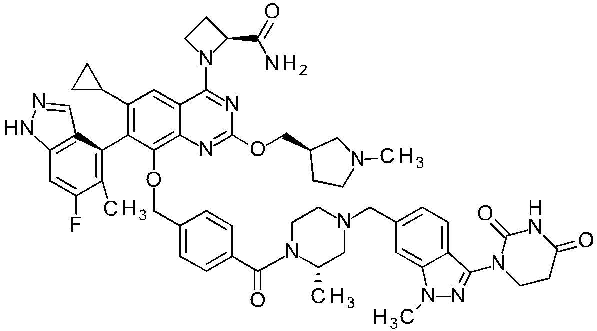 |
| 8 | 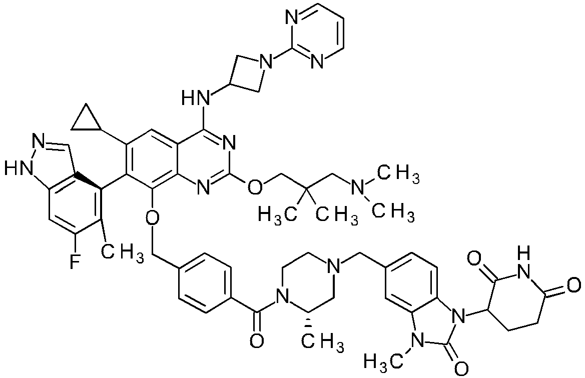 |
| 9 | 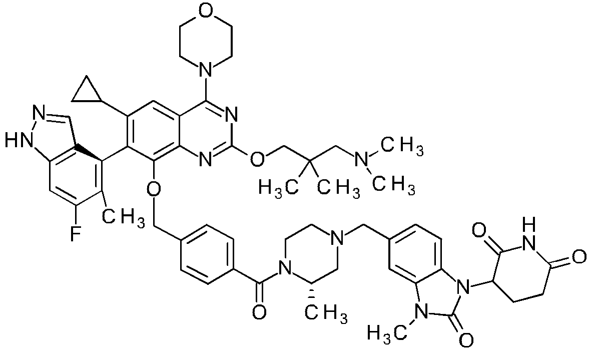 |

**[Table 5-4]**

| Ex | Str |
|---|---|
| 10 | 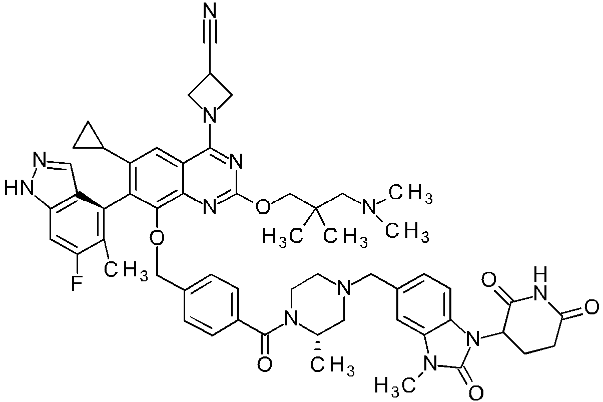 |
| 11 | 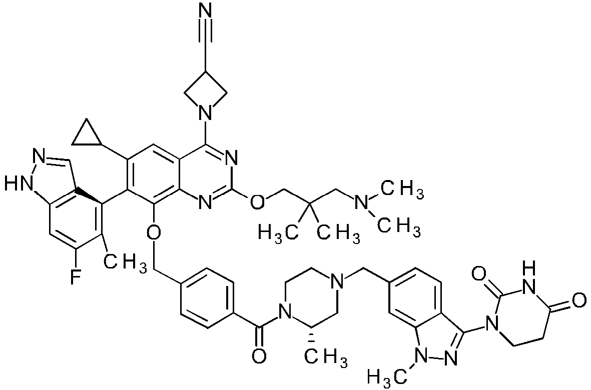 |
| 12 | 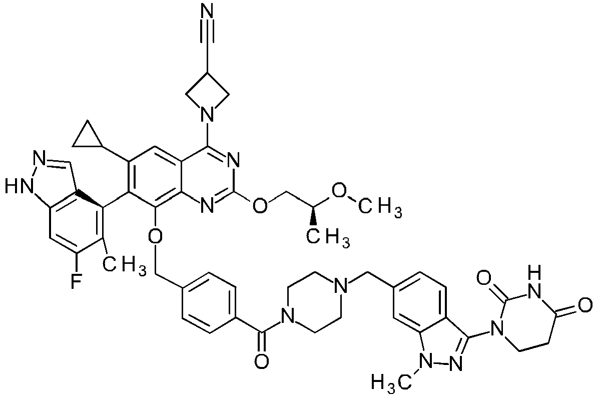 |

**[Table 5-5]**

| Ex | Str |
|---|---|
| 13 | 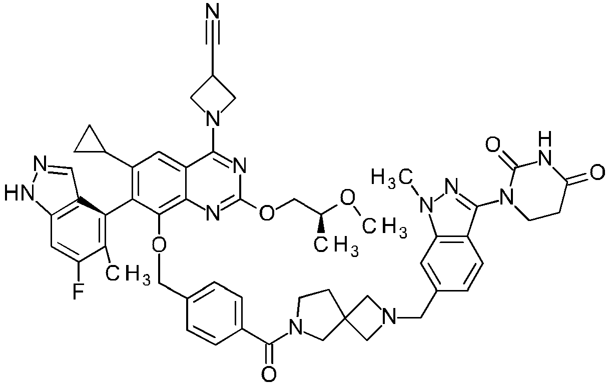 |
| 14 | 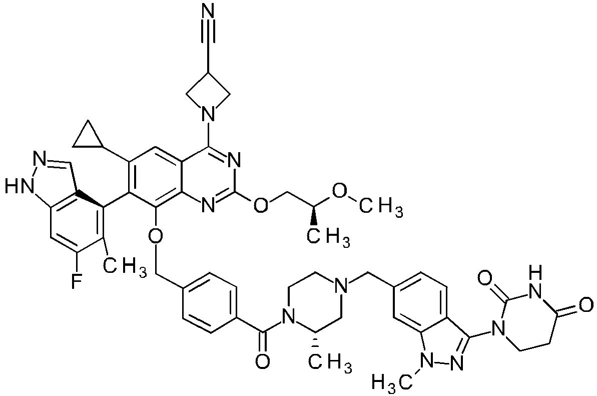 |
| 15 | 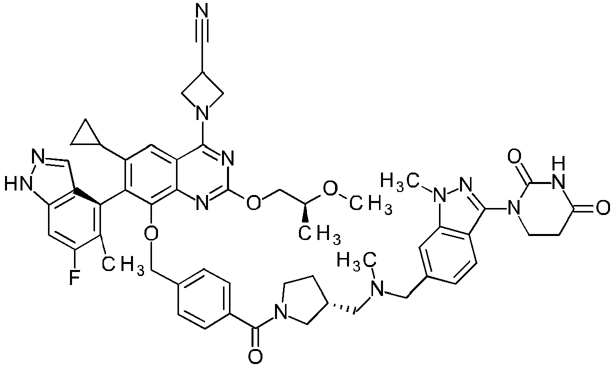 |

**[Table 5-6]**

| Ex | Str |
|---|---|
| 16 | 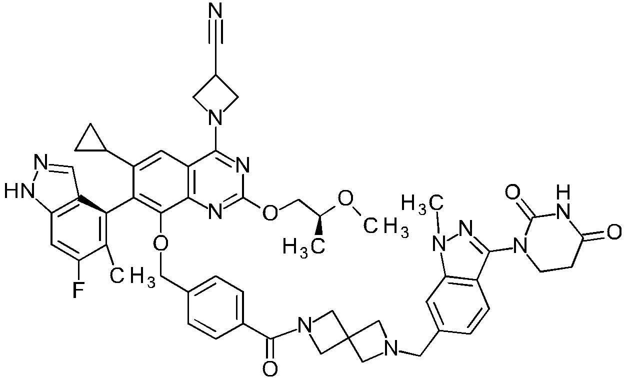 |
| 17 | 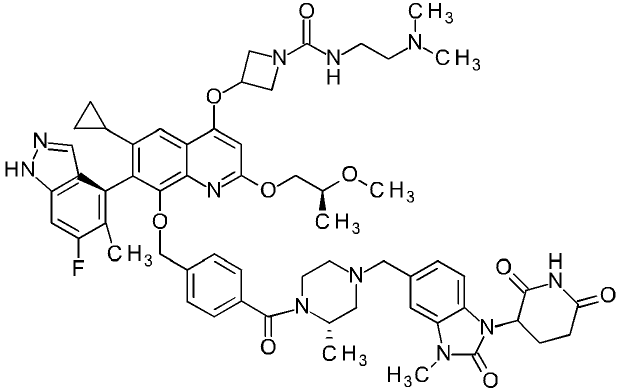 |
| 18 | 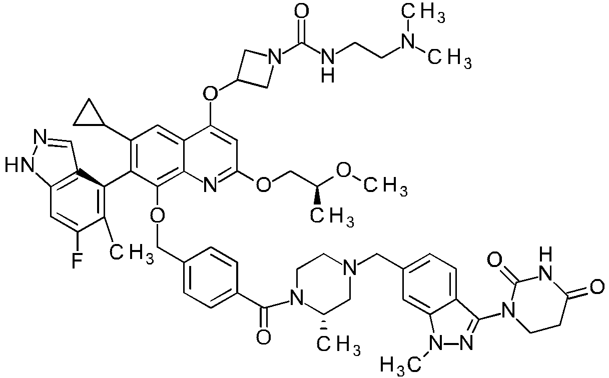 |

**[Table 5-7]**

| Ex | Str |
|---|---|
| 19 | 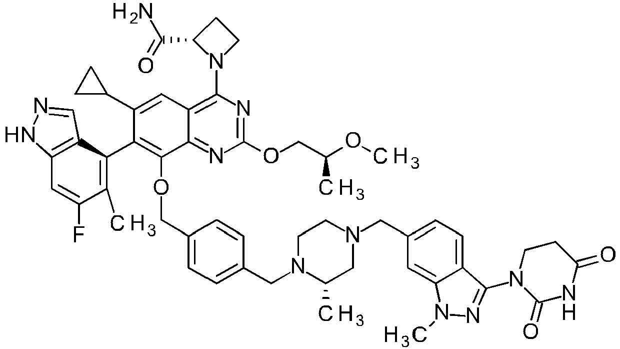 |
| 20 | 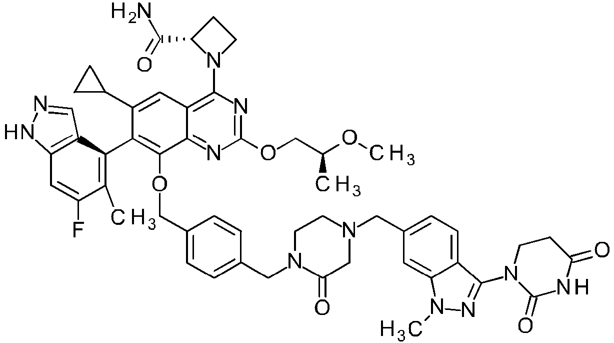 |

**[Table 5-8]**

| Ex | Str |
|---|---|
| 21 | 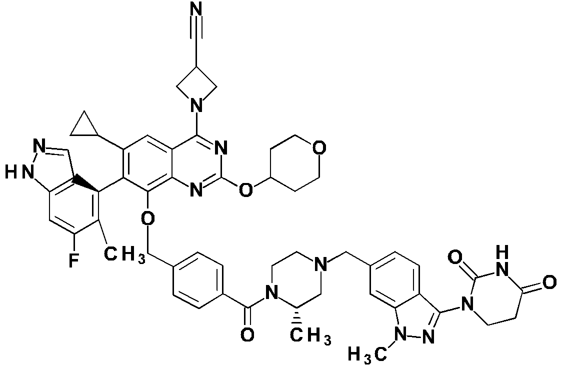 |
| 22 | 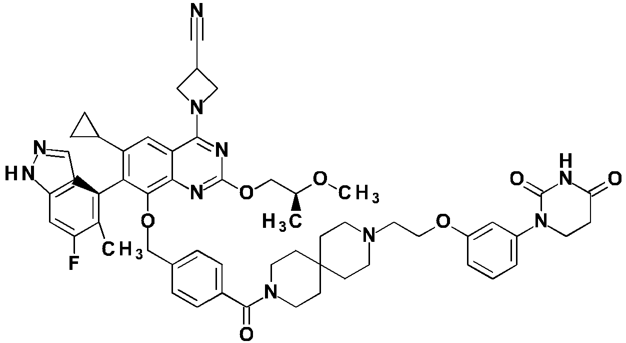 |

**[Table 6-1]**

| Ex | Syn | DAT |
|---|---|---|
| 1 | 2 | ESI+: 1095.6 |
| 2 | 2 | ESI+: 990.8 |
| | | NMR(90°C): 0.48-0.56 (m, 1H), 0.61-0.69 (m, 3H), 1.12 (d, 3H), 1.26 (d, 3H), 1.29-1.42 (m, 1H), 1.93-2.08 (m, 5H), 2.08-2.15 (m, 1H), 2.60-2.74 (m, 3H), 2.76-2.83 (m, 1H), 2.83-2.92 (m, 1H), 3.12-3.21 (m, 1H), 3.28 (s, 3H), 3.32 (s, 3H), 3.46 (d, 1H), 3.51-3.59 (m, 1H), 3.65-3.77 (m, 2H), 3.94-4.01 (m, 1H), 4.17(br, 1H), 4.23-4.34 (m, 2H), 4.68-4.75 (m, 2H), 4.79-4.86 (m, 2H), 4.90 (d, 1H). 5.19-5.30 (m, 2H), 6.82-6.87 (m, 2H), 6.96-7.03 (m, 2H), 7.05-7.10 (m, 3H), 7.16 (s, 1H), 7.27 (d, 1H), 7.41 (s, 1H), 10.72 (s, 1H), 12.76 (br, 1H) |
| 3 | 3 | ESI+: 1048.7 [M+Na]+ |
| 4 | 3 | ESI+: 1017.4 |
| 5 | 5 | ESI+: 993.7 |
| | | NMR(90°C): 0.44-0.55 (m, 1H), 0.55-0.69 (m, 3H), 1.11 (d, 3H), 1.27 (d, 3H), 1.29-1.38 (m, 1H), 1.96 (d, 3H), 2.01-2.10 (m, 1H), 2.13-2.20 (m, 1H), 2.31-2.39 (m, 1H), 2.64-2.78 (m, 4H), 2.78-2.86 (m, 1H), 3.13-3.23 (m, 1H), 3.27 (s, 3H), 3.51-3.60 (m, 1H), 3.64-3.80 (m, 3H), 3.89-3.94 (m, 2H), 3.95 (s, 3H), 4.18 (br, 1H), 4.28 (d, 2H), 4.46-4.53 (m, 1H), 4.63-4.72 (m, 1H), 4.88-4.94 (m, 1H), 5.09-5.15 (m, 1H), 5.17-5.24 (m, 1H), 6.84 (d, 2H), 7.05-7.09 (m, 2H), 7.11 (dd, 1H), 7.20 (s, 1H), 7.26 (d, 1H), 7.41 (s, 1H), 7.44 (s, 1H), 7.60 (dd, 1H), 10.14 (s, 1H), 12.75 (br, 1H) |
| 6 | 2 | ESI+: 1049.7 |
| 7 | 2 | ESI+: 1018.8 |
| 8 | 2 | ESI+: 1099.7 |
| 9 | 2 | ESI+: 1036.7 |
| 10 | 2 | ESI+: 1031.8 |
| 11 | 2 | ESI+: 1016.7 |
| 12 | 2 | ESI+: 961.6 |
| 13 | 2 | ESI+: 987.5 |
| 14 | 2 | ESI+: 975.6 |
| 15 | 2 | ESI+: 1011.6 [M+Na]+ |
| 16 | 2 | ESI+: 973.6 |
| 17 | 2 | ESI+: 1094.7 |
| 18 | 2 | ESI+: 1079.6 |
| 19 | 20 | ESI+: 1001.6 [M+Na]+ |
| 20 | 20 | ESI+: 979.8 |

**[Table 6-2]**

| Ex | Syn | DAT |
|---|---|---|
| 21 | 2 | ESI+: 987.9 |
| 22 | 2 | ESI+: 1027.5 [M+Na]+ |

### Industrial Applicability

The compound or a salt thereof of the present invention is excellent in the degradation-inducing action on a G12V mutant KRAS protein, and is useful as a G12V mutant KRAS inhibitor and can be used as an active ingredient of a pharmaceutical composition, for example, a pharmaceutical composition for treating pancreatic cancer and/or lung cancer.

## Claims

1. A compound of the formula (I) or a salt thereof, 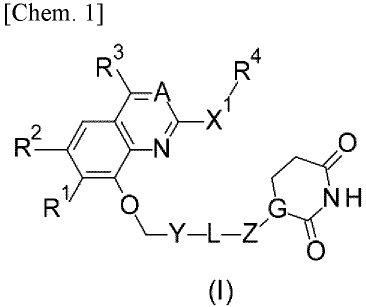 (wherein in the formula,
A is CR^{A} or N,
R^{A} is H or C₁₋₃ alkyl,
X¹ is -CH₂-, -O- or -NR^{X1}-,
R^{X1} is H or optionally substituted C₁₋₃ alkyl,
when X¹ is -NR^{X1}-, R^{X1} and R⁴ present on the same nitrogen atom, together with a nitrogen atom adjacent thereto, optionally form an optionally substituted 4-membered to 6-membered saturated heterocyclic group,
R¹ is naphthyl optionally substituted with OH, or R¹ is the formula (II) or the formula (III) below, 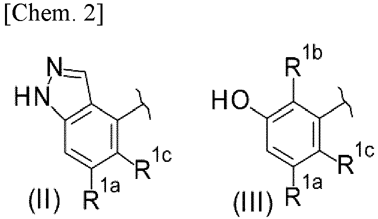
R^{1a} and R^{1b}, which are the same as or different from each other, are H, methyl, F or Cl,
R^{1c} is F, Cl, methyl or ethyl,
R² is H, halogen, C₁₋₃ alkyl, cyclopropyl or vinyl, where the C₁₋₃ alkyl is optionally substituted with a group selected from the group consisting of OH and OCH₃,
R³ is a group selected from the group consisting of the formula (IV), the formula (V), the formula (VI), the formula (VII), the formula (VIII), the formula (IX), the formula (X), the formula (XI), the formula (XII) and the formula (XXVI) below, 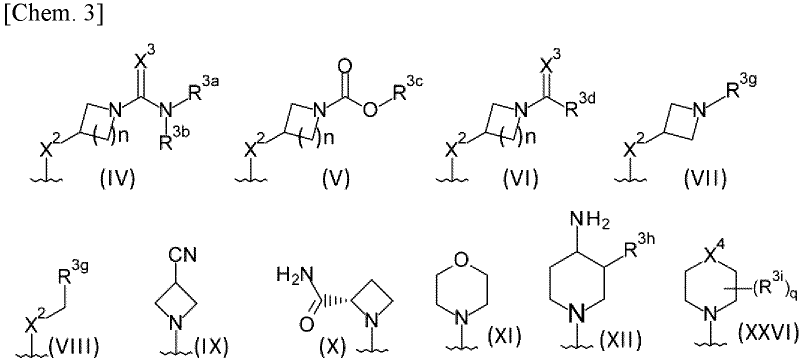
R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}, or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2},
R^{3b} is H or C₁₋₃ alkyl,
R^{3c} and R^{3d} are -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}, or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f} and -NR^{N1}R^{N2},
R^{3e} is H, F or C₁₋₃ alkyl,
R^{3f} is H or C₁₋₃ alkyl,
R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
R^{3h} is H, F or C₁₋₃ alkyl,
each R³ⁱ, which is the same as or different from each other, is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
two R³ⁱ present on the same carbon atom, together with the carbon atom adjacent thereto, optionally form a spiro ring having a ring selected from the group consisting of a C₃₋₆ cycloalkane and a 4-membered to 6-membered saturated hetero ring, where the spiro ring is optionally substituted with one or two groups selected from the group consisting of C₁₋₃ alkyl, -O-(C₁₋₃ alkyl), OH, halogen and oxo, or
R³ⁱ present on two adjacent carbon atoms, together with the two carbon atoms, optionally forms a fused ring having a ring selected from the group consisting of a C₃₋₆ cycloalkane and a 4-membered to 6-membered saturated hetero ring, where the fused ring is optionally substituted with one or two groups selected from the group consisting of C₁₋₃ alkyl, -O-(C₁₋₃ alkyl), OH, halogen and oxo, or
R³ⁱ present on two non-adjacent carbon atoms, together with the two carbon atoms, optionally forms a bridged structure composed of one or two carbon atoms, and a ring having the bridged structure is optionally substituted with one or two groups selected from the group consisting of C₁₋₃ alkyl, -O-(C₁₋₃ alkyl), OH, halogen and oxo,
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group, or
R^{3e} and R^{N1}, together with the carbon atom and the nitrogen atom to which they are attached, optionally form an optionally substituted 4-membered to 6-membered saturated heterocyclic group,
X² is -O-, -NH- or -N(C₁₋₃ alkyl)-,
X³ is O or S,
X⁴ is -CH₂-, -CH₂-CH₂- or -O-CH₂-,
n is 1 or 2,
p is 1 or 2,
q ranges from 1 to 8,
R⁴ is C₁₋₆ alkyl, piperidinyl optionally substituted with R^{4a} or tetrahydropyranyl, where the C₁₋₆ alkyl is optionally substituted with a group selected from the group consisting of F, OH, OCH₃, R^{4a}, cyclopropyl, N(R^{4a})₂, pyrrolidinyl optionally substituted with R^{4a} and tetrahydrofuranyl,
R^{4a} is optionally substituted C₁₋₃ alkyl,
Y is phenylene optionally substituted with F or Cl or pyridinediyl,
L is -(L¹-L²-L³-L⁴-L⁵).
L¹, L², L³, L⁴ and L⁵, which are the same as or different from each other, are groups selected from the group consisting of a bond, -O-, -NR^{L1}-, an optionally substituted saturated heterocyclic divalent group containing one or two nitrogen atoms, optionally substituted C₁₋₃ alkylene and C=O,
R^{L1} is H or C₁₋₃ alkyl,
Z is a group selected from the group consisting of the formula (XIII), the formula (XIV), the formula (XV), the formula (XVI), the formula (XVII), the formula (XVIII), the formula (XIX) and the formula (XX) below, 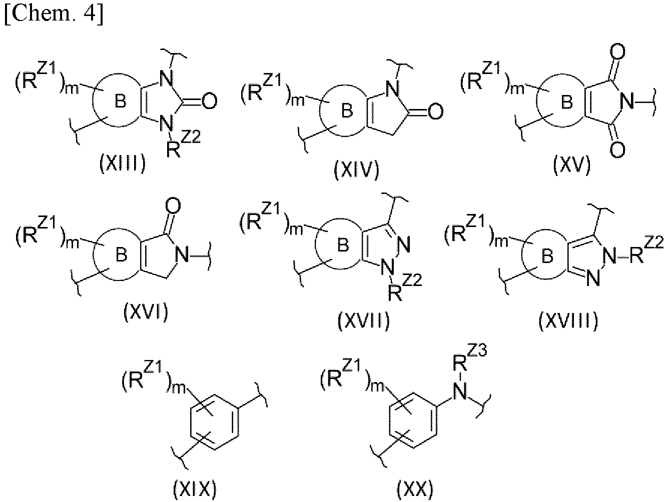
ring B is a benzene ring or a 6-membered hetero ring containing one or two nitrogen atoms,
R^{Z1} is H, C₁₋₃ alkyl, -O-(C₁₋₃ alkyl), -NR^{Z4}₂, -CONR^{Z4}₂ or -NR^{Z4}COR^{Z5},
R^{Z2} is H or C₁₋₃ alkyl,
R^{Z3} is H or C₁₋₃ alkyl,
each R^{Z4}, which is the same as or different from each other, is H or C₁₋₃ alkyl,
R^{Z5} is C₁₋₃ alkyl,
L is attached to ring B in the formulae (XIII) to (XVIII) above or to the benzene ring in the formula (XIX) and the formula (XX),
m is 1 or 2, and
G is CH or N,
provided that when G is N, Z is the formula (XVII), the formula (XVIII) or the formula (XIX) above).

2. The compound or a salt thereof according to claim 1,
wherein X¹ is -O- or -NR^{X1}-,
R^{X1} is H or optionally substituted C₁₋₃ alkyl,
when X¹ is -NR^{X1}-, R^{X1} and R⁴ present on the same nitrogen atom, together with a nitrogen atom adjacent thereto, optionally form an optionally substituted 4-membered to 6-membered saturated heterocyclic group,
R¹ is the formula (II) below, 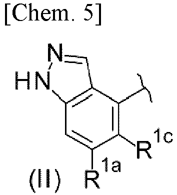
R^{1a} is H, methyl, F or Cl,
R^{1c} is F, Cl, methyl or ethyl,
R² is cyclopropyl or vinyl,
R³ is a group selected from the group consisting of the formula (IV), the formula (VII), the formula (VIII), the formula (IX), the formula (X), the formula (XI) and the formula (XII) below, 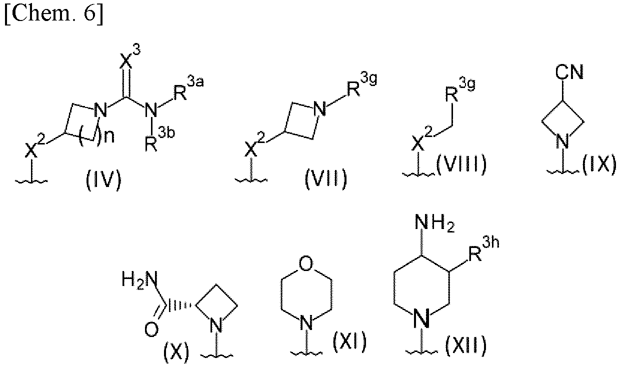
R^{3a} is -(CH₂)ₚCHR^{3e}-NR^{N1}R^{N2}; -(CH₂)ₚCHR^{3e}-OR^{3f}; a 4-membered to 6-membered saturated heterocyclic group optionally substituted with a group selected from the group consisting of C₁₋₃ alkyl, -C₁₋₃ alkylene-OR^{3f}, -C₁₋₃ alkylene-NR^{N1}R^{N2} and -NR^{N1}R^{N2}, or C₃₋₆ cycloalkyl optionally substituted with a group selected from the group consisting of C₁₋₃ alkyl, -C₁₋₃ alkylene-OR^{3f}, C₁₋₃ alkylene-NR^{N1}R^{N2}, -OR^{3f} and -NR^{N1}R^{N2},
R^{3b} is H or C₁₋₃ alkyl,
R^{3e} is H, F or C₁₋₃ alkyl,
R^{3f} is H or C₁₋₃ alkyl,
R^{3g} is optionally substituted C₃₋₆ cycloalkyl, optionally substituted 5-membered heteroaryl, optionally substituted 6-membered heteroaryl or an optionally substituted 4-membered to 6-membered saturated heterocyclic group,
R^{3h} is H, F or C₁₋₃ alkyl,
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group, or
R^{3e} and R^{N1}, together with the carbon atom and the nitrogen atom to which they are attached, optionally form an optionally substituted 4-membered to 6-membered saturated heterocyclic group,
X² is -O-, -NH- or -N(C₁₋₃ alkyl)-,
X³ is O or S,
n is 1 or 2,
p is 1 or 2,
Y is phenylene optionally substituted with F or Cl,
Z is a group selected from the group consisting of the formula (XIII), the formula (XVII) and the formula (XIX) below, 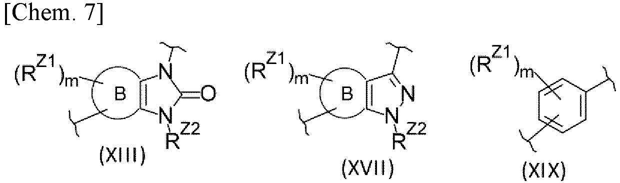
ring B is a benzene ring or a 6-membered hetero ring containing one or two nitrogen atoms,
R^{Z1} is H, C₁₋₃ alkyl, -O-(C₁₋₃ alkyl), -NR^{Z4}₂, -CONR^{Z4}₂ or -NR^{Z4}COR^{Z5},
R^{Z2} is H or C₁₋₃ alkyl,
each R^{Z4}, which is the same as or different from each other, is H or C₁₋₃ alkyl,
R^{Z5} is C₁₋₃ alkyl,
L is attached to ring B in the formula (XIII) or (XVII) above,
m is 1 or 2, and
G is CH or N,
provided that when G is N, Z is the formula (XVII) or the formula (XIX) above.

3. The compound or a salt thereof according to claim 2,
wherein A is CR^{A} or N,
R^{A} is H,
X¹ is -O-,
R¹ is the formula (II) below, 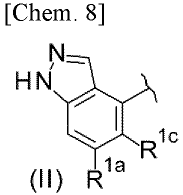
R^{1a} is F,
R^{1c} is methyl,
R² is cyclopropyl,
R³ is a group selected from the group consisting of the formula (IV), the formula (VII), the formula (VIII), the formula (IX), the formula (X), the formula (XI) and the formula (XII) below, 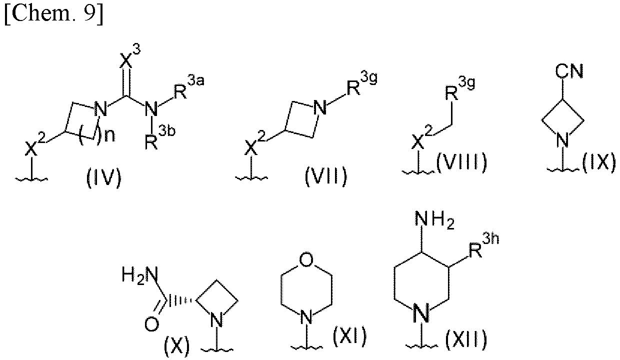
R^{3a} is -(CH₂)ₚCHR^{3e}-NR^{N1}R^{N2},
R^{3b} is H or C₁₋₃ alkyl,
R^{3e} is H,
R^{3g} is optionally substituted 6-membered heteroaryl,
R^{3h} is H or F,
R^{N1} and R^{N2}, which are the same as or different from each other, are C₁₋₃ alkyl,
X² is -O- or -NH-,
X³ is O or S,
n is 1,
p is 1,
R⁴ is C₁₋₆ alkyl optionally substituted with a group selected from the group consisting of OCH₃, N(C₁₋₃ alkyl)₂ and pyrrolidinyl optionally substituted with R^{4a} or tetrahydropyranyl,
R^{4a} is optionally substituted C₁₋₃ alkyl,
Y is phenylene,
L is -(L¹-L²-L³-L⁴-L⁵)-, and L¹ contained in L is attached to Y,
L¹ is C₁₋₃ alkylene or C=O,
L² is an optionally substituted saturated heterocyclic divalent group containing one or two nitrogen atoms,
L³ is C₁₋₃ alkylene,
L⁴ is a bond, -O- or -N(C₁₋₃ alkyl)-,
L⁵ is a bond or C₁₋₃ alkylene,
Z is a group selected from the group consisting of the formula (XIII), the formula (XVII) and the formula (XIX) below, 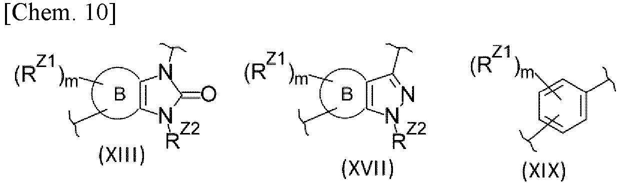
ring B is a benzene ring,
R^{Z1} is H or C₁₋₃ alkyl,
R^{Z2} is H or C₁₋₃ alkyl,
L is attached to ring B in the formula (XIII) or (XVII) above or to the benzene ring in the formula (XIX), and
m is 1 or 2.

4. The compound or a salt thereof according to claim 3, wherein R³ is a group selected from the group consisting of the formula (IV-1), the formula (VII-1), the formula (VIII-1), the formula (IX), the formula (X), the formula (XI) and the formula (XII-1) below, 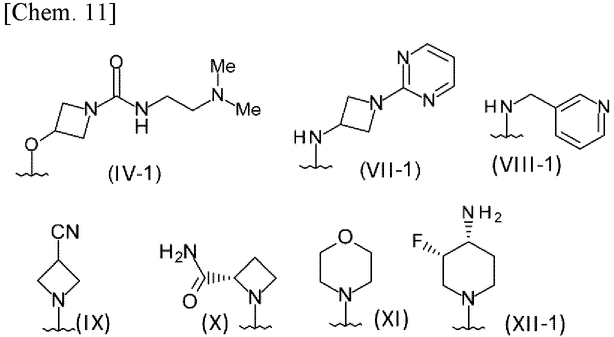
L is one group selected from the group consisting of the following formulae (XXVII) to (XXXIV), wherein a carbon atom with * is attached to Y, 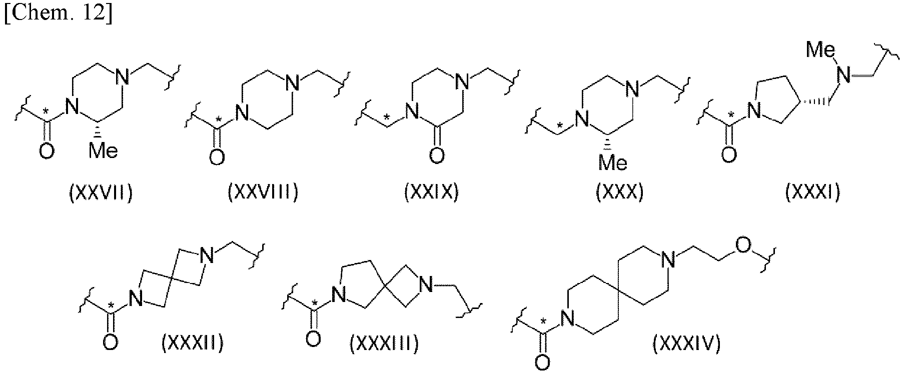
Z is a group selected from the group consisting of the formula (XIII-1), the formula (XVII-1) and the formula (XIX-1), 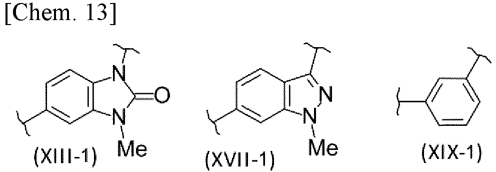
L is attached to a benzene ring in the formula (XIII-1) and the formula (XVII-1) above, and
G is CH or N,
provided that when G is N, Z is the formula (XVII-1) or the formula (XIX-1) above.

5. A pharmaceutical composition comprising the compound or a salt thereof according to claim 1 and one or more pharmaceutically acceptable excipients.

6. The pharmaceutical composition according to claim 5, which is a pharmaceutical composition for treating pancreatic cancer and/or lung cancer.

7. Use of the compound or a salt thereof according to claim 1 for the manufacture of a pharmaceutical composition for treating pancreatic cancer and/or lung cancer.

8. The compound or a salt thereof according to claim 1 for use in treatment of pancreatic cancer and/or lung cancer.

9. Use of the compound or a salt thereof according to claim 1 for treatment of pancreatic cancer and/or lung cancer.

10. A method for treating pancreatic cancer and/or lung cancer, the method comprising administering an effective amount of the compound or a salt thereof according to claim 1 to a subject.
